# FASCICULE DE BREVET EUROPEEN

(11) **EP 1 103 549 B1**
(45) Date de publication et mention de la délivrance du brevet: **04.06.2003**
(21) Numéro de dépôt: 00403068.0
(22) Date de dépôt: 06.11.2000
(51) Int. Cl.: C07D 307/52, C07D 307/64, C07D 241/24, C07D 233/54, C07D 231/16, C07D 207/32, C07D 209/14, C07D 213/53, C07C 257/22, C07D 405/12, C07D 401/12, C07D 403/12, A61K 7/42

(54) **Compositions cosmétiques photoprotectrices contenant des dérivés aminoamidines et utilisations**
Kosmetische photoschutzzusammenstellungen enthaltende Aminoamidine Derivate und Verwendungen
Photoprotective cosmetic compositions containing aminoamidine derivatives and uses thereof

(30) Priorité: 29.11.1999 FR 9914999
(43) Date de publication de la demande: 30.05.2001
(73) Titulaire: L'OREAL, 75008 Paris (FR)
(72) Inventeur: Breton, Philippe, 78150 Le Chesnay (FR)
(74) Mandataire: Miszputen, Laurent

(56) Documents cités:
- US-A- 5 298 647
- BILLINGTON,D.C.: "SYNTHESIS AND ANTIMYCOBACTERIAL ACTIVITY OF SOME HETEROARYLCARBOXAMIDRAZONE DERIVATIVES." DRUG DESIGN AND DISCOVERY, vol. 15, no. 4, 1998, pages 269-75, XP000930054
- CHEMICAL ABSTRACTS, vol. 74, no. 28, 1971 Columbus, Ohio, US; abstract no. 125632s, H. FOKS ET AL.: "PYRAZINE DERIVATIVES I." page 471; XP002145031 & DISS. PHARM. PHARMACOL., vol. 23, no. 1, 1971, pages 49-58,
- CHEMICAL ABSTRACTS, vol. 94, no. 28, 1981 Columbus, Ohio, US; abstract no. 65532k, K.N. ZELENIN ET AL.: "STRUCTURE OF PRODUCTS OF THE CONDENSATION OF AMIDRAZONES WITH MONOCARBONYL COMPOUNDS." page 715; XP002145032 & ZH. ORG. KHIM., vol. 16, no. 5, 1980, pages 942-59, USSR
- CHEMICAL ABSTRACTS, vol. 128, no. 23, 1998 Columbus, Ohio, US; abstract no. 278800d, E.G. BELOZERTSEVA ET AL.: "SYNTHESI AND ANTIHYPERTENSIVE ACTIVITY OF SALTS OF 1-ALKYLIDENE(ARYLIDENE)BENZAMIDRAZONES." page 36; colonne 2; XP002145033 -& Chem. Abs. Chemical Subsance Index Vol 128 XP002145030 & KHIM-PHARM. ZH., vol. 31, no. 8, 1997, pages 16-18, USSR

## Description

L'invention concerne des compositions cosmétiques ou dermatologiques, destinées à la photoprotection de la peau et/ou des cheveux contre le rayonnement UV, en particulier le rayonnement solaire comprenant une quantité efficace d'au moins un dérivé aminoamidine de structure particulière

L'invention concerne également l'utilisation dans la préparation de formulations cosmétiques ou dermatologiques photoprotectrices de ces dérivés d'aminoamidines particuliers à titre de filtres solaires actifs dans le domaine des rayonnements UV.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

De nombreux composés destinés à la photoprotection (UV-A et/ou UV-B) de la peau ont été proposés à ce jour.

La plupart d'entre eux sont des composés aromatiques présentant une absorption des rayons UV dans la zone comprise entre 280 et 315 nm, ou dans la zone comprise entre 315 et 400 nm ou bien encore dans l'ensemble de ces deux zones. Ils sont le plus souvent formulés dans des compositions antisolaires qui se présentent sous la forme d'une émulsion de type huile-dans-eau (c'est à dire un support cosmétiquement acceptable constitué d'une phase continue dispersante aqueuse et d'une phase discontinue dispersée huileuse) et qui contiennent donc, à des concentrations diverses, un ou plusieurs filtres organiques classiques à fonction aromatique, lipophiles et/ou hydrophiles, capables d'absorber sélectivement les rayonnements UV nocifs, ces filtres (et leurs quantités) étant sélectionnés en fonction du facteur de protection solaire recherché (le facteur de protection solaire s'exprimant mathématiquement par le rapport du temps d'irradiation nécessaire pour atteindre le seuil érythématogène avec le filtre UV au temps nécessaire pour atteindre le seuil érythématogène sans filtre UV).

Outre leur pouvoir filtrant, ces composés à activité anti-UV doivent également présenter de bonnes propriétés cosmétiques dans les compositions qui les contiennent, une bonne solubilité dans les solvants usuels et en particulier les corps gras tels que les huiles et les graisses, ainsi qu'une bonne stabilité vis à vis des radiations UV (photostabilité).

La Demanderesse a découvert de manière surprenante une nouvelle famille de filtres UV organiques du type dérivé d'aminoamidine présentant, outre des propriétés filtrantes excellentes dans le domaine des rayonnements UV-A et/ou UV-B, une très bonne solubilité dans les solvants organiques usuels et notamment les corps gras tels que les huiles, une excellente photostabilité ainsi que d'excellentes propriétés cosmétiques, les rendant particulièrement appropriés à une utilisation comme filtres solaires dans des compositions cosmétiques destinées à la protection de la peau et/ou des cheveux contre le rayonnement ultraviolet.

La présente invention a ainsi pour premier objet une composition cosmétique à usage topique, en particulier pour la photoprotection de la peau et/ou des cheveux, caractérisée par le fait qu'elle comprend, dans un support cosmétiquement acceptable au moins un dérivé d'aminoamidine répondant à la formule (I) suivante : 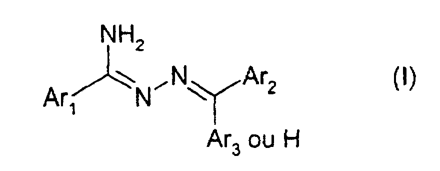 dans laquelle:
- Ar₁ représente
   (i) un noyau aromatique de formule suivante : 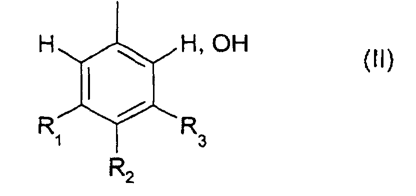 où R₁, R₂, R₃, identiques ou différents, désignent un atome d'hydrogène ; un radical alkyle en C₁-C₈ linéaire ou ramifié ; un radical alkoxy en C₁-C₈; R₁, R₂, R₃ peuvent former un cycle aromatique ou non fusionné ou bien un hétérocycle aromatique à 5 ou 6 chaînons.
   (ii) un noyau aromatique à 6 atomes répondant à la structure suivante : 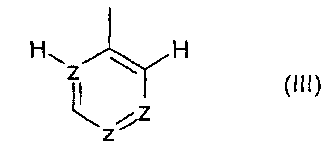 où Z désigne C ou N;
- Ar₂ représente un noyau aromatique répondant à la définition de Ar₁ telle que définie précédemment ou bien un noyau aromatique à 5 atomes répondant à la structure suivante : 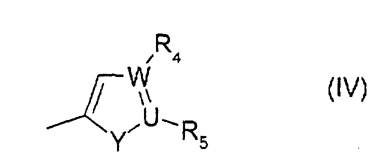 dans laquelle :
- Y représente un atome de soufre, d'oxygène, de carbone ou d'azote ;
- W représente un atome de carbone ou d'azote ;
- R₄ désigne
   (i) un atome d'hydrogène lorsque W est un atome d'azote
   (ii) R₄ désigne un atome d'hydrogène , un radical alkyle en C₁-C₈ linéaire ou ramifié ; un radical alkoxy en C₁-C₈ ou peut former un cycle aromatique ou non fusionné , lorsque W est un atome de carbone ;
- U représente un atome de carbone ou d'azote, lorsque W est un atome de carbone ;
- R₅ désigne :
   (i) un atome d'hydrogène lorsque U désigne azote;
   (ii) un atome d'hydrogène ; un radical alkyle en C₁-C₈ linéaire ou ramifié ; un radical alkoxy en C₁-C₈ ou peut former un cycle aromatique ou non fusionné, lorsque U est un atome de carbone ;
- Ar₃ désigne un noyau aromatique répondant à la définition de Ar₂ telle que définie précédemment ou bien représente un atome d'hydrogène.

Les dérivés d'aminoamidine de formule (I) conformes à l'invention sont connus dans la littérature chimique et notamment certains d'entre eux ont été décrits dans l'article de BILLINGTON, D. C.; COLEMAN, M. D.; IBIABUO, J.; LAMBERT, P. A.; RATHBONE, D. L.; TIMS, K. J. *Drug Des. Discovery* **1998**, 15(4), 269-275.

Les dérivés d'aminoamidine de formule (I) conformes à l'invention peuvent être préparés selon un procédé de synthèse en trois étapes à partir d'un nitrile selon le schéma réactionnel suivant:

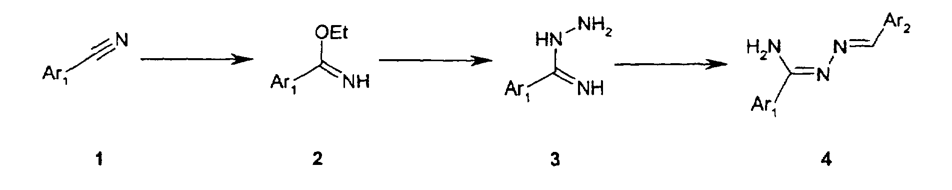

L'étape permettant de passer de **1** à **2** est la synthèse des iminoéthers de Pinner décrite par exemple dans PINNER, A. *Chem. Ber.* **1877**, 10, 1889 ; ROGER, R. ET NEILSON D. *Chem. Rev.***1961**, 61, 179 ; RAPOPORT, H *J.* Org. *Chem.* **1981**, 46, 2465.

De nombreux nitriles aromatiques sont disponibles chez la plupart des fournisseurs de produits chimiques comme par exemple :
4-nitrobenzonitrile (cas : 619-72-7) chez Acros référence 12848-0250
3-cyanopyridine (cas : 100-54-9) chez Acros référence 110861000
4-aminobenzonitrile (cas : 873-74-5) chez Acros référence 164470100
4-fluorobenzonitrile (cas : 1194-02-1) chez Acros référence 160810250
4-acetylbenzonitrile (cas : 1443-80-7) chez Acros référence 167580250.

Certains iminoéthers sont des produits commerciaux comme par exemple :
Chlorhydrate de l'ethyl-4-hydroxybenzimidate (cas : 54998-28-6) chez Aldrich référence 32,446-9
Chlorhydrate de l'éthylbenzimidate (cas : 5333-86-8) chez Fluka référence 12268.

L'étape permettant de passer de **2** à **3** se fait par action de l'hydrazine et est décrite dans la littérature notamment par CASE F. H. J. Org. Chem. **1965**, 30, 931 et aussi TAYLOR E. C. et MARTIN S. F. *J. Org. Chem.* **1972**, 37, 3958 ou encore par REPIC O., MATTNER P. G. ET SHAPIRO M. J. *J. Heterocyclic Chem.* **1982**, 19, 1201

L'étape permettant de passer de **3** à **4** est la condensation d'un aldéhyde aromatique en milieu alcoolique. Ce type de réaction est décrit dans la littérature notamment par MAMOLO M. G.; VIO L. ; BANFI E. ; PREDOMINATO M. ; FABRIS C. ; ASARO F. *Farmaco* **1992**, 47, 1055 et aussi par BILLINGTON D. C.; COLEMAN M. D, ; IBIABUO J. ; LAMBERT P. A. ; RATHBONE D. L ; TIMS K. J. Drug Des. Dis. **1998**, 15, 269.

De nombreux aldéhydes aromatiques sont disponibles chez la plupart des fournisseurs de produits chimiques comme par exemple :
Pyrrole-2Pyrrole-2-carboxaldehyde (cas : 1003-29-8) chez Aldrich référence P7,340-4
Trans-3-(2-furyl)acroleine (cas : 39511-08-5) chez Aldrich référence F2,060-2
2-methylindole-3-carboxaldehyde (cas : 5416-80-8) chez Aldrich référence 26,243-9
2-furaldehyde (cas : 98-01-1) chez Aldrich référence 31,991-0
4-methyl-5-imidazolecarboxaldehyde (cas :68282-53-1) chez Aldrich référence 39,215-4
1-methylindole-3-carboxaldehyde (cas : 19012-03-4) chez Aldrich référence 35,798-7

Parmi les dérivés d'aminoamidine de formule (I) conformes à l'invention, on peut citer ceux décrits dans le tableau suivant :

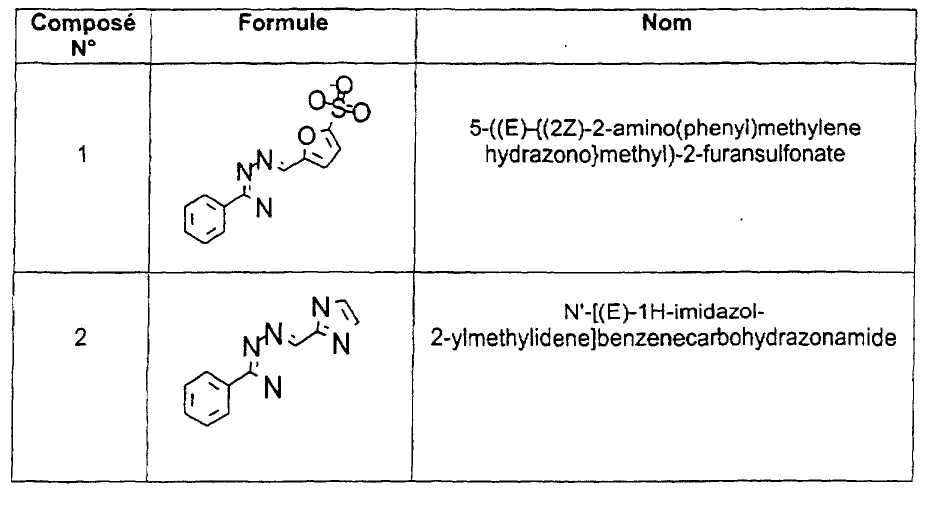

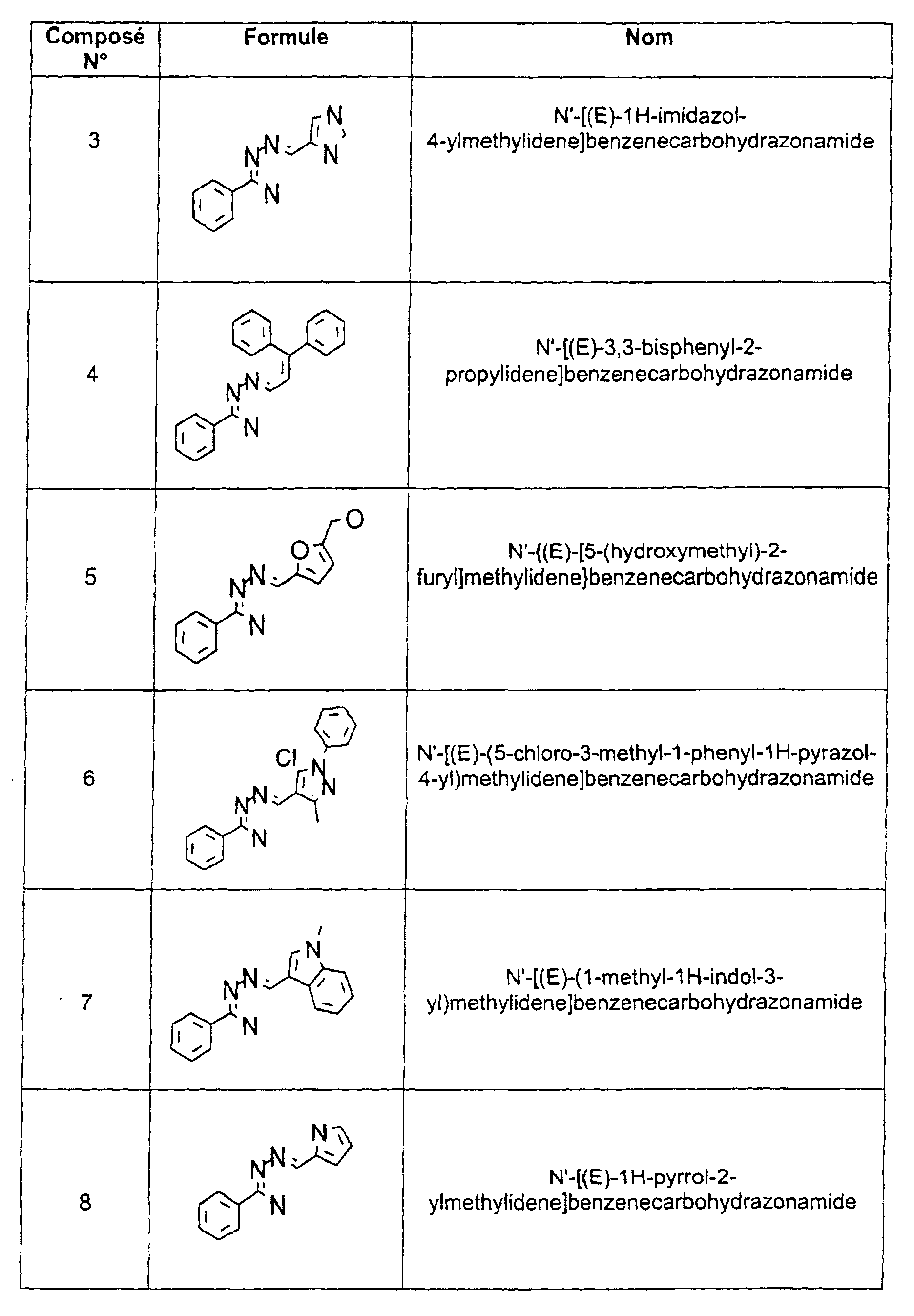

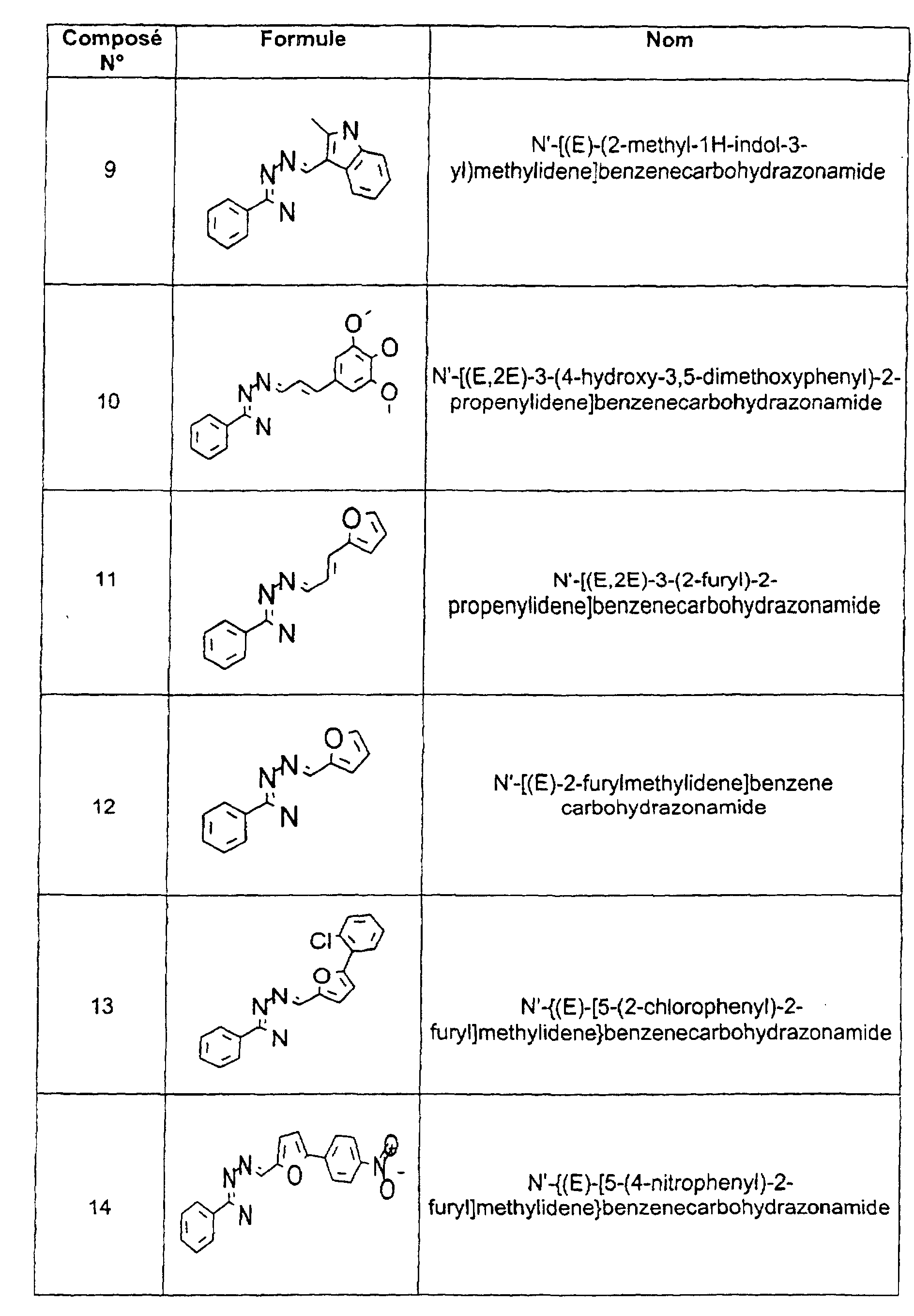

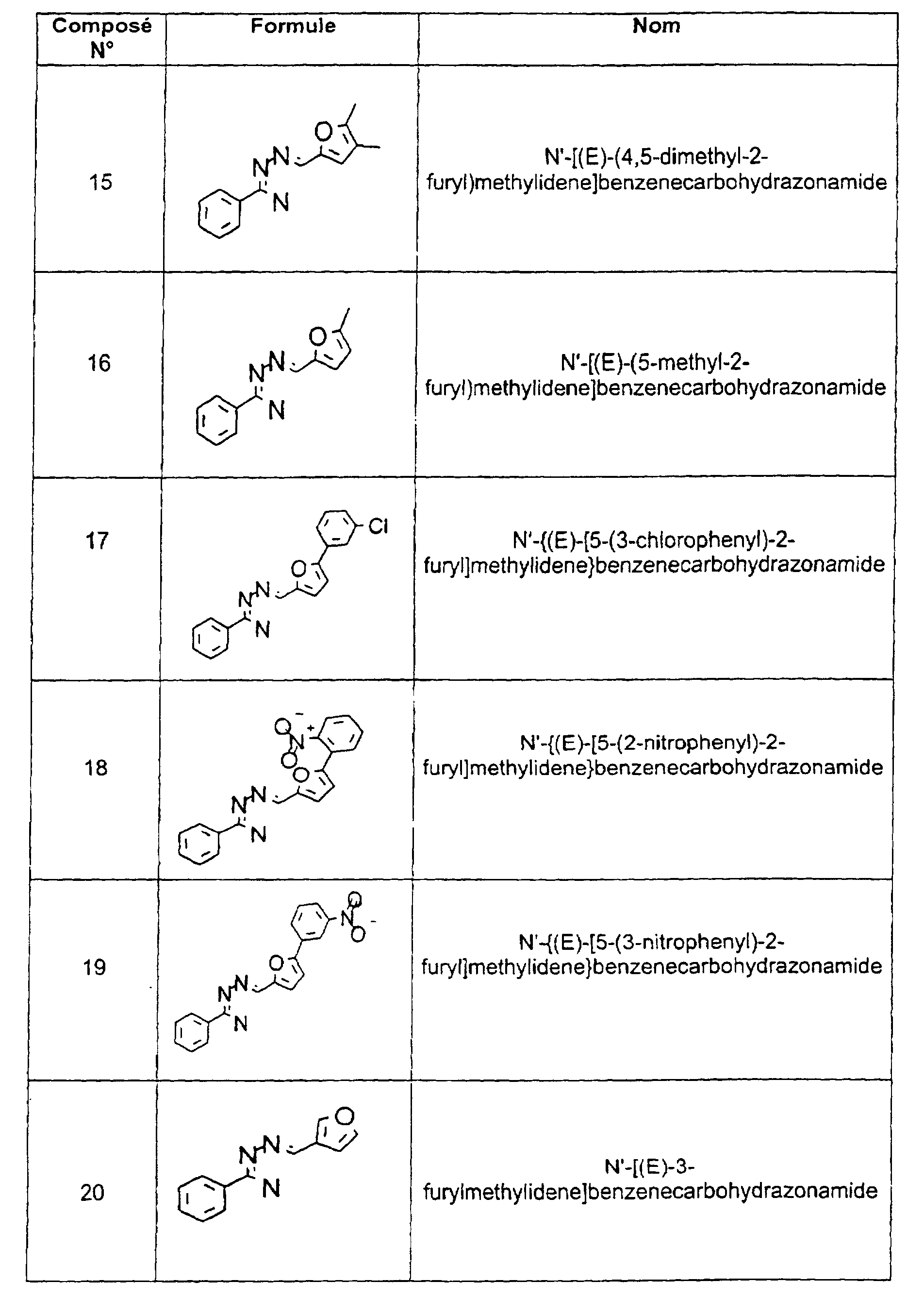

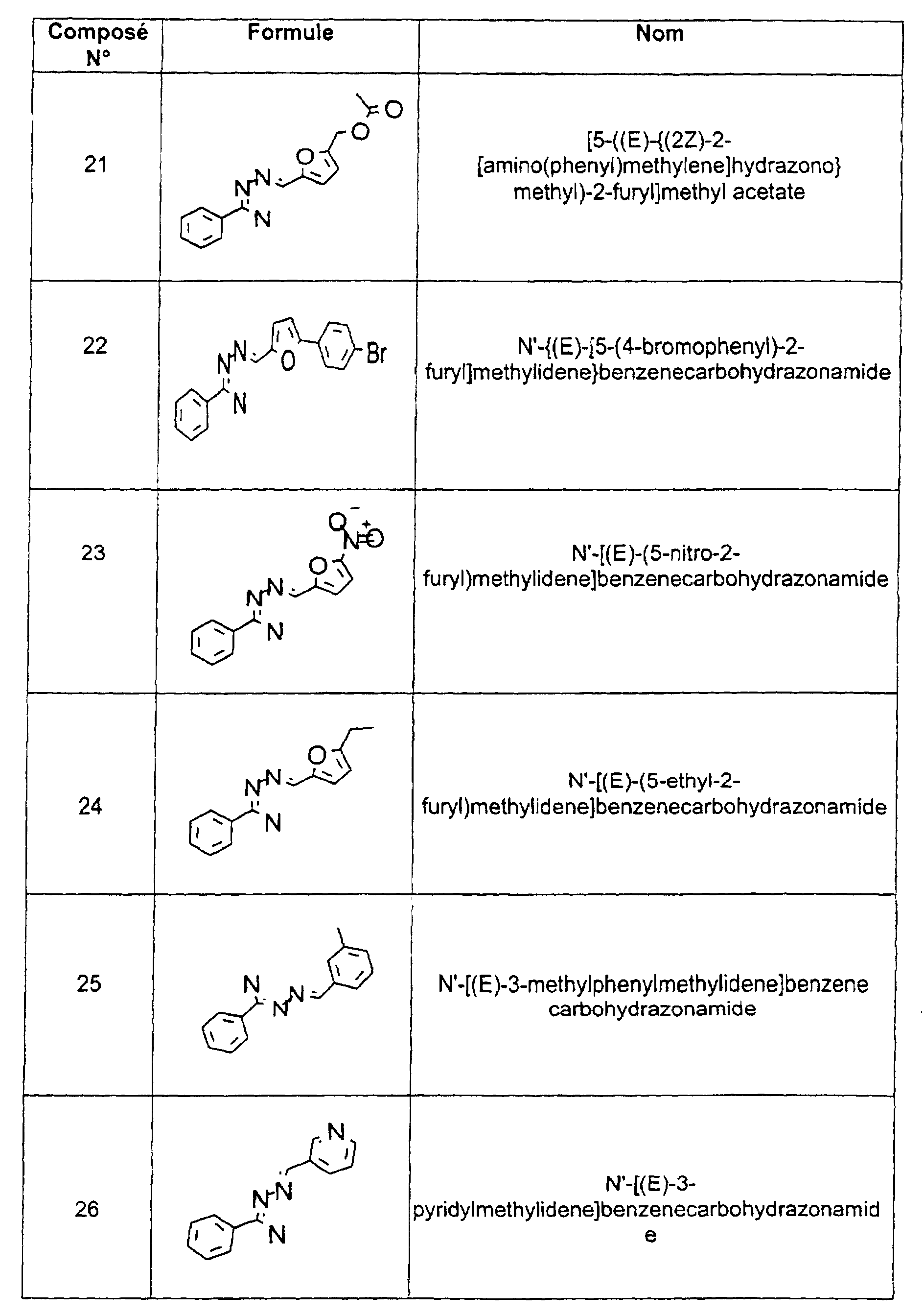

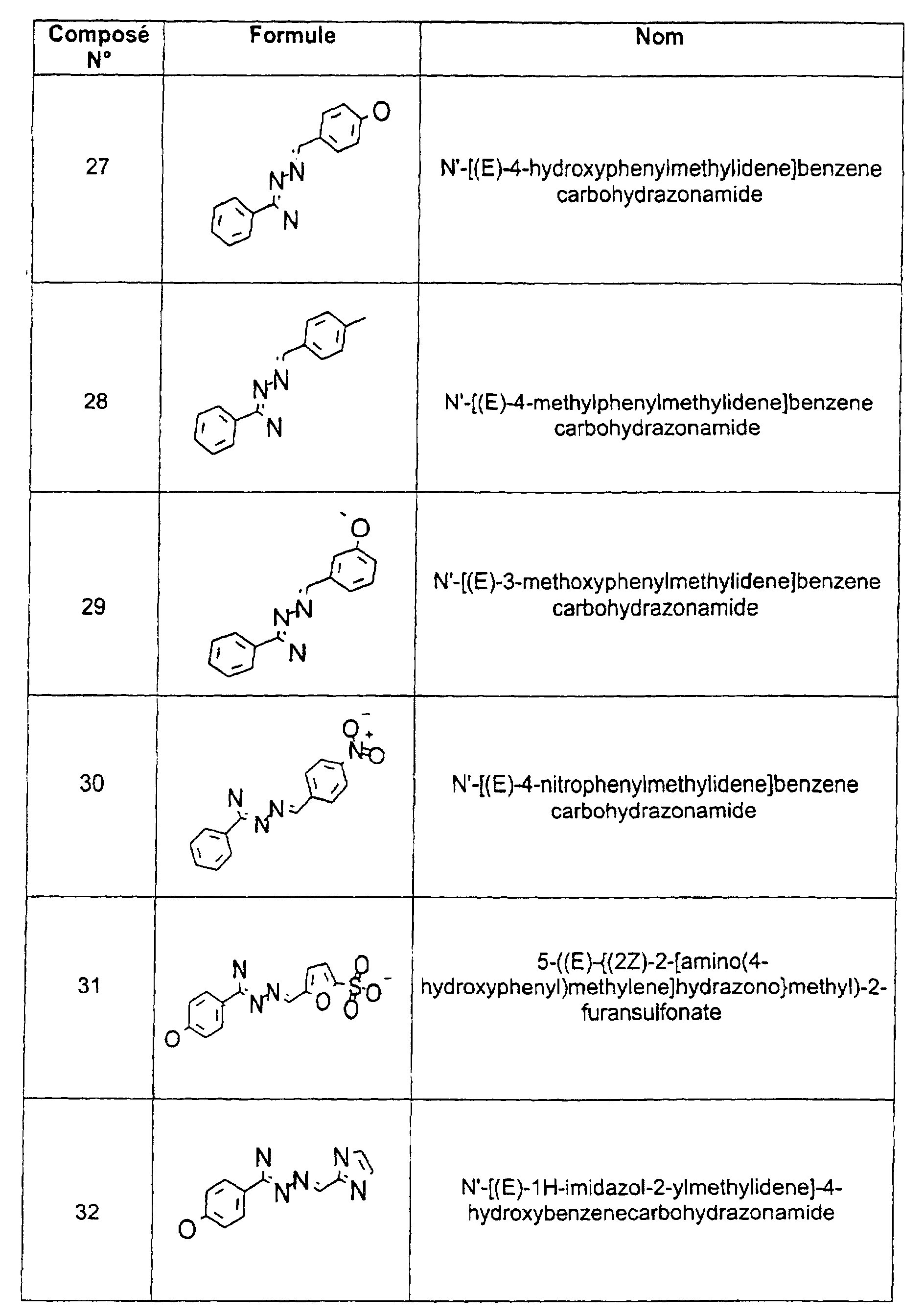

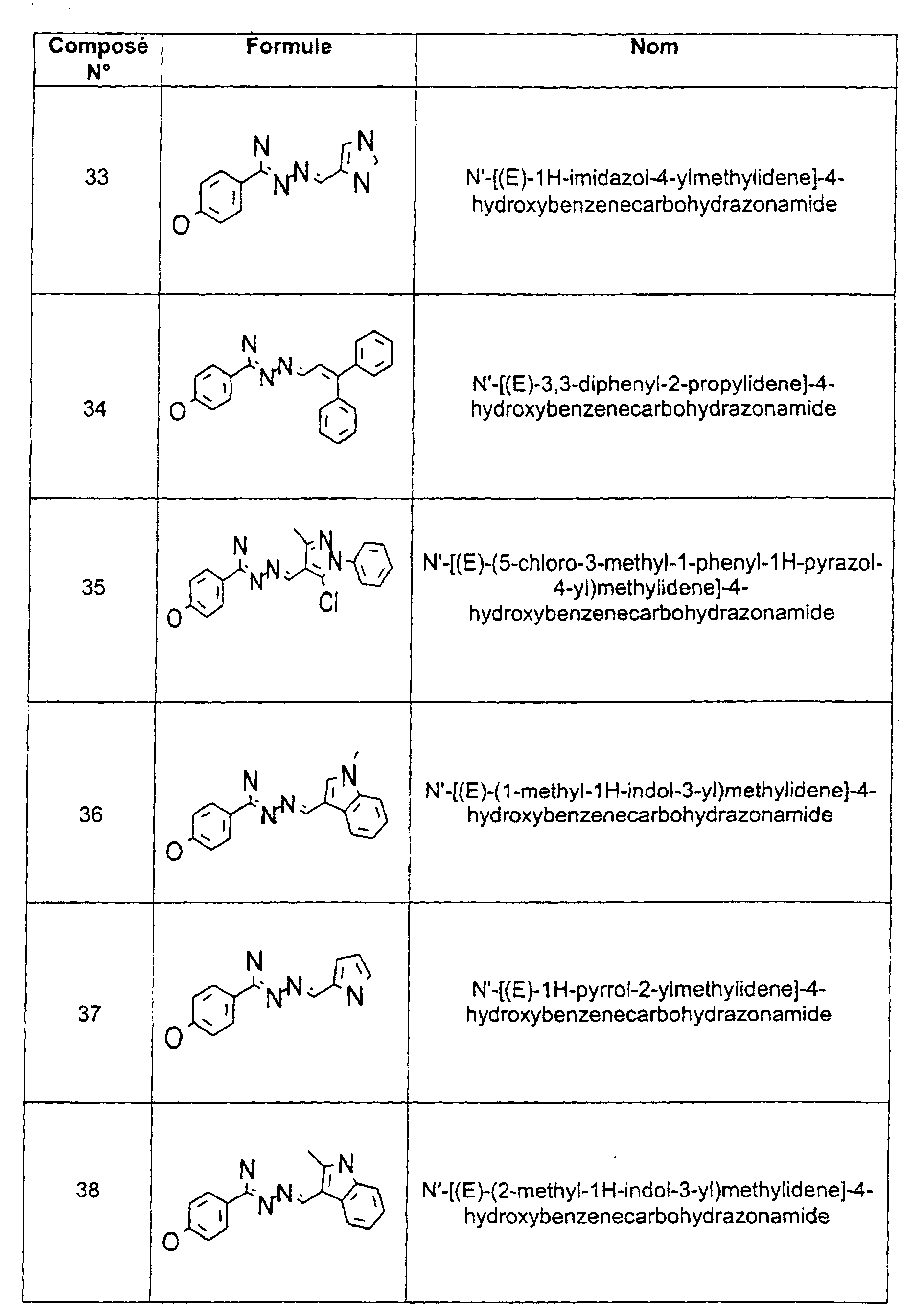

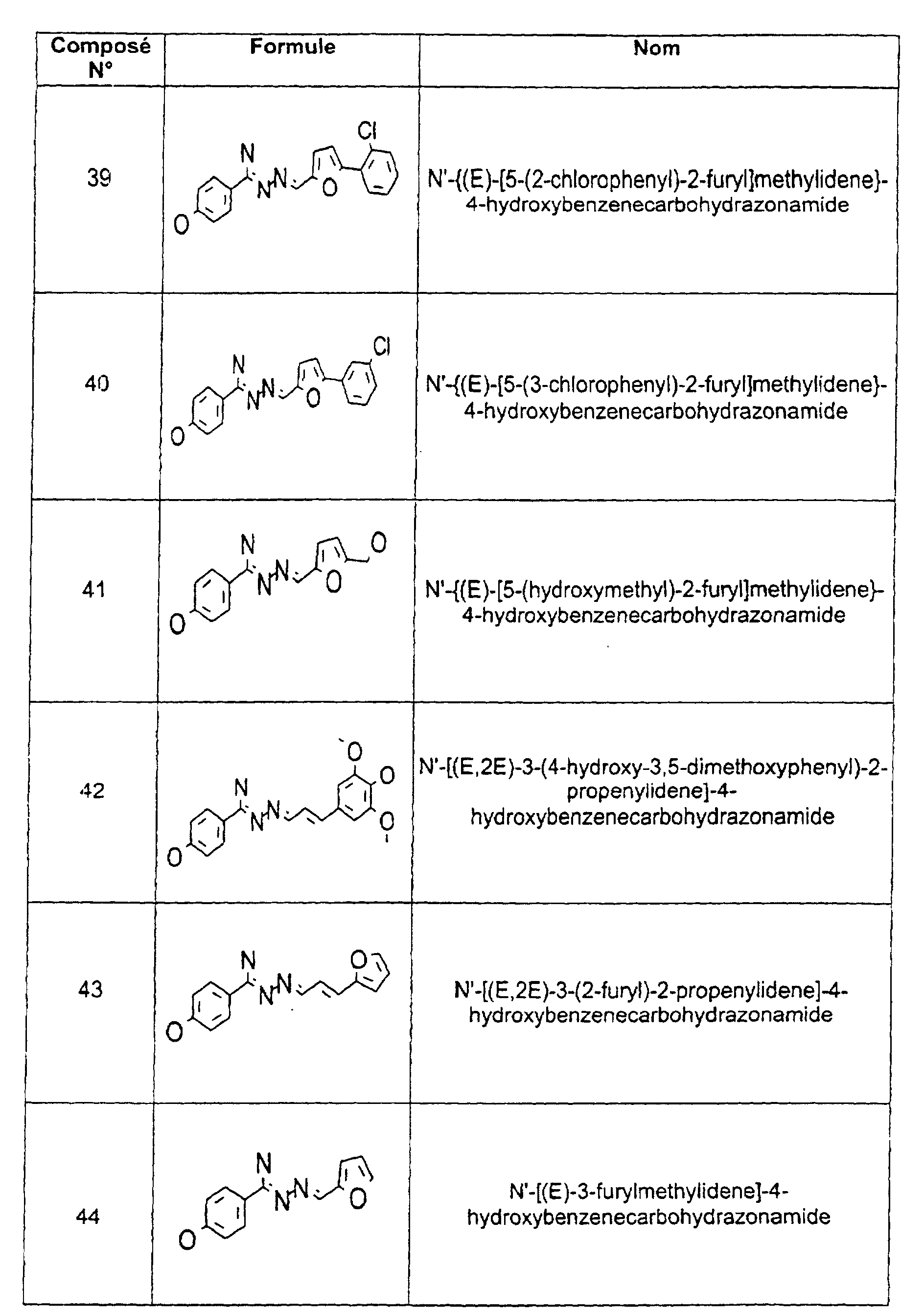

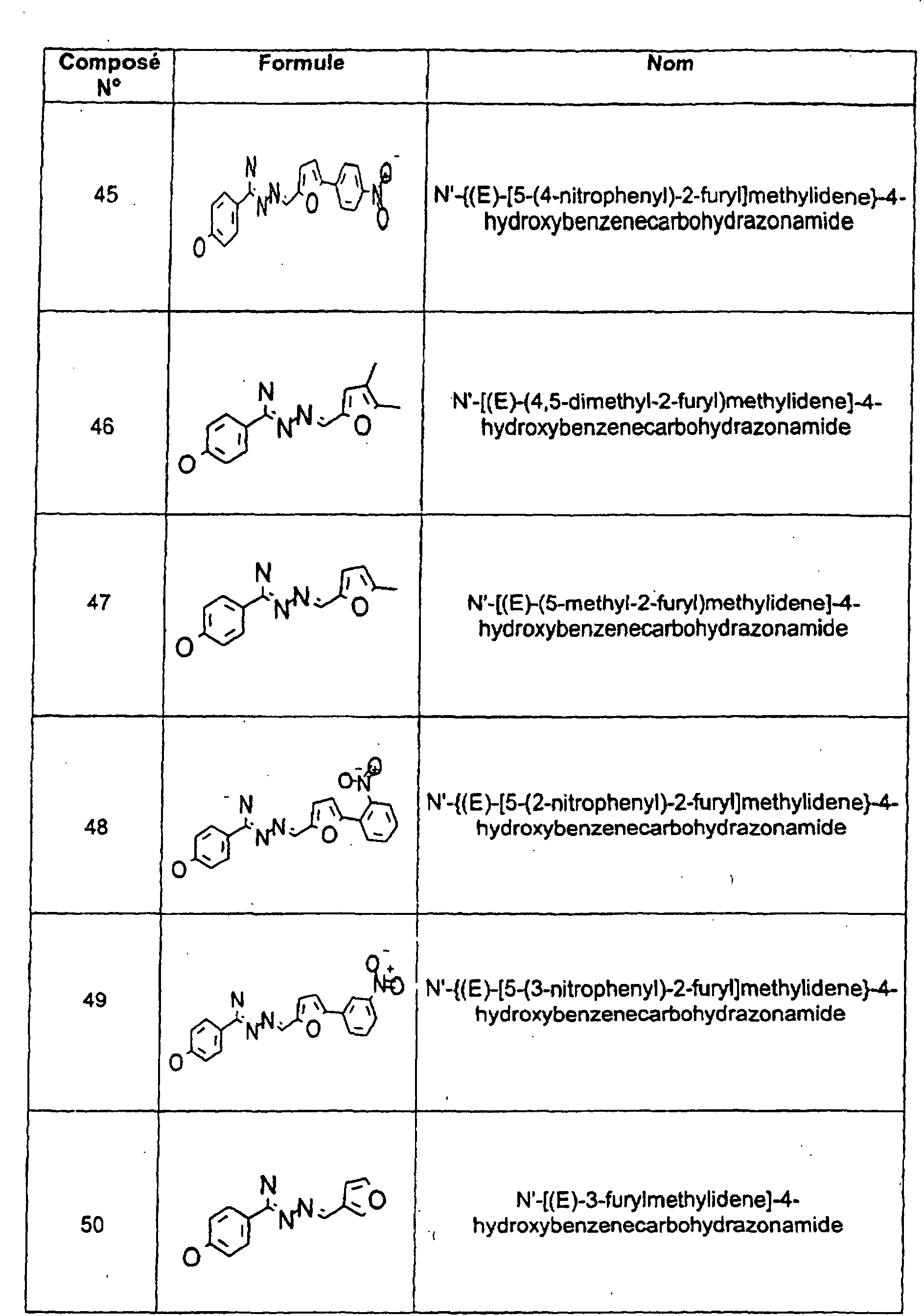

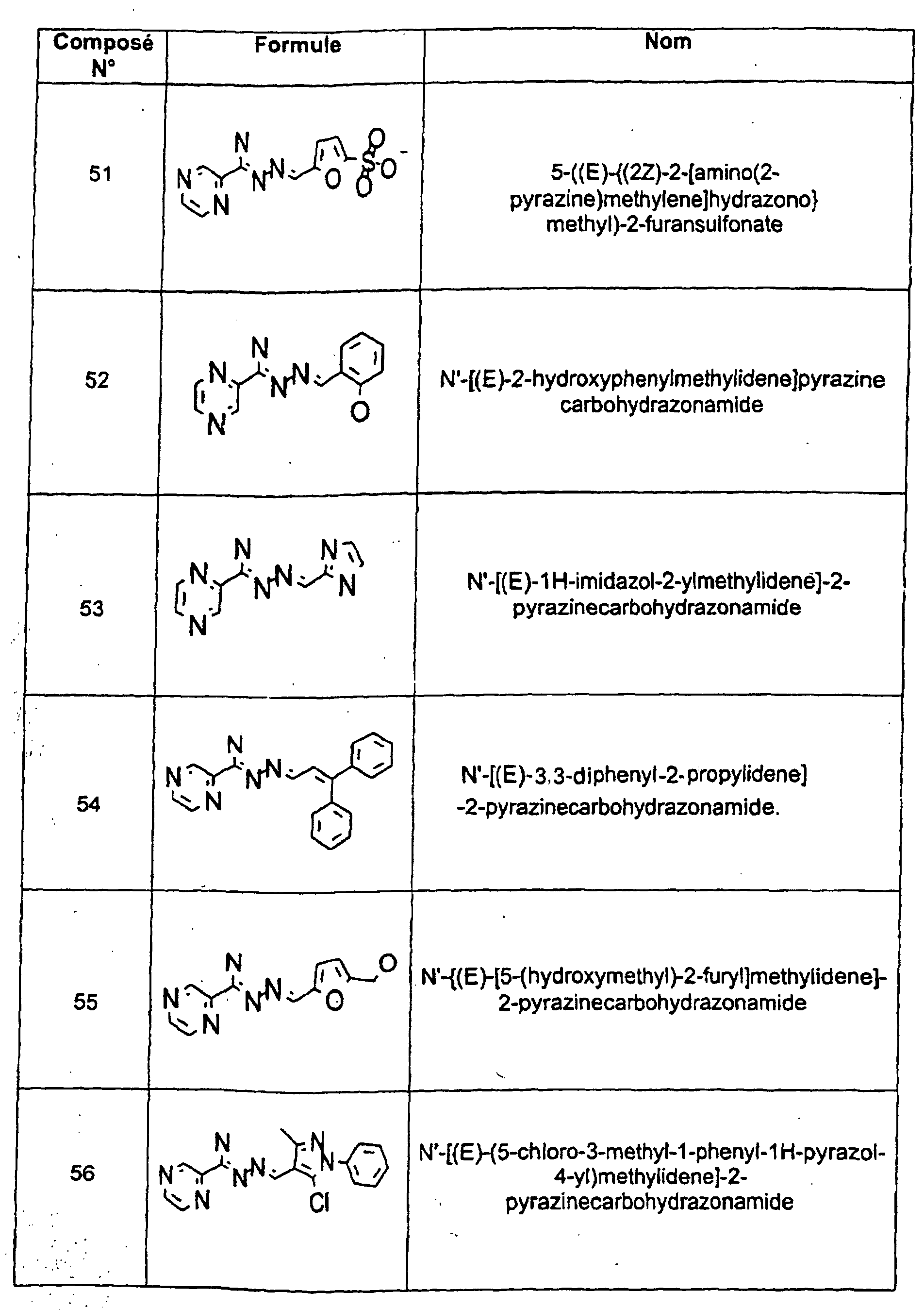

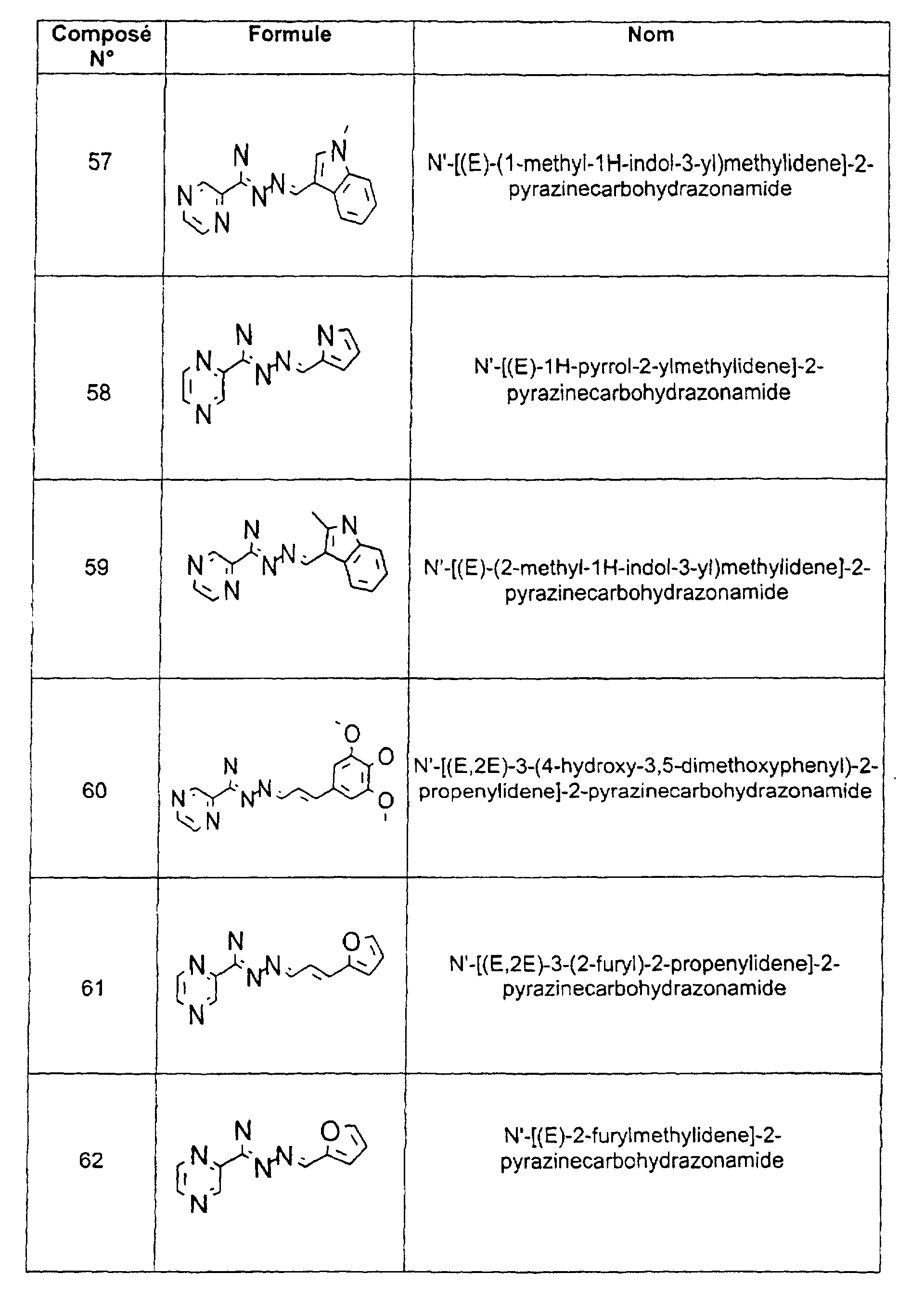

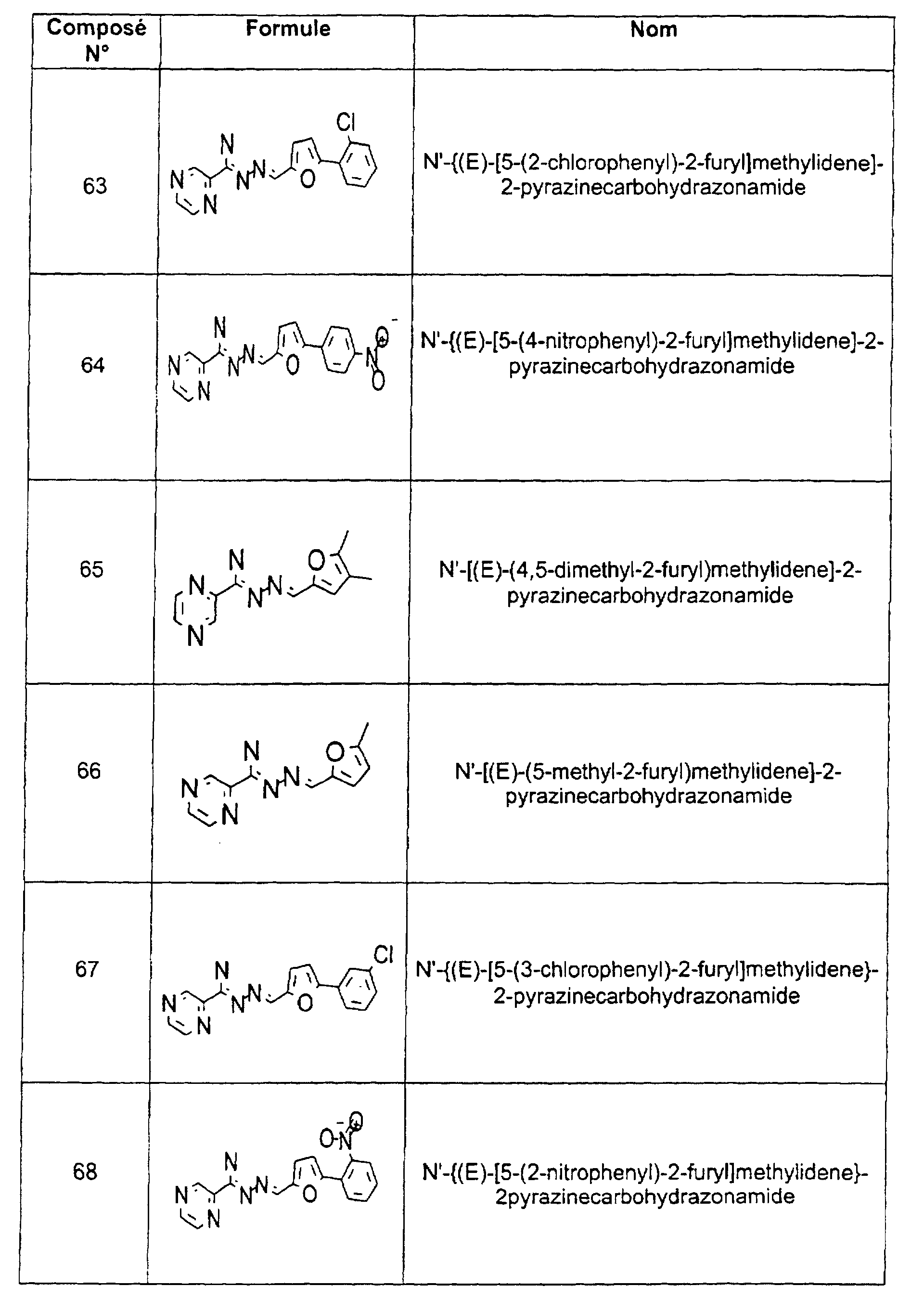

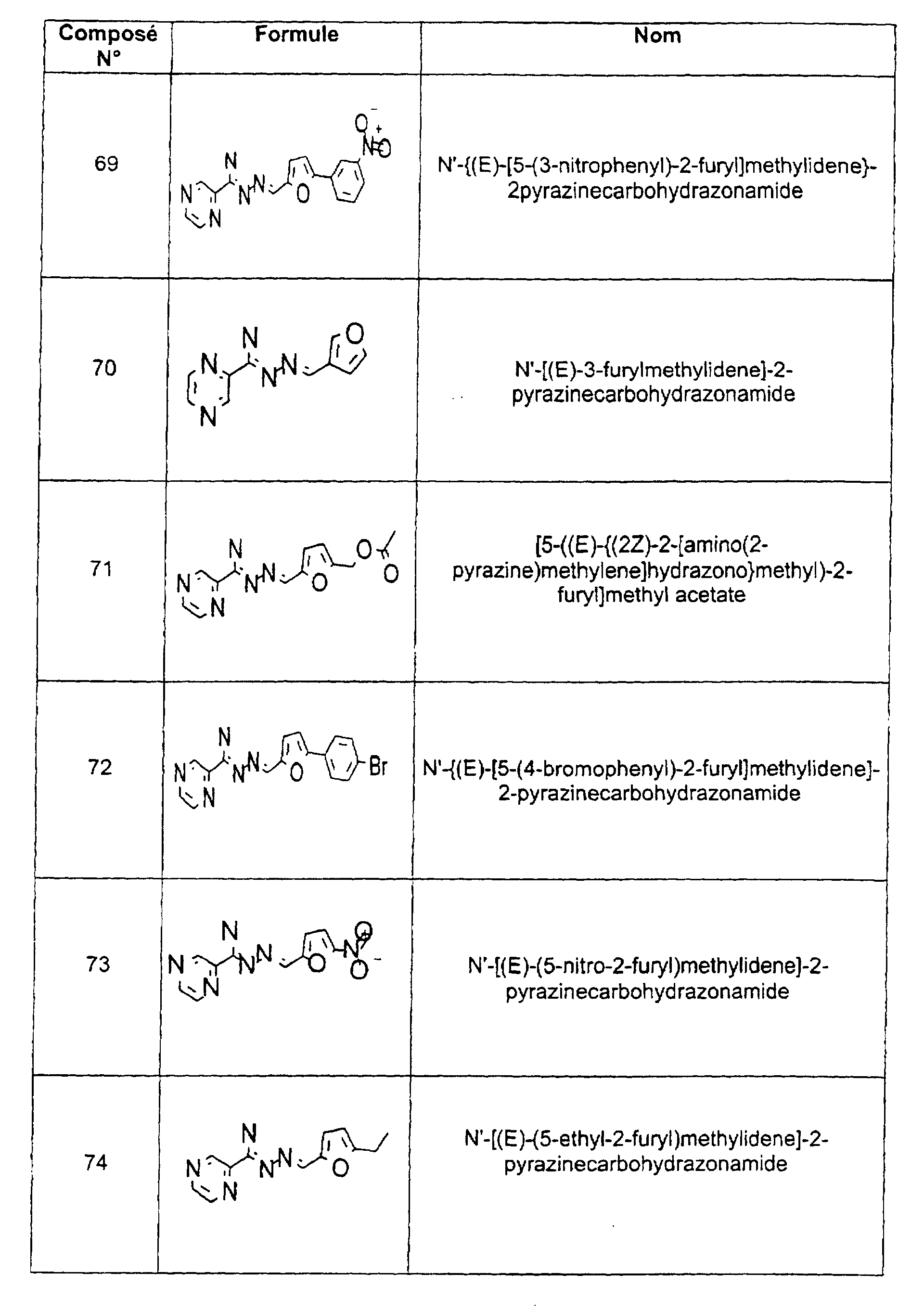

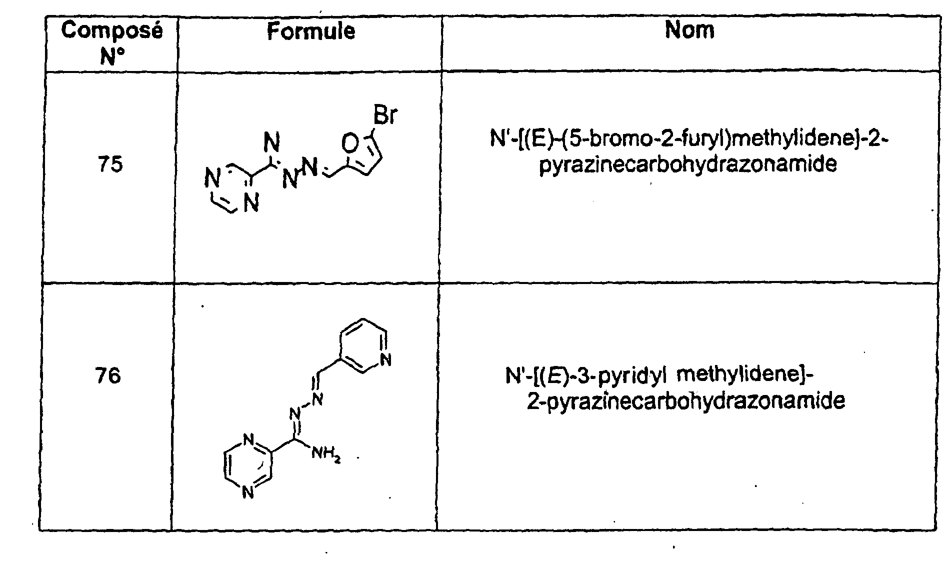

Les composés de formule (I) plus particulièrement préférés sont :
- le N'-[(E)-3-methoxyphenylmethylidene]benzenecarbohydrazonamide (Composé 29) ;
- le N'-[(E)-2-hydroxyphenylmethylidene]pyrazinecarbohydrazonamide (Composé 52) ;
- le N'-[(E)-3-methylphenylmethylidene]-2-pyrazinecarbohydrazonamide (Composé 76).

Parmi les composés selon l'invention numérotés de 1 à 76, la plupart sont nouveaux et constituent un autre objet de l'invention, certains d'entre eux sont connus tels que les composés suivants :
- le N'-[(E)-3-methoxyphenylmethylidene]benzenecarbohydrazonamide (Composé 29)
- le N'-[(E)-2-hydroxyphenylmethylidene]pyrazinecarbohydrazonamide (Composé 52)
- le N'-[(E)-1 H-pyrrol-2-ylmethylidene]-2-pyrazinecarbohydrazonamide (Composé 58)
- le N'-[(E)-2-furylmethylidene]-2-pyrazinecarbohydrazonamide (Composé 62)
- le N'-[(E)-(5-methyl-2-furyl)methylidene]-2-pyrazinecarbohydrazonamide (composé 66)
- le N'-[(E)-(5-nitro-2-furyl)methylidene]-2-pyrazinecarbohydrazonamide (Composé 73)
- le N'-[(E)-3-pyridyl methylidene]-2-pyrazinecarbohydrazonamide (Composé 76)

Notamment les composés 29, 52 et 76 sont décrits dans l'article *Drug. Des. Discovery* **1998**, 15(4), 269-275 et ont respectivement comme Registry Number (CAS.) : 221084-02-2 ; 31649-03-3 et 221083-80-3.

Les composés de formule (I) sont généralement présents dans la composition de l'invention dans des proportions comprises entre 0,1 % et 20 % en poids, de préférence entre 0,5 % et 10 % en poids, par rapport au poids total de la composition.

Les compositions cosmétiques antisolaires selon l'invention peuvent bien entendu contenir un ou plusieurs filtres organiques complémentaires actifs dans l'UVA et/ou l'UVB (absorbeurs), liposolubles ou hydrosolubles. Ces filtres organiques peuvent être notamment choisis parmi les dérivés cinnamiques ; les dérivés de dibenzoylméthane; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP 933376 ; les dérivés de la benzophénone ; les dérivés de β, β'-diphénylacrylate, les dérivés de benzimidazole; les dérivés bis-benzoazolyle tels que ceux décrits dans les brevets EP-A-0669323 et US 2,463,264 ; les dérivés de bisbis-hydroxyphénolbenzotriazole tels que ceux décrits dans les demandes US 5237 071, US 5 166 355, GB-A-2 303 549, DE 197 26 184 et EP-A-893 119; les dérivés de l'acide p-aminobenzoïque; les polymères hydrocarbonés filtres et les silicones filtres tels que ceux décrits notamment dans la demande WO-93/04665.

Comme exemples de filtres solaires complémentaires actifs dans l'UV-A et/ou l'UV-B, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-boman-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxobom-3-ylidène)-tolyl-4-sulfonique et ses sels
le 3-(4'-sulfo)benzylidèn-bornan-2-one et ses sels,
le 3-(4'méthylbenzylidène)-d,l-camphre,
le 3 -benzylidène-d,l-camphre,
l'acide benzène 1,4-di(3-méthylidène-10-camphosulfonique) et ses sels,
l'acide urocanique,
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxobom-3-ylidèn)méthyl] benzyl]-acrylamide,
l'acide 1,4-bisbenzimidazolyl-phénylen-3,3',5,5'-tétrasulfonique et ses sels.
les polyorganosiloxanes à fonction benzalmalonate
les polyorganosiloxanes à fonction benzotriazole tel que le Drometrizole Trisiloxane.
le 2,2'-méthylène-bis-[6-(2H-benzotriazol-2-yl )-4-(1,1,3,3-tetraméthylbutyl)phénol] sous forme soluble vendue sous le nom MIXXIM BB/100 par le société FAIRMOUNT CHEMICAL et sous forme insoluble micronisée sous le nom TINOSORB M ;
le 2,2'-méthylène-bis-[6-(2H-benzotriazol-2-yl)-4-(methyl)phénol] vendu sous le nom MIXXIM BB/200 par le société FAIRMOUNT CHEMICAL.

Les compositions selon l'invention peuvent également contenir des agents de bronzage et/ou de brunissage artificiels de (a peau (agents autobronzants), tels que par exempte de la dihydroxyacétone (DHA).

Les compositions selon l'invention peuvent encore contenir des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir ce ou ces éventuels composés complémentaires et/ou leurs quantités de manière telle que les propriétés avantageuses, attachées intrinsèquement aux dérivés d'aminoamidine conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (BANGHAM, STANDISH AND WATKINS. *J. Mol. Biol.* **1965**, 13, 238 ; FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation.

L'invention a encore pour objet l'utilisation d'un dérivé d'aminoamidine de formule (I) tel que défini précédemment pour la fabrication d'une composition destinée à protéger des matières sensibles au rayonnement ultraviolet, en particulier au rayonnement solaire.

L'invention a encore pour objet l'utilisation d'un dérivé d'aminoamidine de formule (I) tel que défini précédemment pour la fabrication d'une composition cosmétique ou dermatologique comme agent filtrant les radiations UV.

L'invention a encore pour objet l'utilisation d'un dérivé d'aminoamidine de formule (I) tel que défini précédemment pour la fabrication d'une composition cosmétique comme agent de contrôle de la variation de la couleur de la peau due aux rayonnement UV.

Les exemples qui suivent illustrent l'invention sans toutefois en limiter la portée.

### EXEMPLES

### EXEMPLE 1

### N'-[(E)-(3-methoxyphenyl)-methylidene]benzenecarbohydrazonamide (composé 29)

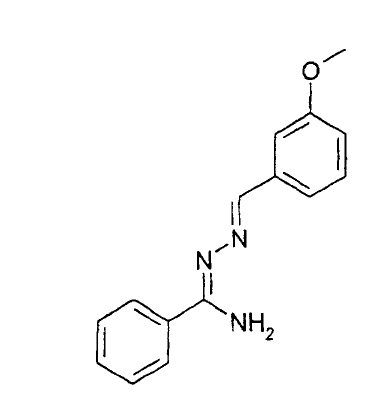

Ce composé est obtenu selon le procédé de synthèse en deux étapes défini ci-dessous à partir de chlorhydrate de l'éthylbenzimidate (cas : 5333-86-8) et de 3-méthoxybenzaldéhyde :

Le chlorhydrate de l'éthylbenzimidate est libéré par action de la soude aqueuse. L'huile obtenue (1g) est mise en solution dans l'éthanol (10ml) puis l'hydrate d'hydrazine (335 mg) est ajouté. Le milieu réactionnel est laissé une nuit à +4°C puis le solvant est évaporé, laissant l'aminoamidine attendue sous forme d'une huile. Cette huile est reprise dans 10 ml d'isopropanol et le 3-méthoxybenzaldéhyde est ajouté (910mg). Le milieu est porté au reflux et chauffé 2 heures. Le produit attendu précipite et est filtré.

RMN ¹H (400MHz, DMSO-d₆, ppm) : 8.43 (s, 1H) ; 7.97 (d, 2H) ; 7.54 (m, 1H) ; 7.49-7.41 (m, 4H) ; 7.34(t, 1H); 7.04(s élargi, 2H) ; 6.98 (dd, 1H) ; 3.83(s, 3H).
RMN ¹³C (100MHz, DMSO-d₆, ppm) : 159.4 ; 158.7 ; 153 ; 137 ; 133.8 ; 130.2 ; 129.5 ; 128; 126.7; 121.1 ; 120.6; 115.7; 111.9; 55.1
Masse moléculaire théorique : 237,3
Masse moléculaire déterminée à partir du spectre de masse des ions positifs enregistrés en ionisation electrospray sur un spectromètre de masse PLATFORM I (Micromass) : 238.

Ce composé présente les propriétés de filtration solaire suivantes :
λₘₐₓ=341nm (éthanol).
domaine spectral où ε est supérieur à 10.000 : 297 à 377nm.
E1% (éthanol) = 1016.

### EXEMPLE 2 :

N'-[(*E*)-3-methylphenylmethylidene]-2-pyrazinecarbohydrazonamide (composé N° 76)

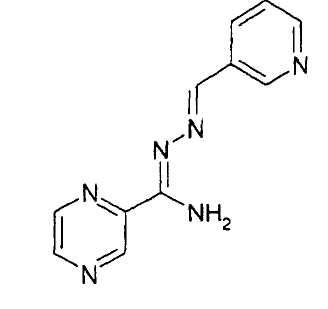

Ce composé a été synthétisé selon le mode opératoire décrit par FOKS H., M. BURACZEWSKA, W. MANOWSKA, J. SAWLEWICZ *Dissert. Pharm. Pharmacol.* **1971**, 23, (1), 49.
RMN ¹H (400MHz, DMSO-d₆, ppm) : 9.39 (d, *1H);* 9.08 (d, 1H) ; 8.78 (d, 1H); 8.74 (d, 1H) ; 8.61(dd, 1H) ; 8.57(s, 1H); 8.38 (ddd, 1H) ; 7.47(m, 1H) ; 7.30(s élargi, 2H).
RMN ¹³C (100MHz, DMSO-d₆, ppm) : 155.7 ; 152.2 ; 150.5 ; 149.4 ; 145.9 ; 145.7 143.3 ; 143 ; 134.4; 131 ; 123.6.
Masse moléculaire théorique : 224,3
Masse moléculaire déterminée à partir du spectre de masse des ions positifs enregistrés en ionisation electrospray sur un spectromètre de masse PLATFORM I (Micromass): 225.

Ce composé présente les propriétés de filtration solaire suivantes :
λₘₐₓ=341nm (éthanol).
domaine spectral où ε est supérieur à 10.000 : 297 à 377nm.
E1% (éthanol) = 1016. 6.

Perte de 5% après soumission au protocole de photostabilité (Selon BERST G., GONZENBACH H., CHRIST R., MARTIN R., DEFLANDRE A., MASCOTTO R. E., JOLLEY J. D. R., LOWELL W., PELZER R., STIEHM T., *Inter. J.* Cosm.Sci. 1996, 18, 167-177. Proposed protocol for determination of photostability Part 1 : cosmetic UV filters.).

### EXEMPLE 3 :

N'-[(E)-2-hydroxyphenylmethylidene]pyrazine carbohydrazonamide (composé 52)

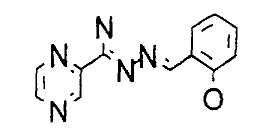

Ce composé a été synthétisé selon le mode opératoire suivant :
La 3-cyanopyrazine (15g) est mise en solution dans 30 mL d'éthanol puisl'hydrate d'hydrazine (15 mL) est ajouté. Le milieu est porté au reflux puis le chauffage est arrété. L'aminoamidine attendue précipite et est filtrée. Cette aminoamidine est ensuite utilisée comme dans le mode opératoire de l'exemple 1.
Masse moléculaire théorique : 241,3
Masse moléculaire déterminée à partir du spectre de masse des ions positifs enregistrés en ionisation electrospray sur un spectromètre de masse PLATFORM I (Micromass). :242.

Ce composé présente les propriétés de filtration suivante :
λₘₐₓ= 354,2 nm (éthanol).
domaine spectral où est supérieur à 10.000 : 320 à 384nm.
E1% (éthanol) = 1010.

### EXEMPLE 4 : N'-[(E)-3-furylmethylidenel-2-pyrazinecarbohydrazonamide (composé 70)

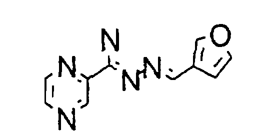

Ce composé a été synthétisé selon le mode opératoire décrit par FOKS H., M. BURACZEWSKA, W. MANOWSKA, J. SAWLEWICZ *Dissert. Pharm. Pharmacol.* **1971**, 23, (1), 49.

RMN ¹H (500MHz, DMSO-d₆, ppm) : 9.36 (d, 1H) ; 8.76 (dd, 1H); 8.71 (dd, 1H) ; 8.44(s, 1H) ; 8.13(s élargi, 1H) ; 7.75(s élargi, 1H) ; 7.06(m, 1H) ; 7.02(s élargi, 2H). Masse moléculaire théorique : 215.2
Masse moléculaire déterminée à partir du spectre de masse des ions positifs enregistrés en ionisation electrospray sur un spectromètre de masse PLATFORM 1 (Micromass) : 216.

Ce composé présente les propriétés de filtration solaire suivantes :
λₘₐₓ=335nm (éthanol).
domaine spectral où ε est supérieur à 10.000 : 297 à 364nm.
E1% (éthanol) = 832.

### EXEMPLES 5 A 33 :

Tous les composés des exemples 5 à 33 qui sont décrits dans le tableau ci-dessous ont été synthétisés selon le mode opératoire décrit dans l'exemple 1. Les masses moléculaires des différents composés ont été déterminées à partir des spectres de masse des ions positifs enregistrés en ionisation electrospray sur un spectromètre de masse PLATFORM I (Micromass). Leurs spectres d'absorption ont été réalisés dans les mêmes conditions que l'exemple 1.

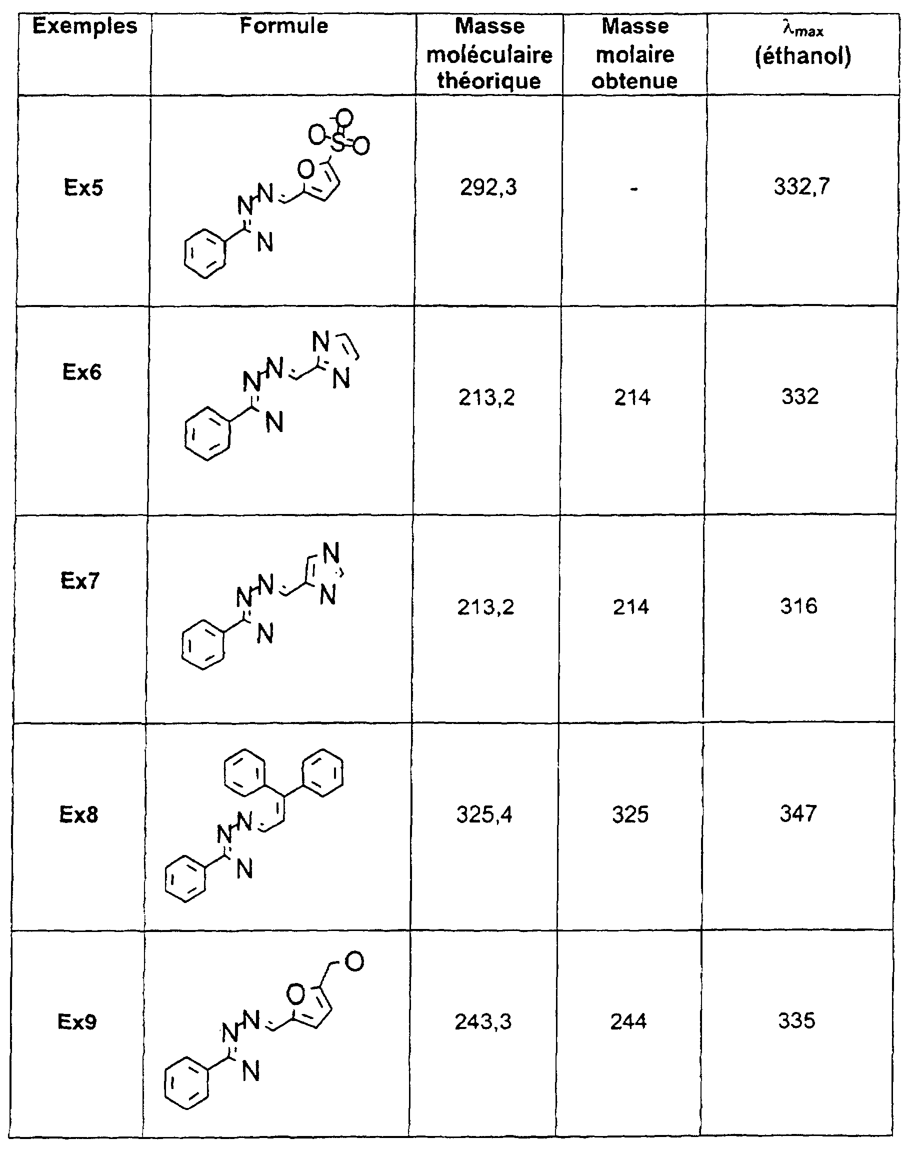

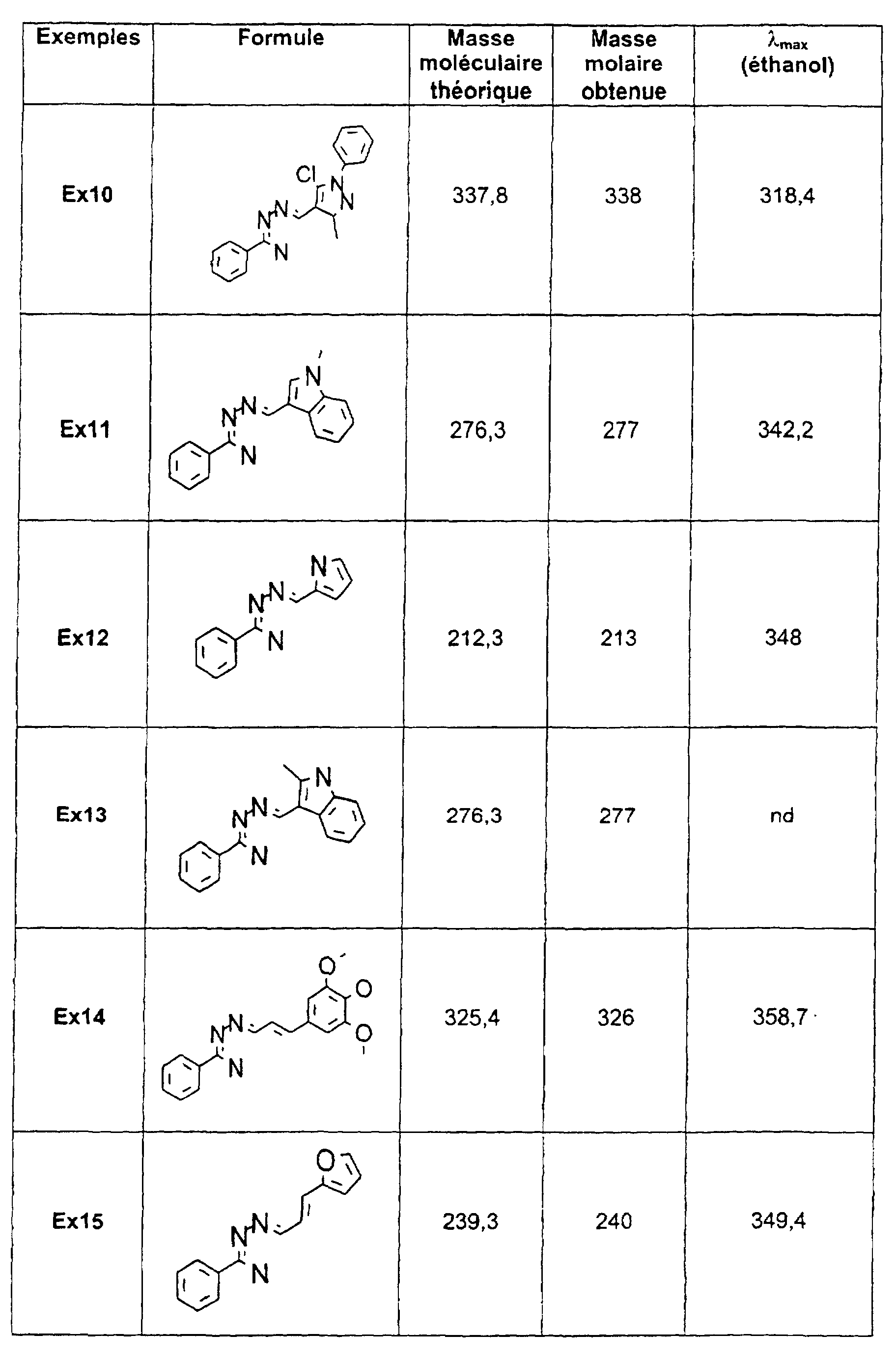

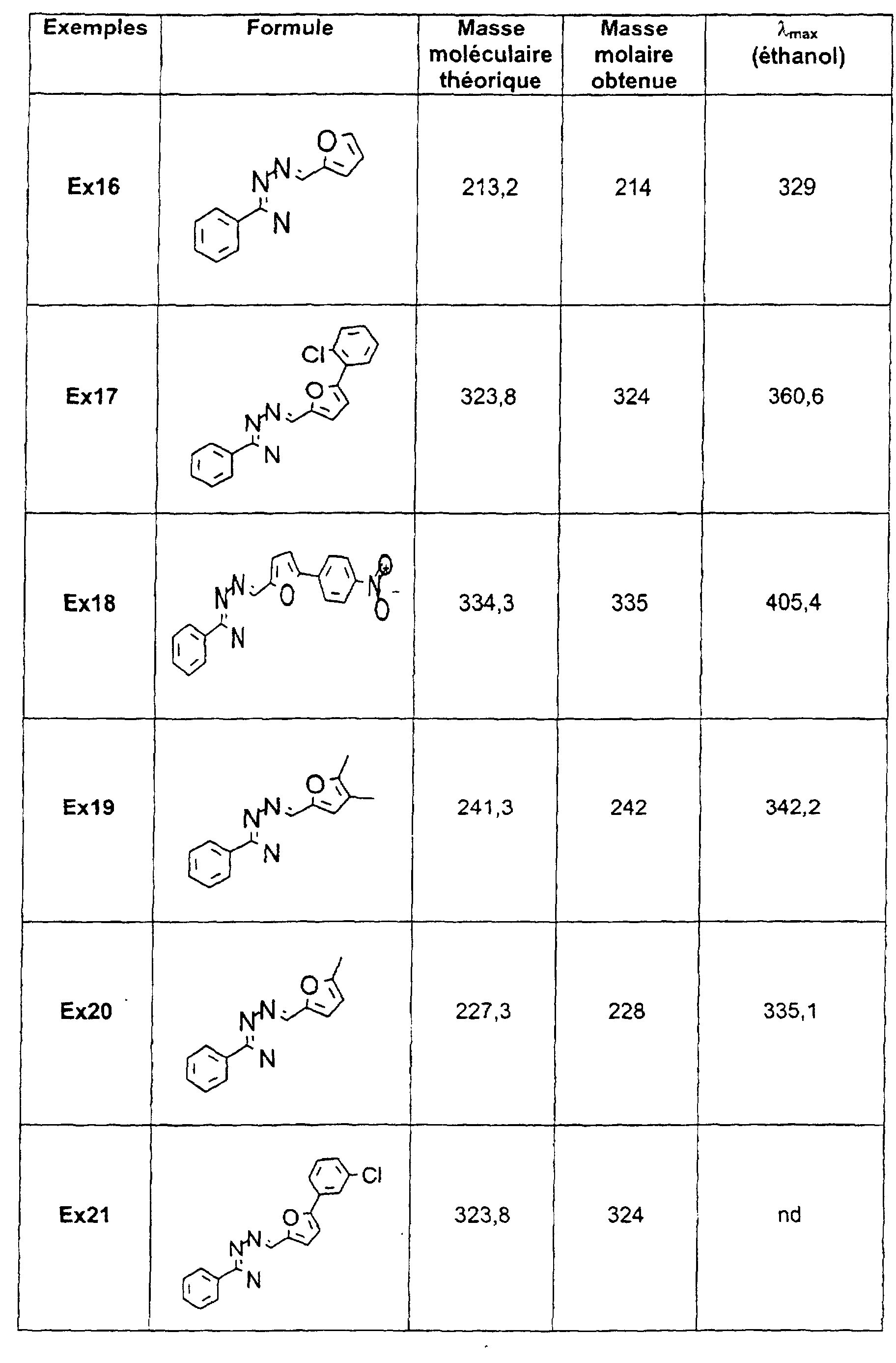

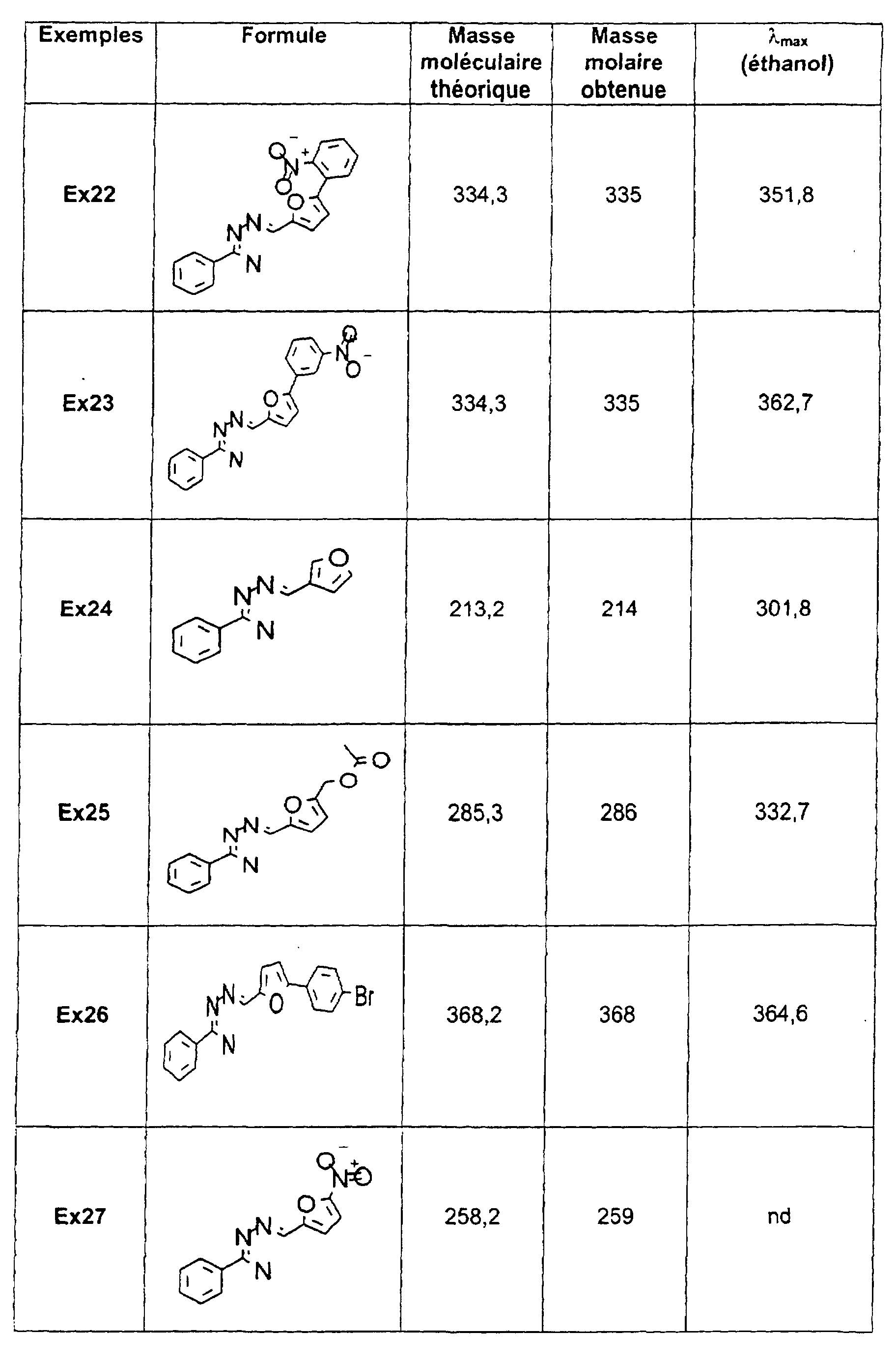

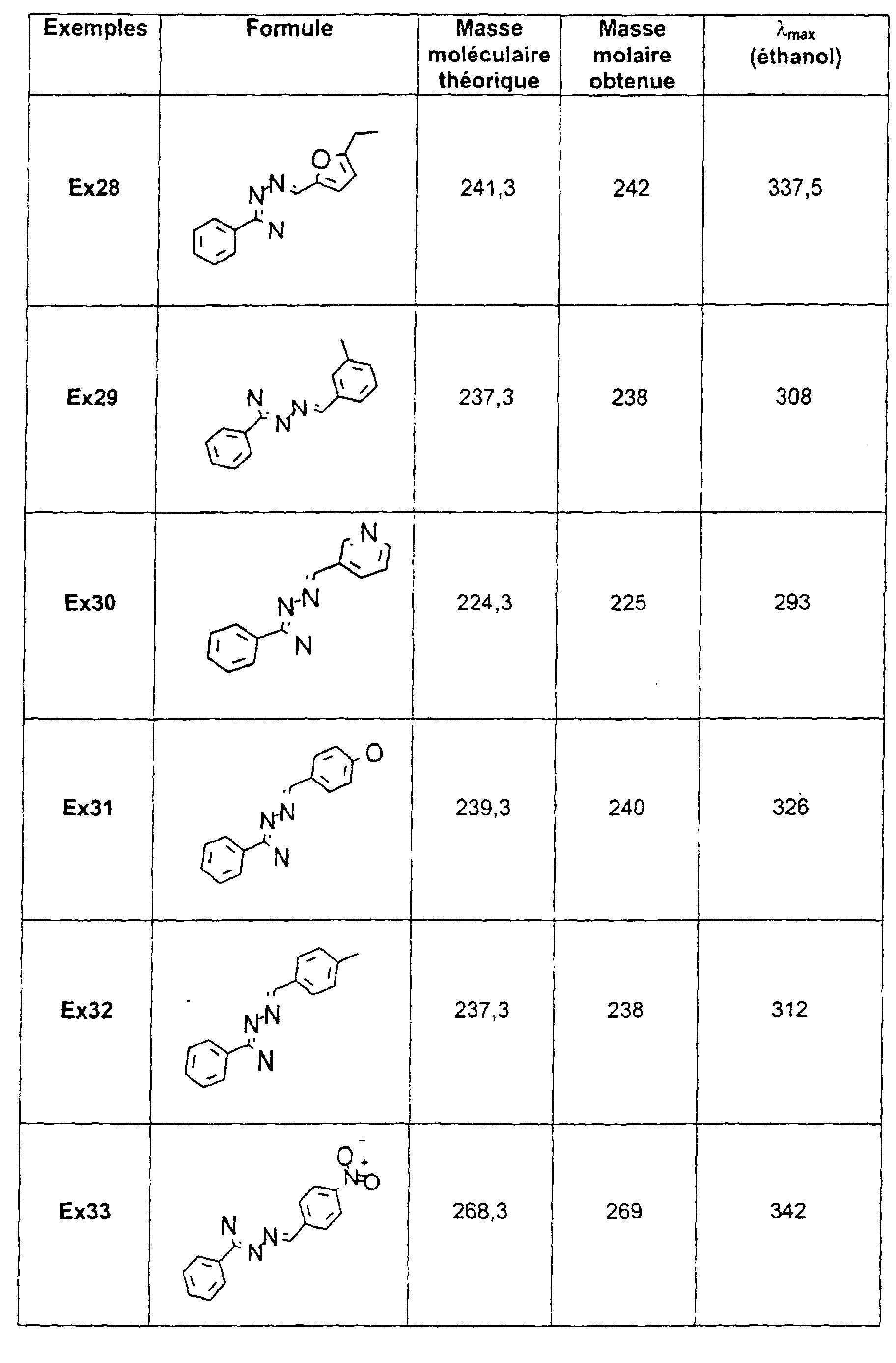

### EXEMPLES 34 A 53 :

Tous les composés des exemples 34 à 53 qui sont décrits dans le tableau ci-dessous ont été synthétisés selon le mode opératoire décrit dans l'exemple 1 en partant de l'éthyl-4-hydroxybenzimidate . Les masses moléculaires des différents composés ont été déterminées à partir des spectres de masse des ions positifs enregistrés en ionisation electrospray sur un spectromètre de masse PLATFORM I (Micromass). Leurs spectres d'absorption ont été réalisés dans les mêmes conditions que l'exemple 1.

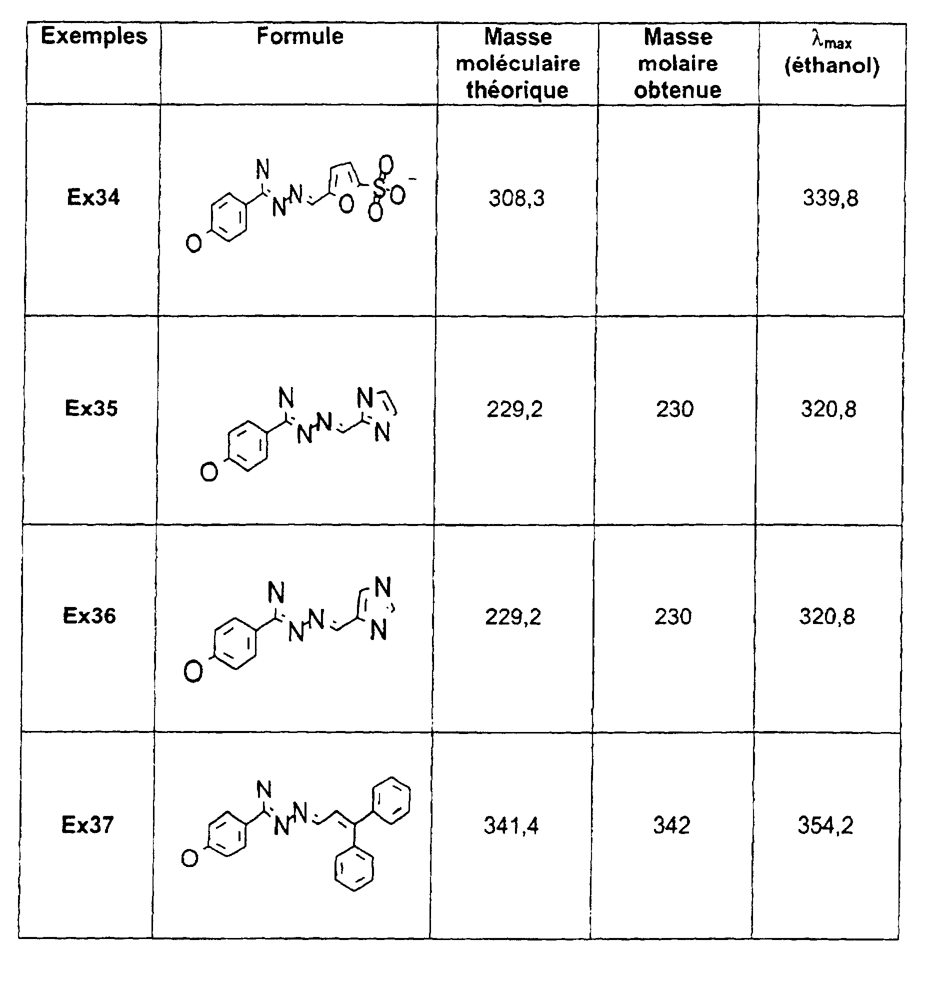

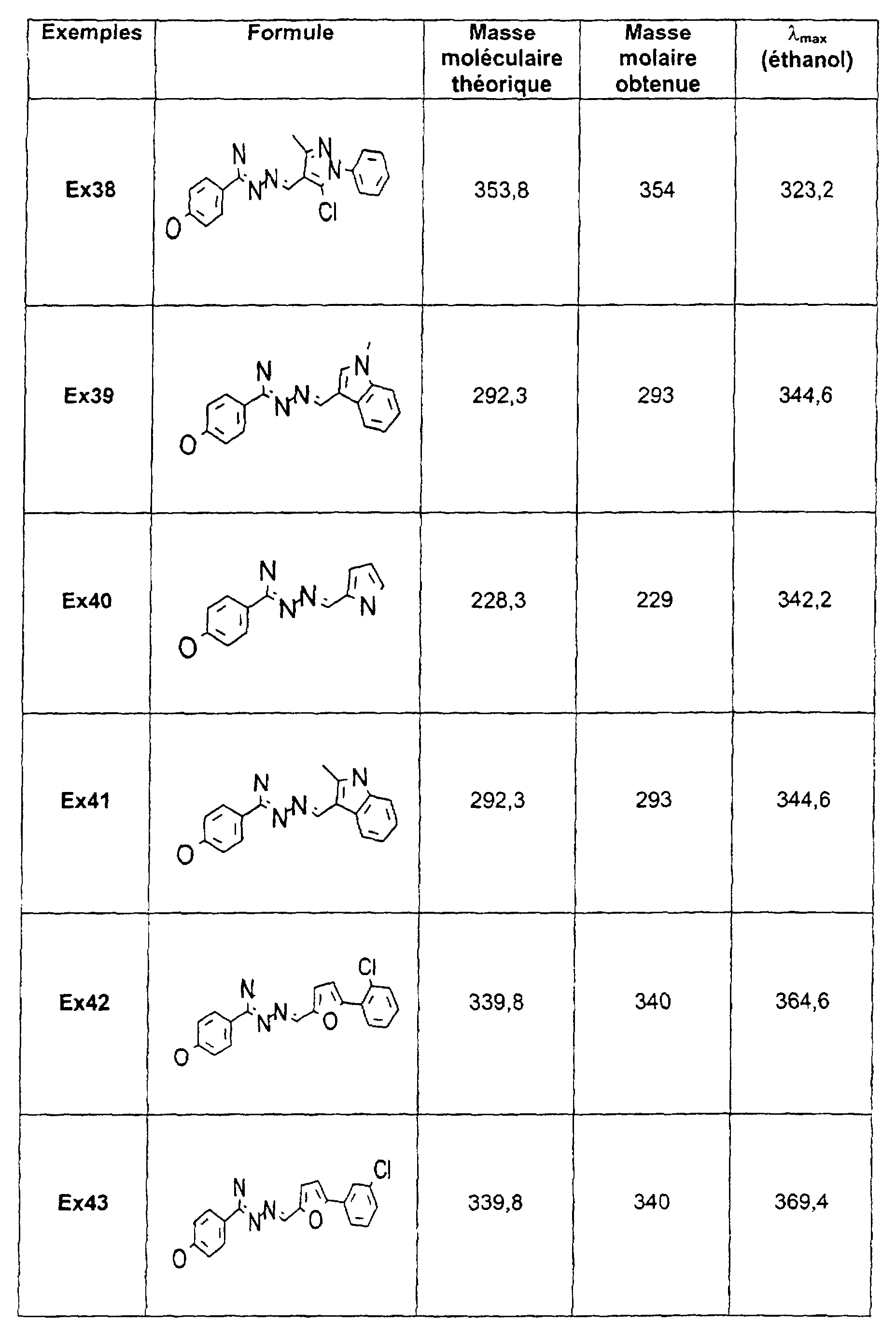

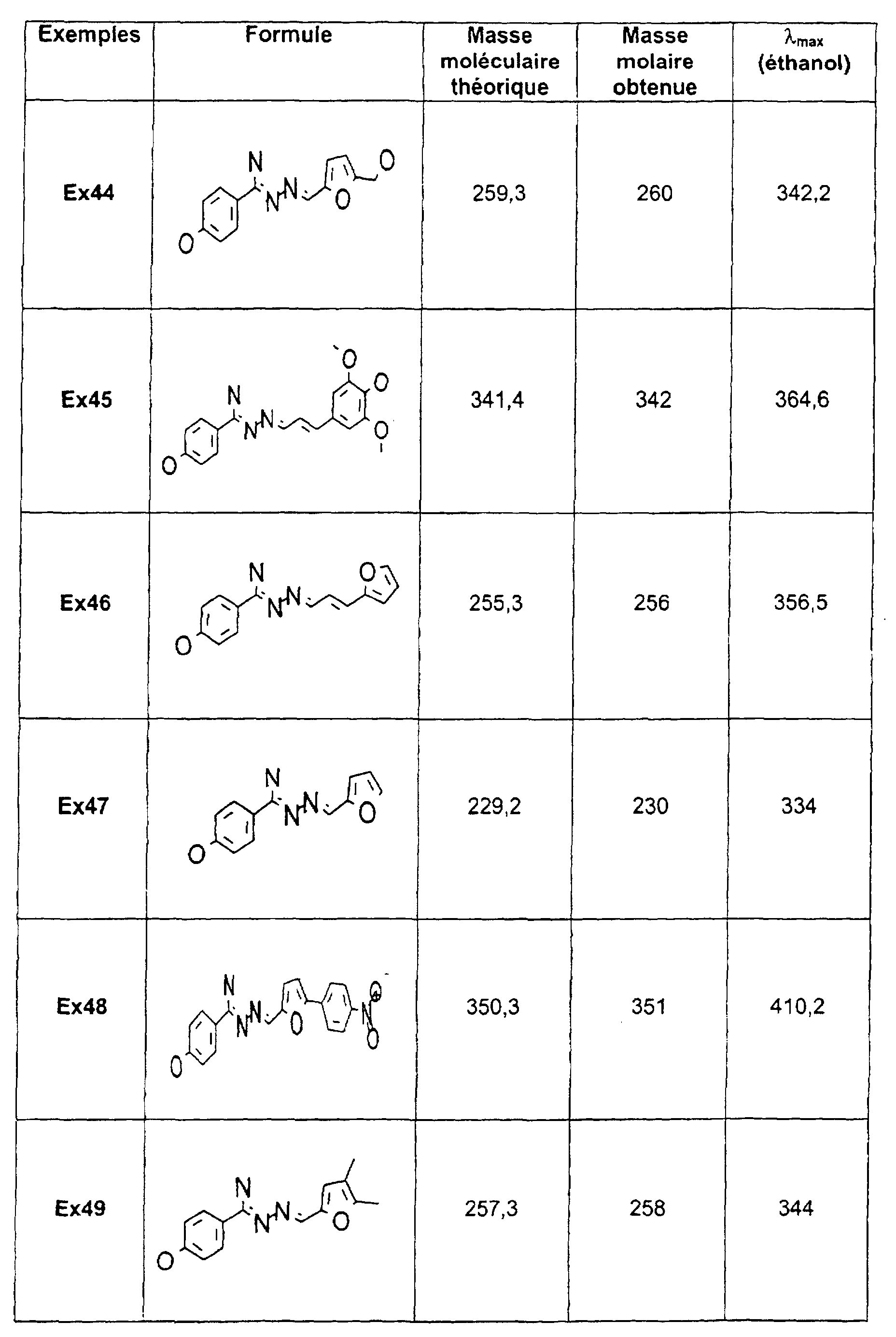

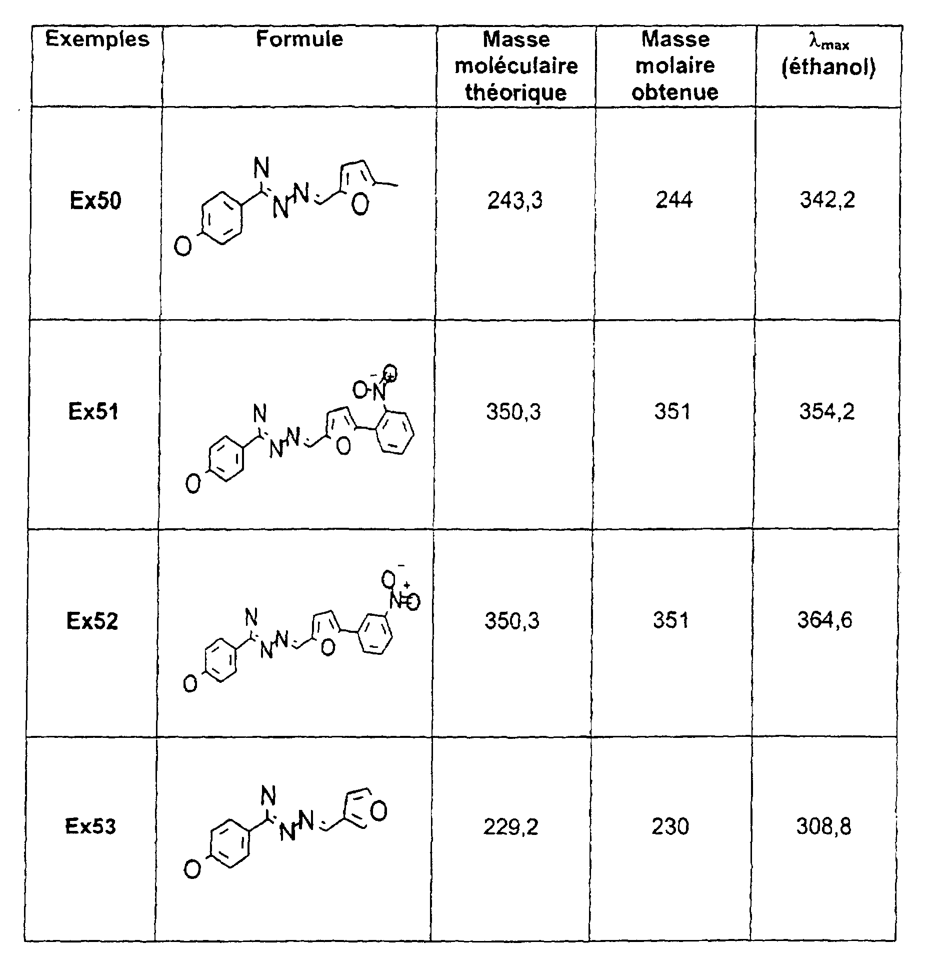

### EXEMPLES 54 A 76 :

Tous les composés des exemples 54 à 76 qui sont décrits dans le tableau ci-dessous ont été synthétisés selon le mode opératoire décrit dans l'exemple 3.

Les masses moléculaires des différents composés ont été déterminées à partir des spectres de masse des ions positifs enregistrés en ionisation electrospray sur un spectromètre de masse PLATFORM I (Micromass). Leurs spectres d'absorption ont été réalisés dans les mêmes conditions que l'exemple 1.

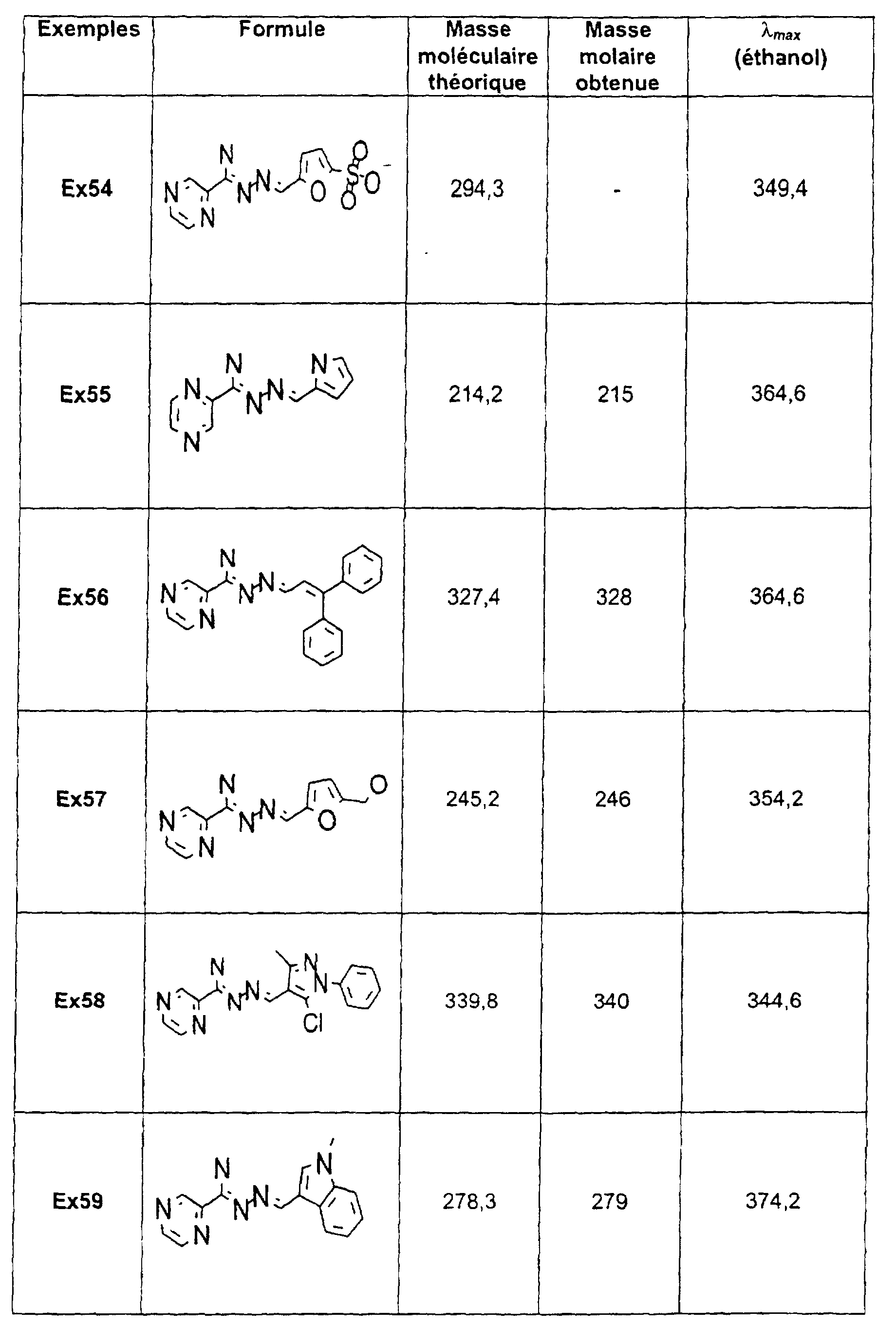

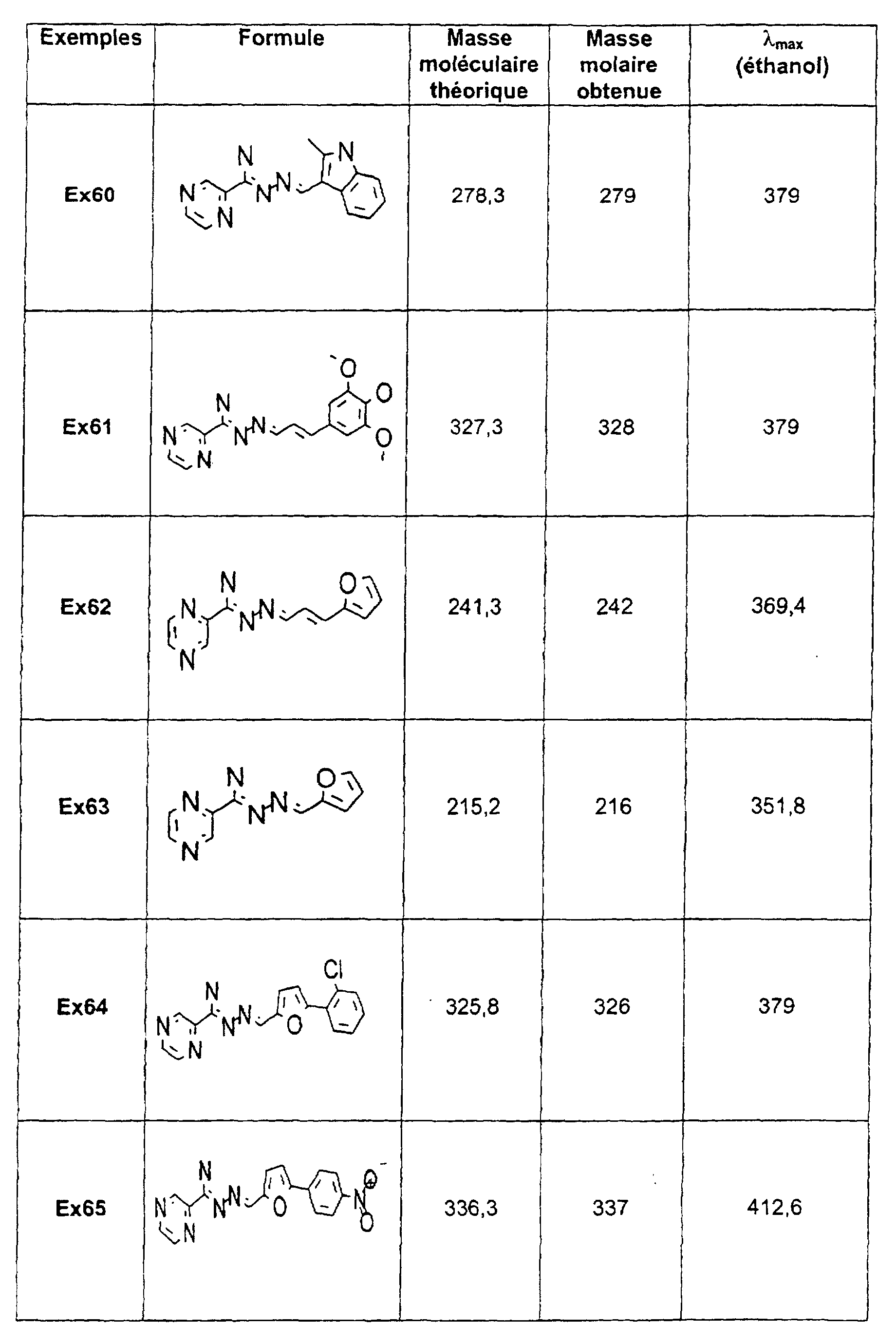

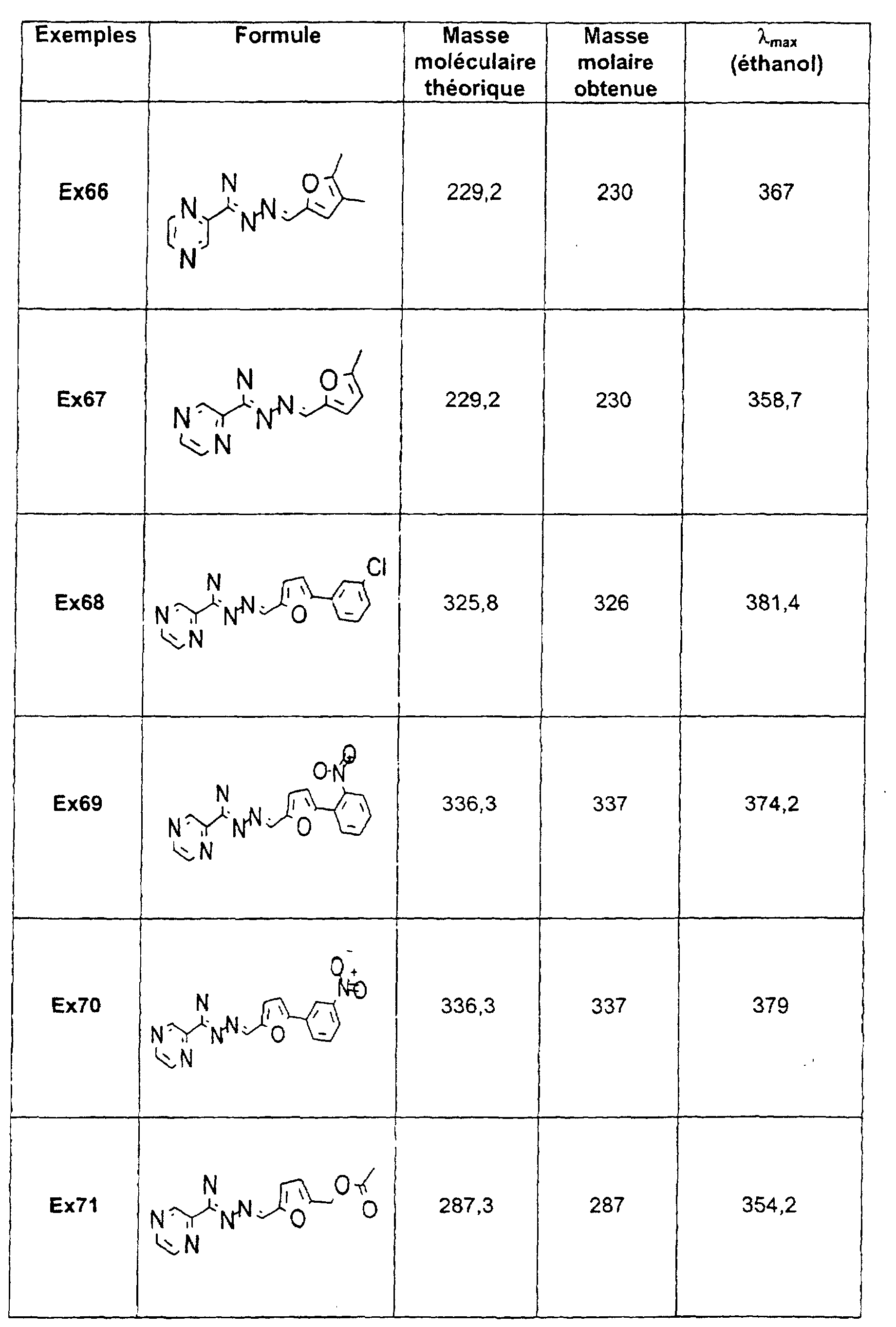

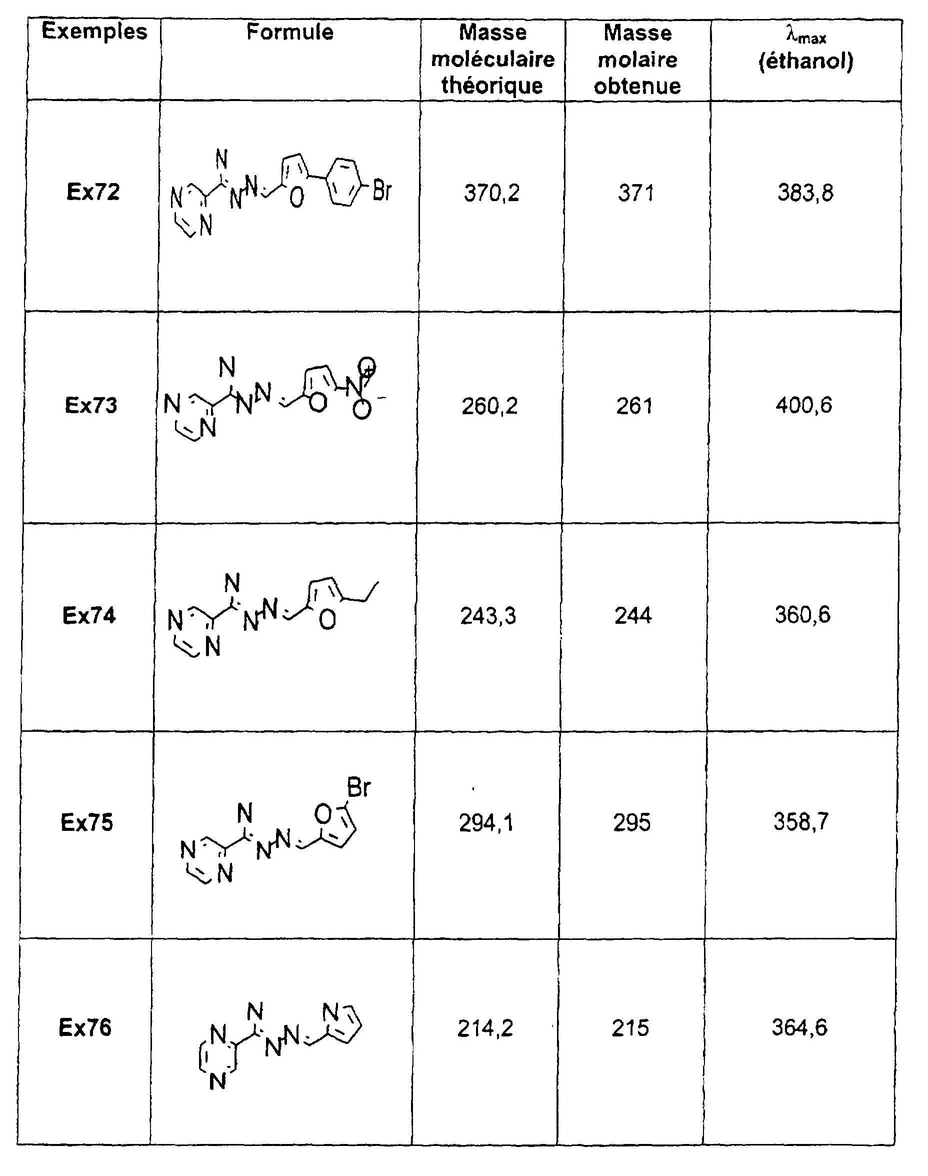

**EXEMPLE DE COMPOSITION 1**

| **COMPOSITION** | **% en poids** |
|---|---|
| Mélange d'alcool cétylstéarylique et d'alcool cétylstéarylique oxyéthyléné (33OE) 80/20 (SINNOWAX AO -HENKEL | 7 |
| Mélange de mono et distéarate de glycérol (CERASYNT SD-V ISP) | 2 |
| Alcool cétylique | 15 |
| Polydiméthyl siloxane (DOW CORNING 200 FLUID -DOW CORNING) | 1 |
| Benzoate d'alcools en C12/C15 (WITCONOL TN -WITCO) | 1.5 |
| Octyl methoxycinnamate (Parsol MCX de GIVAUDAN) | 3 |
| N'-[(*E*)-3-methylphenylmethylidene]-2-pyrazinecarbohydrazonamide (Composé n° 76 tel que préparé dans l'exemple 2) | 4 |
| Butyl methoxydibenzoyl méthane (Parsol 1789 ; GIVAUDAN) | 2 |
| Glycérine | 15 |
| Triéthanolamine | qs pH 7 |
| Conservateurs | qs |
| Eau déminéralisée qsp | 100 |

### EXEMPLE DE COMPOSITION 2

| **COMPOSITION** | **% en poids** |
|---|---|
| Mélange mono /distéarate de glycérol / stéarate de polyéthylène glycol (100 OE) (ARLACEL 165 FL - ICI) | 2 |
| Alcool stéarylique (LANETTE 18 - HENKEL) | 1 |
| Acide stéarique d'huile de palme (STÉARINE TP - STEARINERIE DUBOIS) | 2.5 |
| Poly dimethylsiloxane (DOW CORNING 200 FLUlD - DOW CORNING) | 0.5 |
| Benzoate d'alcools en C121C15 (WITCONOL TN -WITCO) | 20 |
| Triethanolamine | 0.5 |
| Glycérine | 5 |
| Phosphate d'alcool hexadecylique,sel de potassium (AMPHISOL K - HOFFMAN LAROCHE) | 1 |
| Acide polyacrylique (SYNTHALEN K - 3V) | 0.3 |
| Hydroxypropyl méthyl cellulose (METHOCEL F4M -DOW CHEMICAL) | 0.1 |
| Butyl méthoxydibenzoyl méthane (Parsol 1789 ; GIVAUDAN) | 2 |
| N'-[(E)-3-furylméthylidene]-2-pyrazinecarbohydrazonamide (Composé n°70 tel que préparé dans l'exemple 4) | 4 |
| Octyl méthoxycinnamate (Parsol MCX de GIVAUDAN) | 3 |
| Triéthanolamine | qs pH 7 |
| Conservateurs | qs |
| Dioxyde de titane (MT100T de TAYCA) | 5 |
| Eau déminéralisée qsp | 100 |

## Revendications

1. Composition cosmétique à usage topique, en particulier pour la photoprotection de la peau et/ou des cheveux, **caractérisée par le fait qu'**elle comprend, dans un support cosmétiquement acceptable au moins un dérivé d'aminoamidine répondant à la formule (I) suivante : 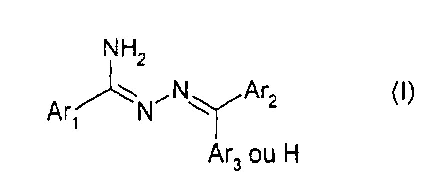 dans laquelle :
- Ar₁ représente
(i) un noyau aromatique de formule suivante : 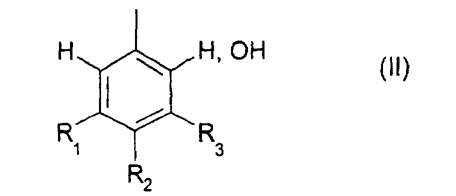 où R₁, R₂, R₃ identiques ou différents, désignent un atome d'hydrogène ; un radical alkyle en C₁-C₈ linéaire ou ramifié ; un radical alkoxy en C₁-C₈ ;
R₁, R₂, R₃ peuvent former un cycle aromatique ou non fusionné ou bien un hétérocycle aromatique à 5 ou 6 chaînons.
(ii) un noyau aromatique à 6 atomes répondant à la structure suivante : 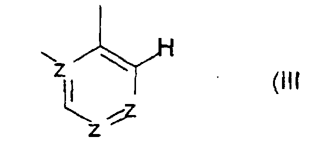 où Z désigne C ou N ;
- Ar₂ représente un noyau aromatique répondant à la définition de Ar₁ telle que définie précédemment ou bien un noyau aromatique à 5 atomes répondant à la structure suivante : 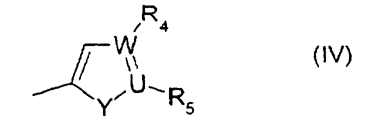 dans laquelle :
- Y représente un atome de soufre, d'oxygène, de carbone ou d'azote;
- W représente un atome de carbone ou d'azote ;
- R₄ désigne :
(i) un atome d'hydrogène lorsque W est un atome d'azote ;
- (ii) R₄ désigne un atome d'hydrogène, un radical alkyle en C₁-C₈ linéaire ou ramifié ; un radical alkoxy en C₁-C₈ ou peut former un cycle aromatique ou non fusionné , lorsque W est un atome de carbone ;
- U représente un atome de carbone ou d'azote, lorsque W est un atome de carbone ;
- R₅ désigne :
(i) un atome d'hydrogène lorsque U désigne azote;
(ii) un atome d'hydrogène ; un radical alkyle en C₁-C₈ linéaire ou ramifié ; un radical alkoxy en C₁-C₈ ou peut former un cycle aromatique ou non fusionné, lorsque U est un atome de carbone ;
- Ar₃ désigne un noyau aromatique répondant à la définition de Ar₂ telle que définie précédemment ou bien représente un atome d'hydrogène.

2. Composition selon la revendication 1, où le dérivé d'aminoamidine est choisi dans le groupe constitué par les composés définis dans le tableau suivant :

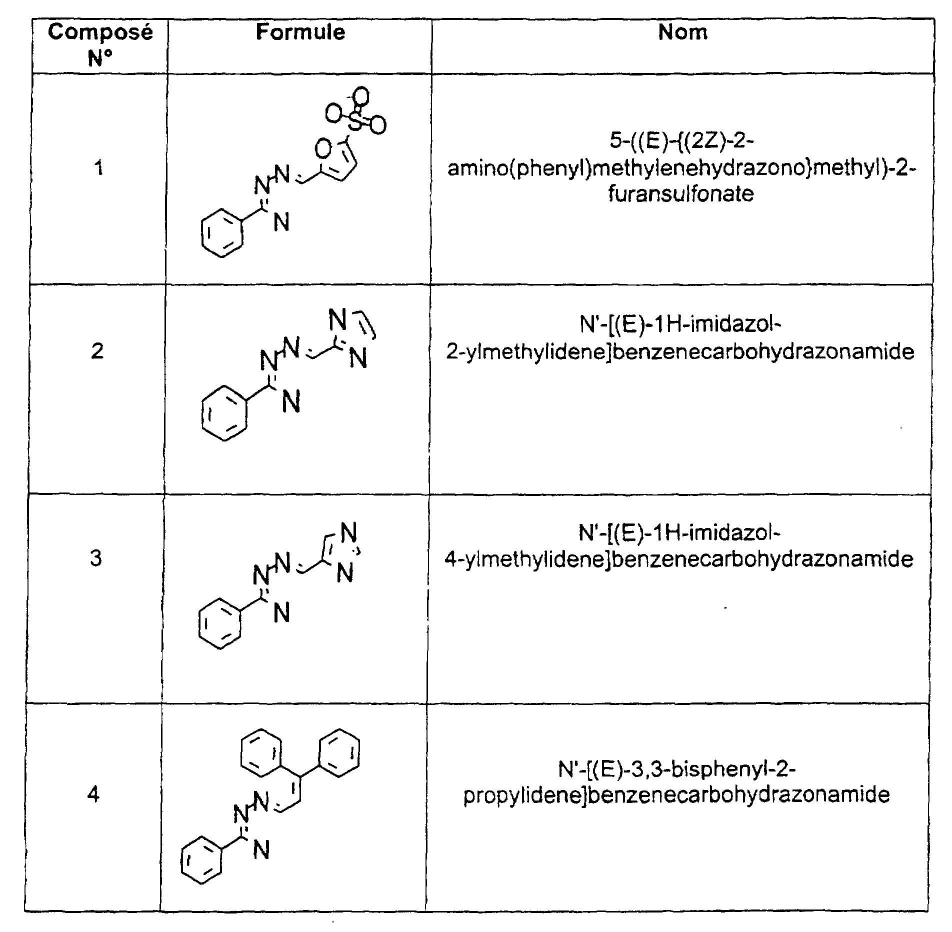

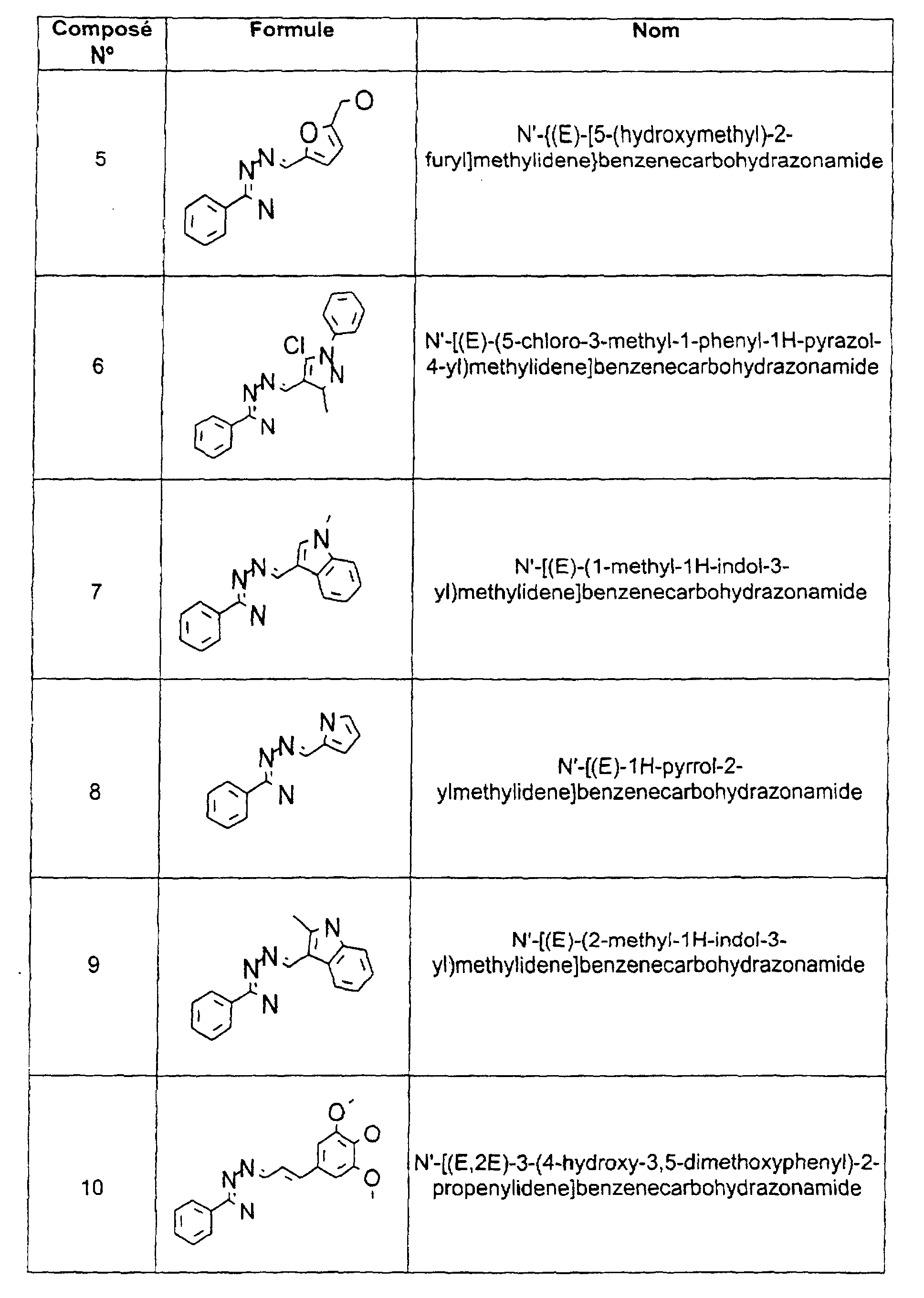

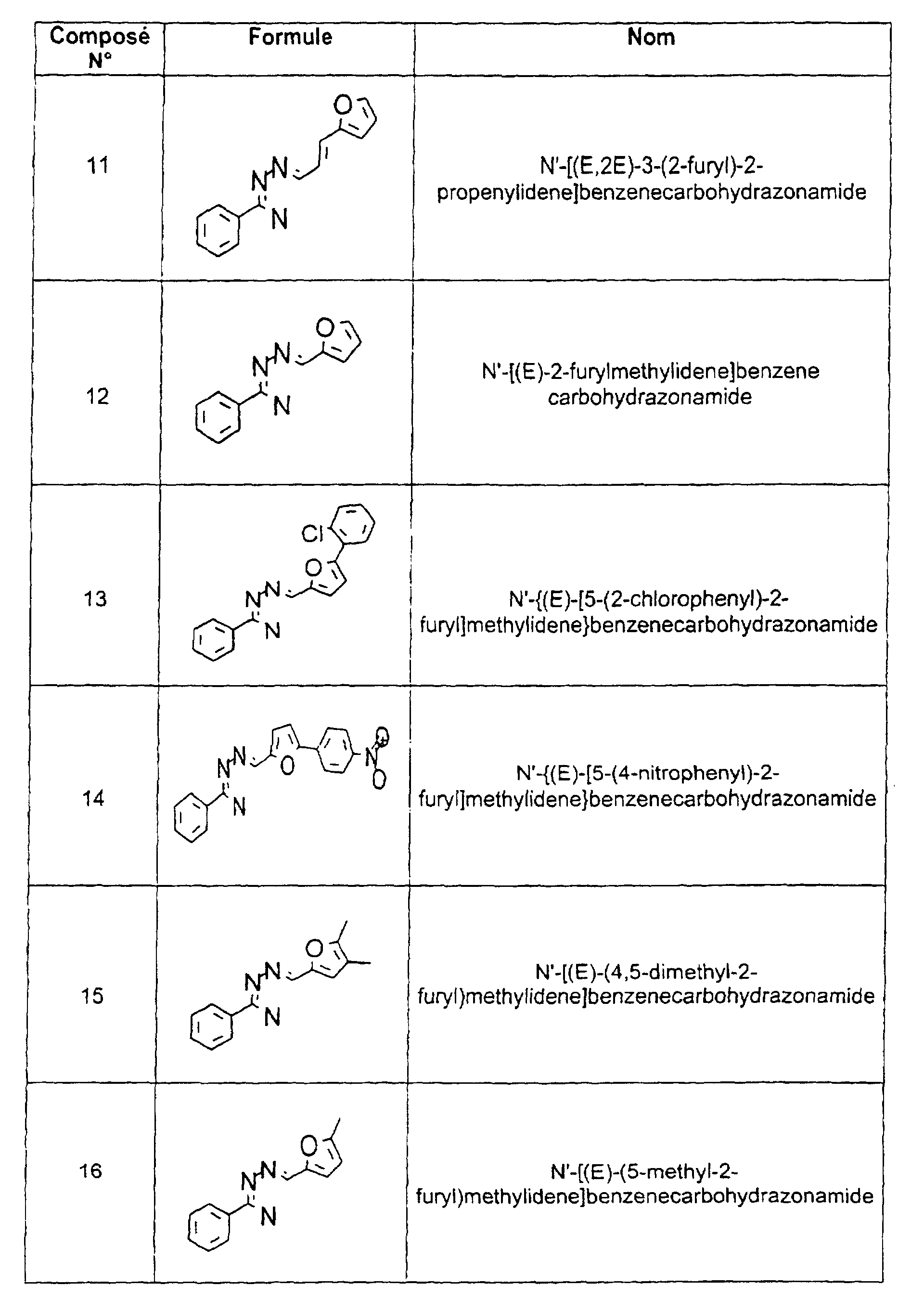

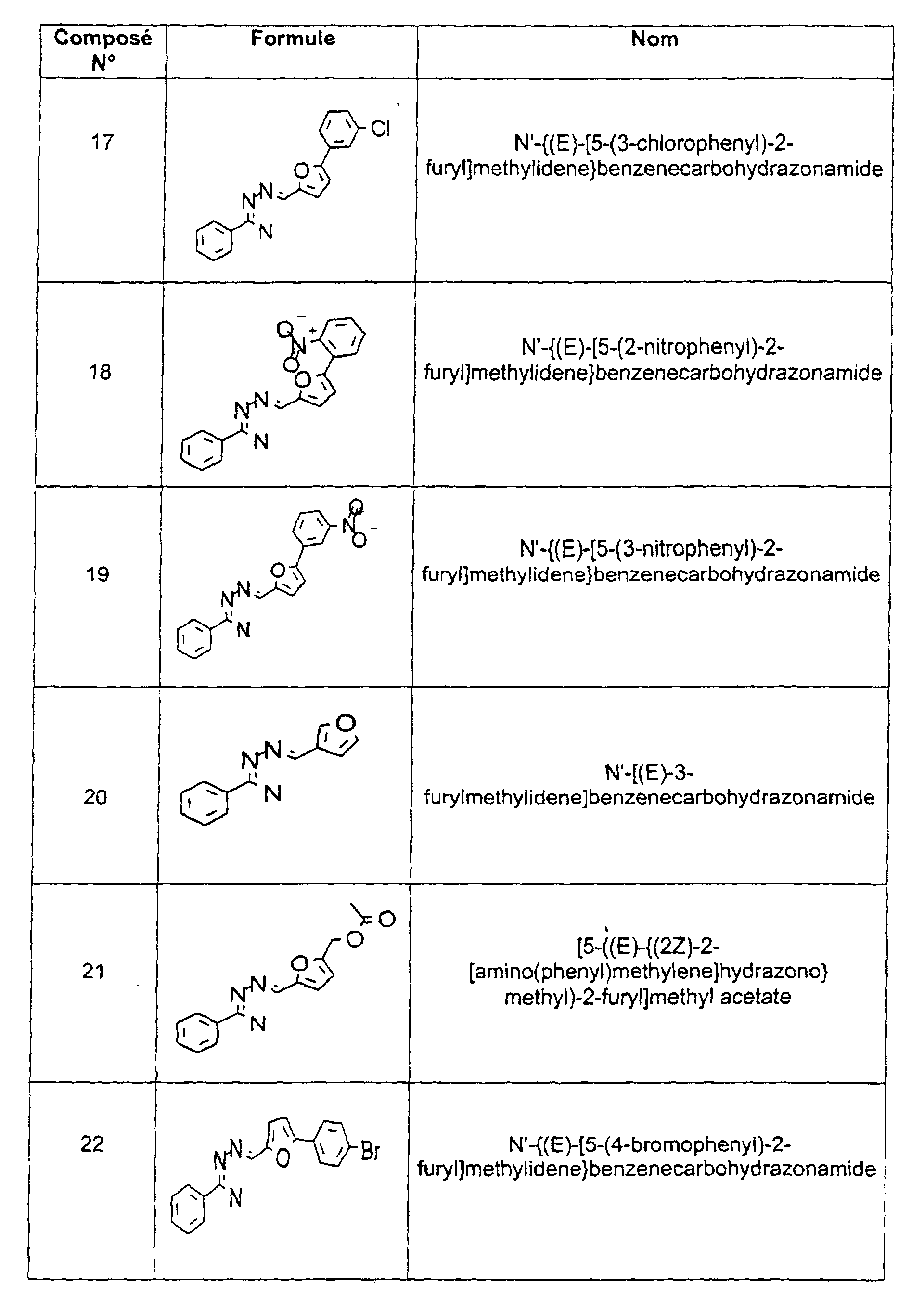

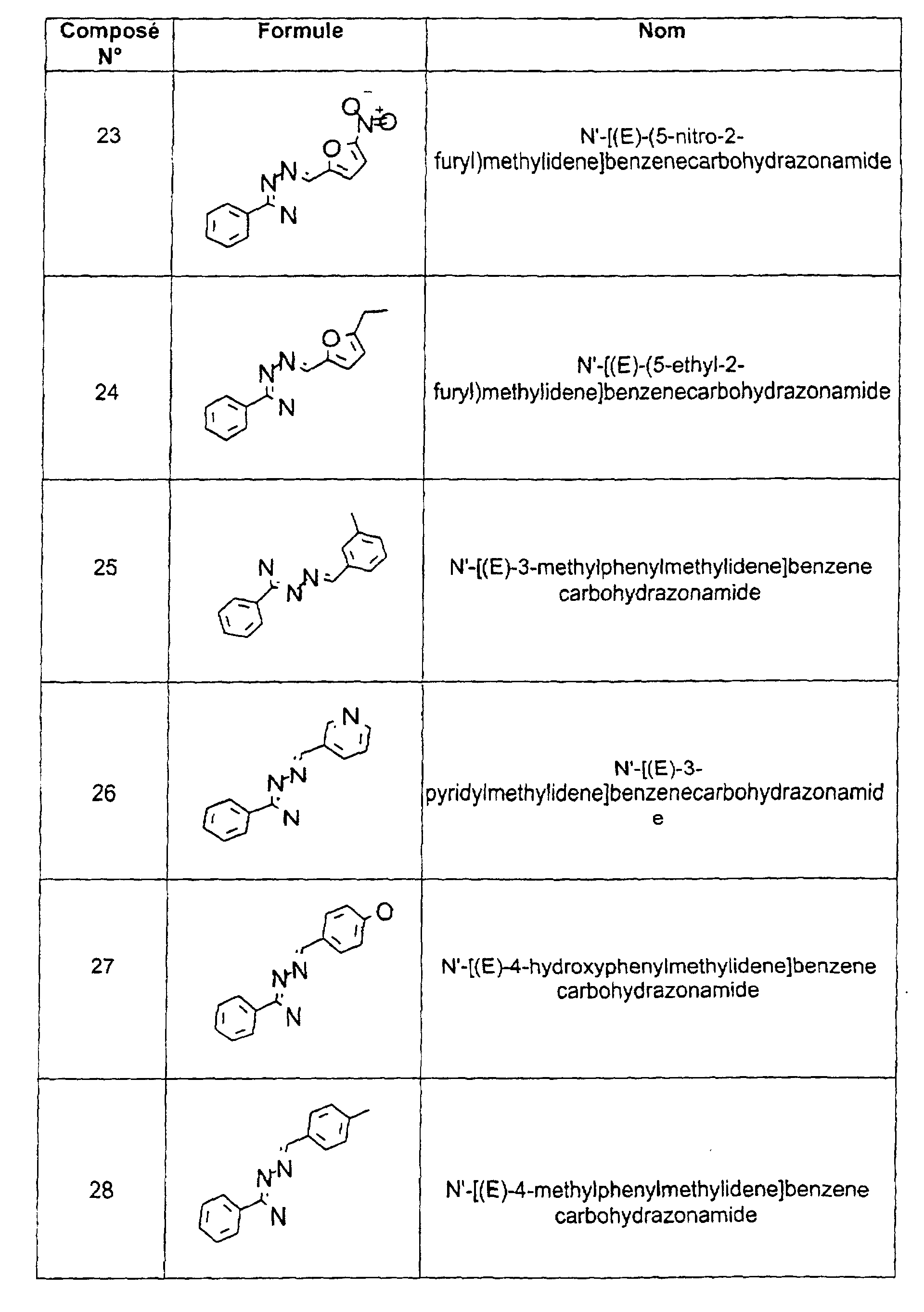

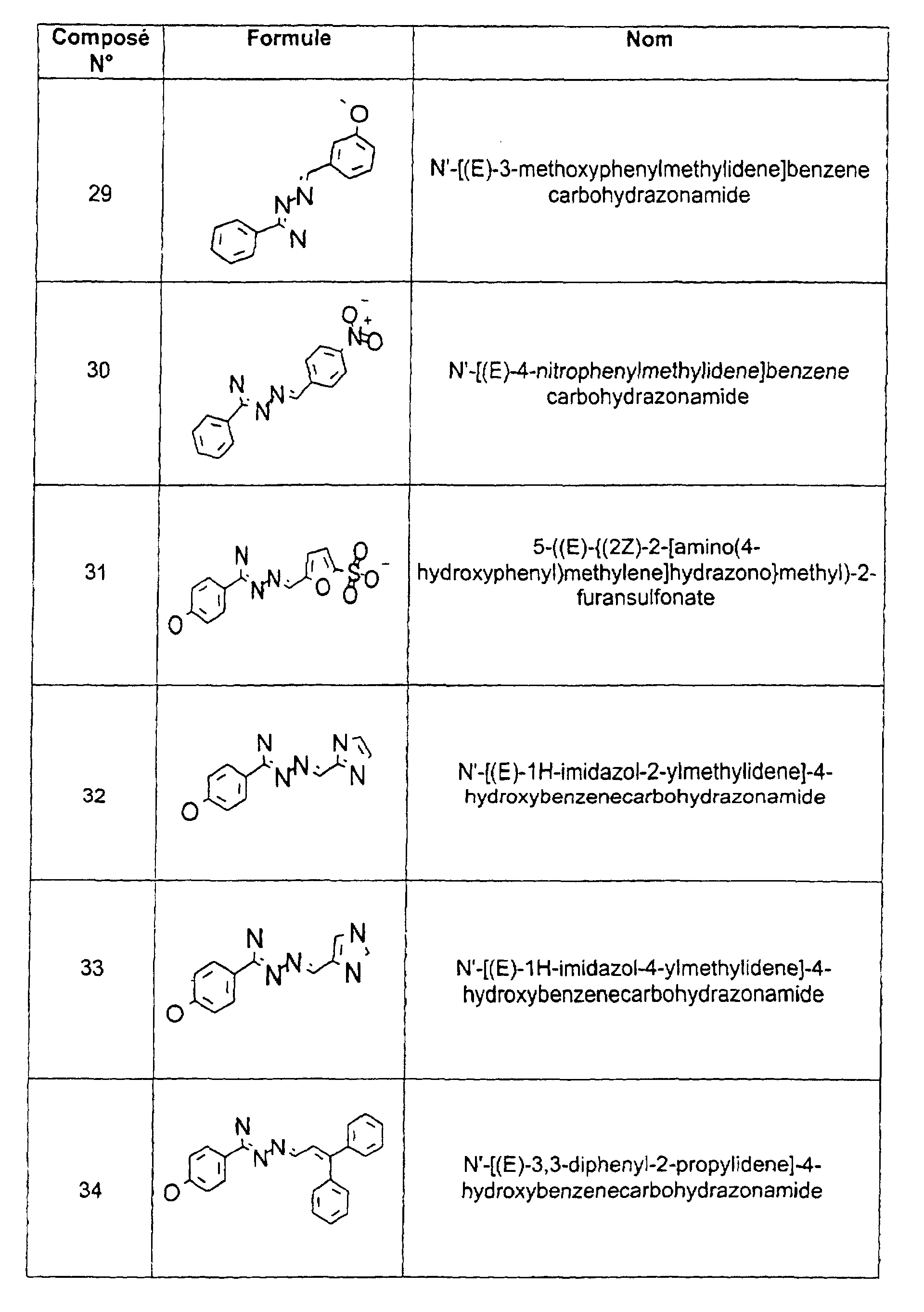

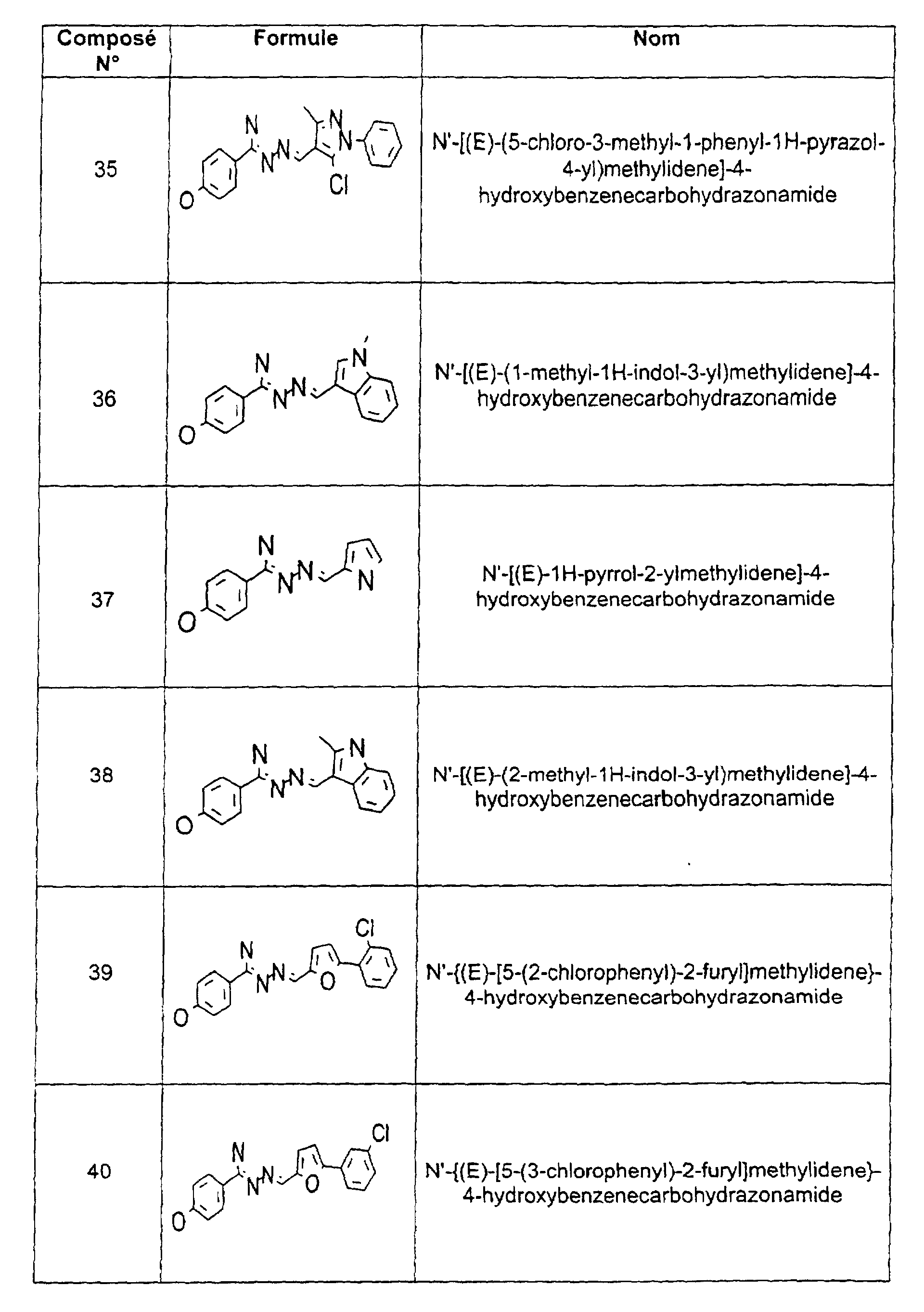

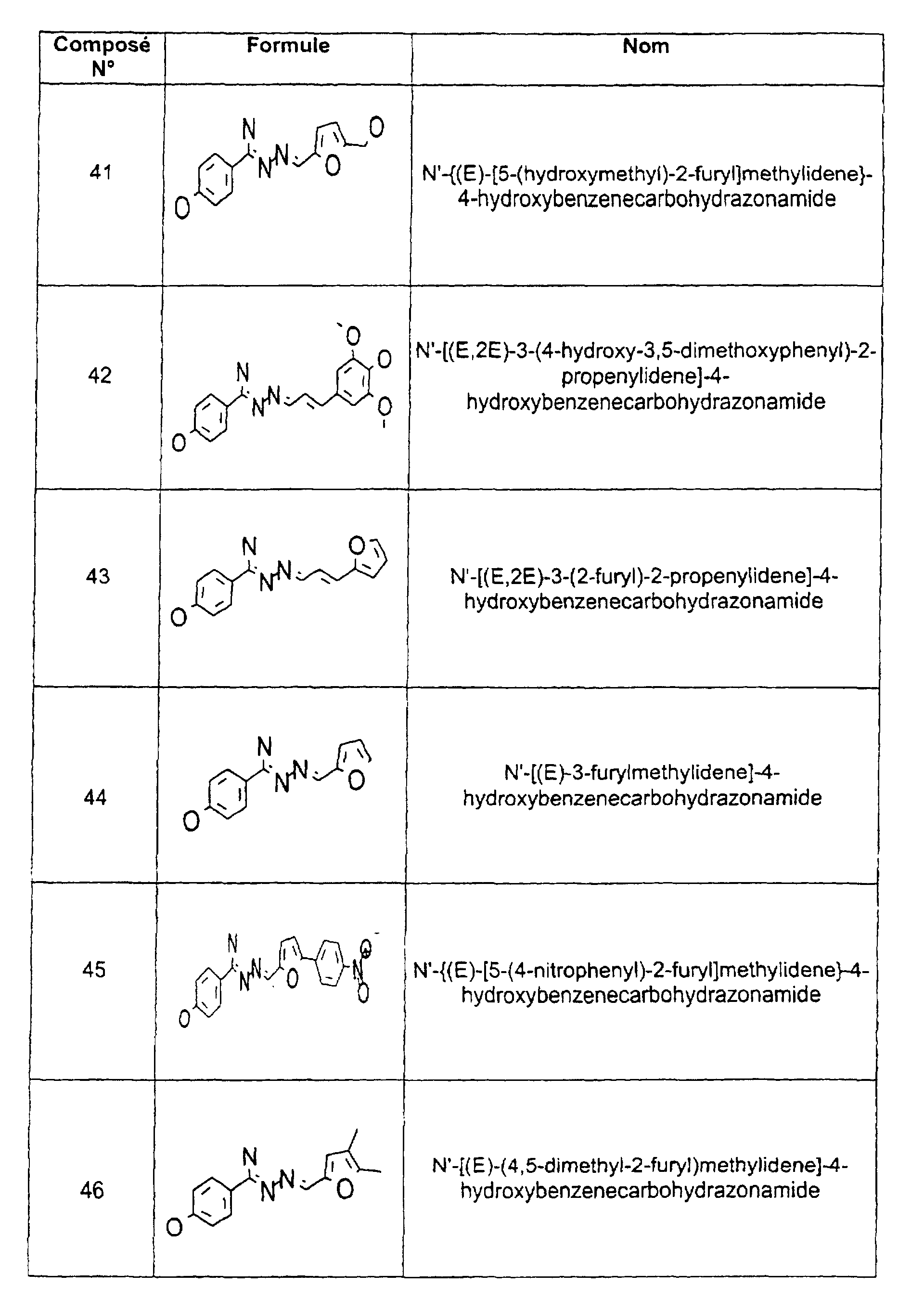

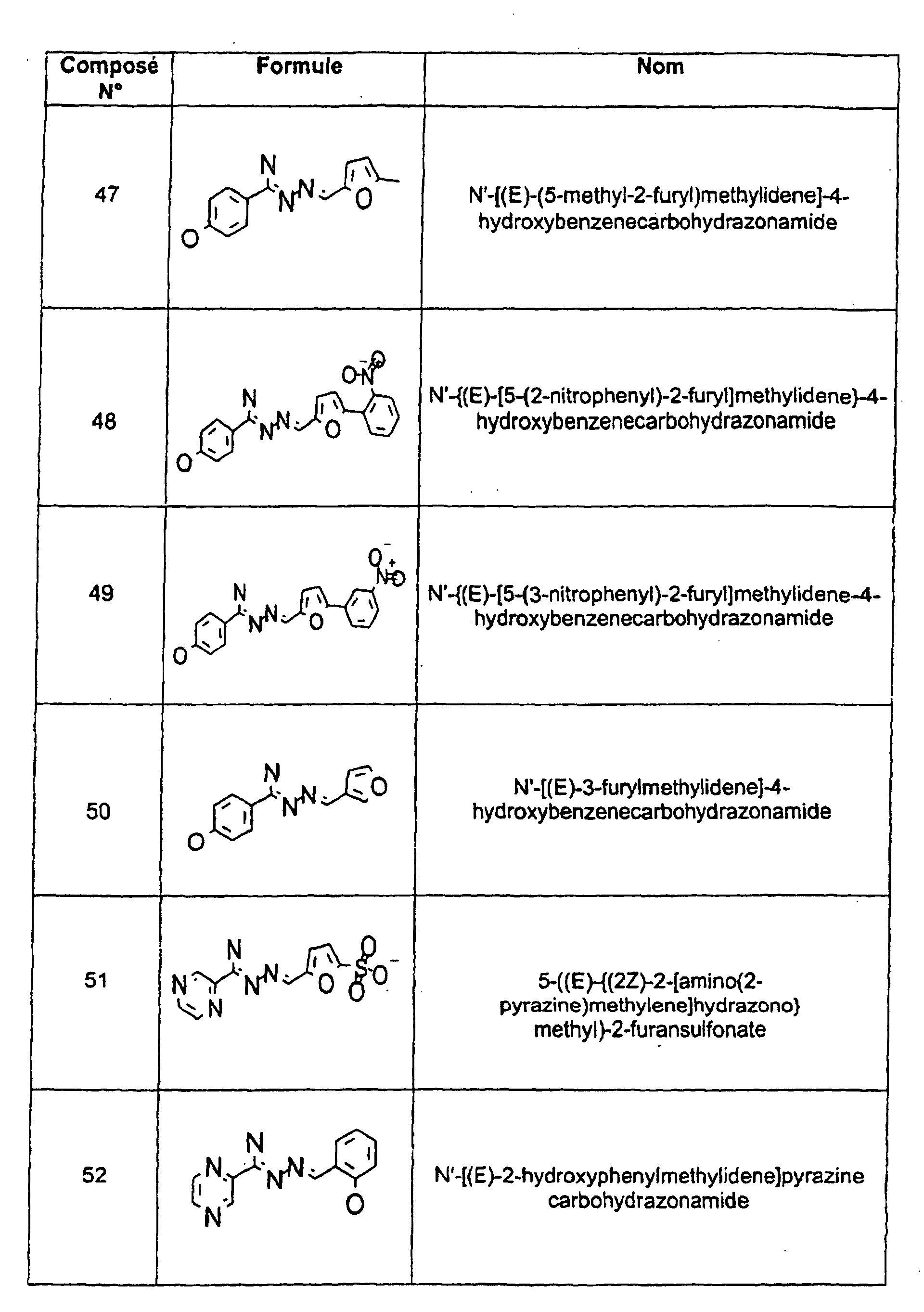

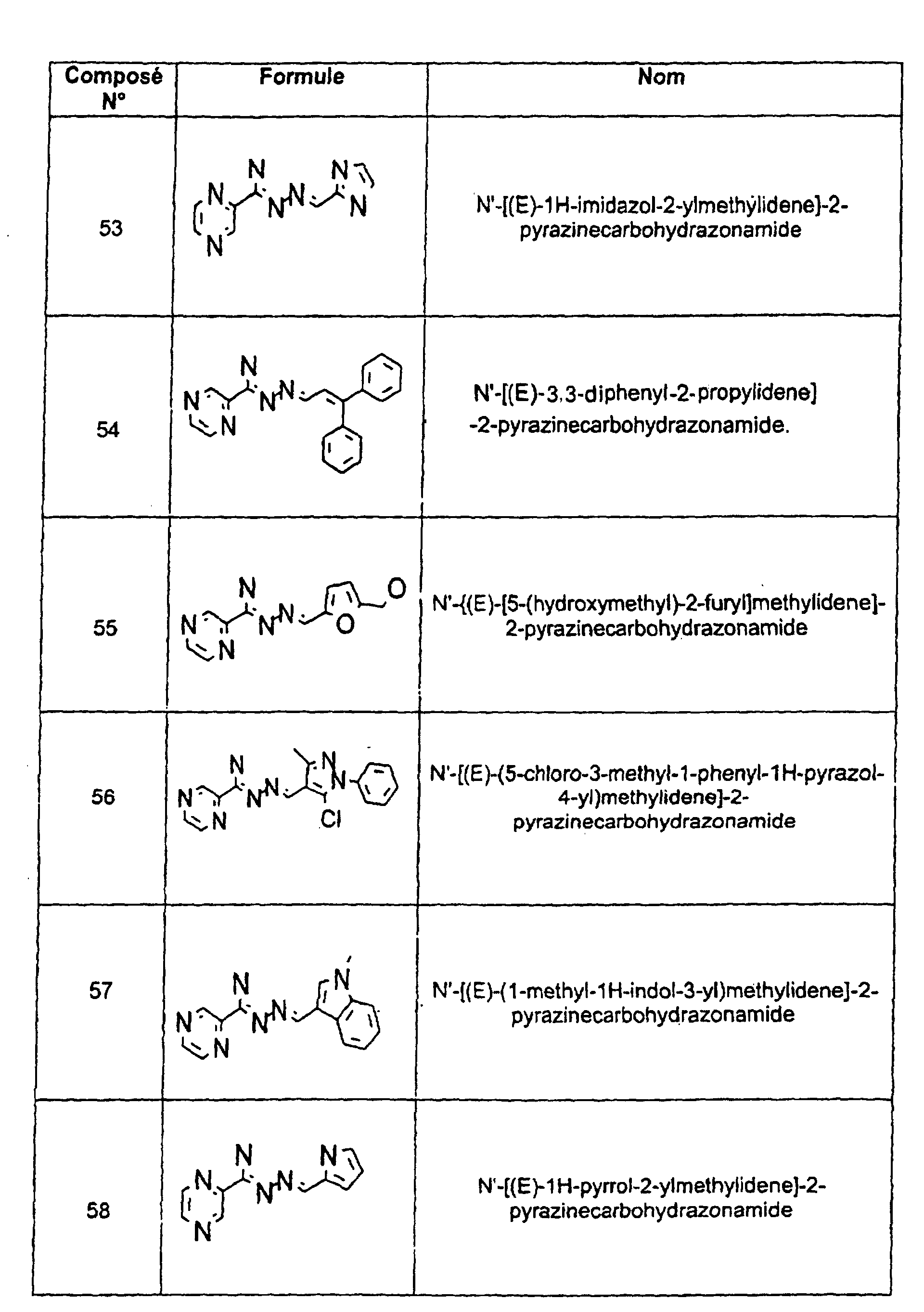

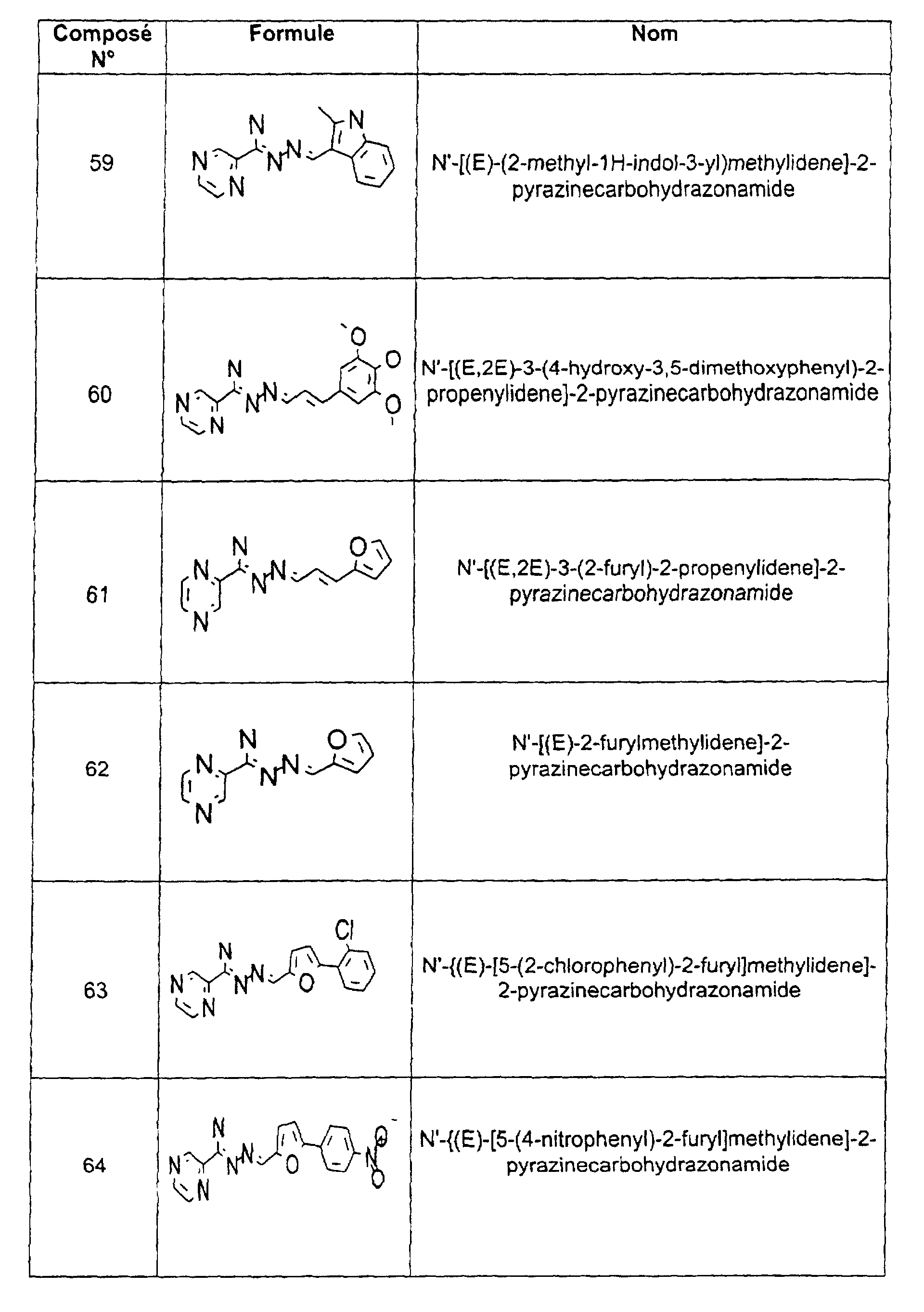

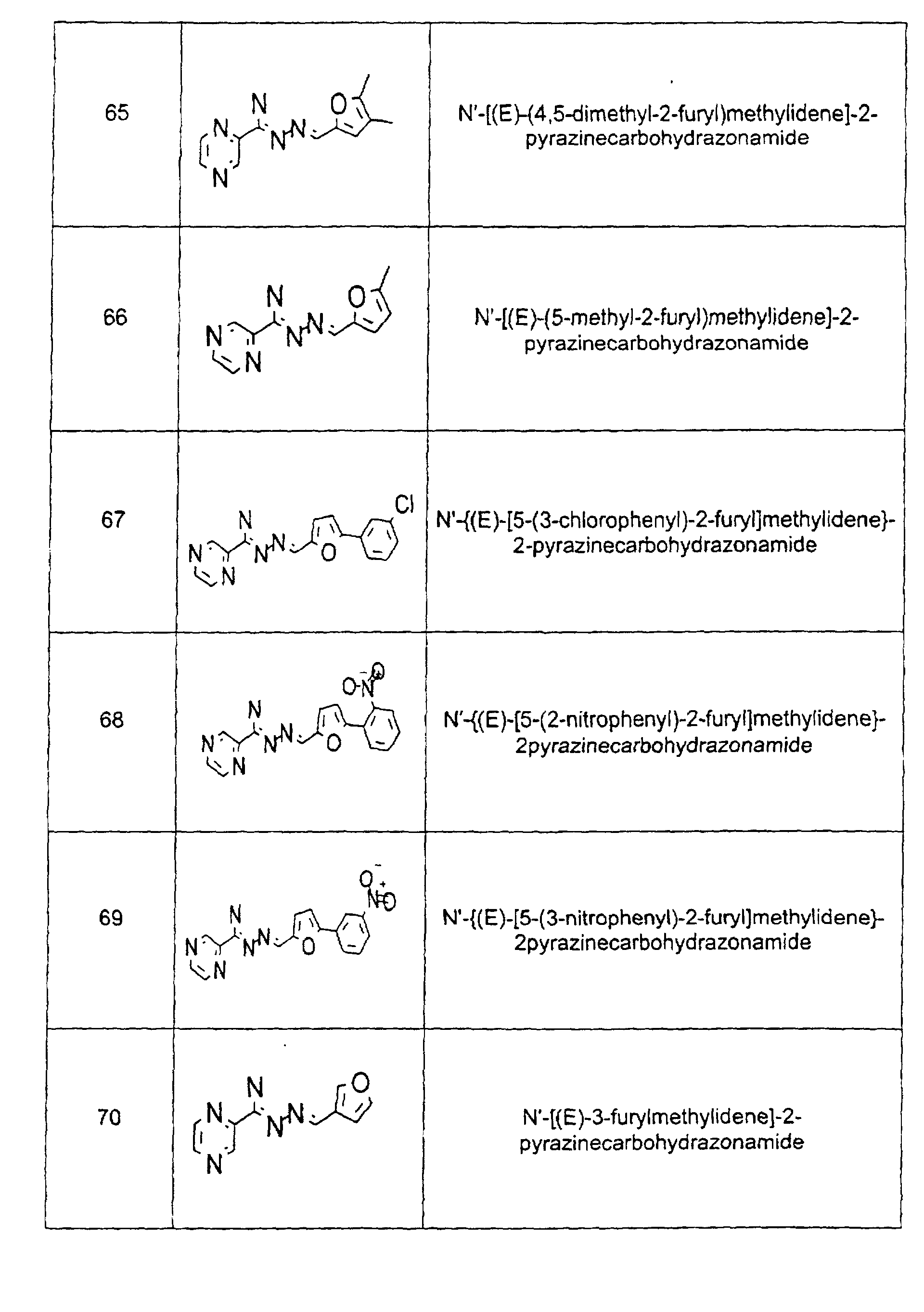

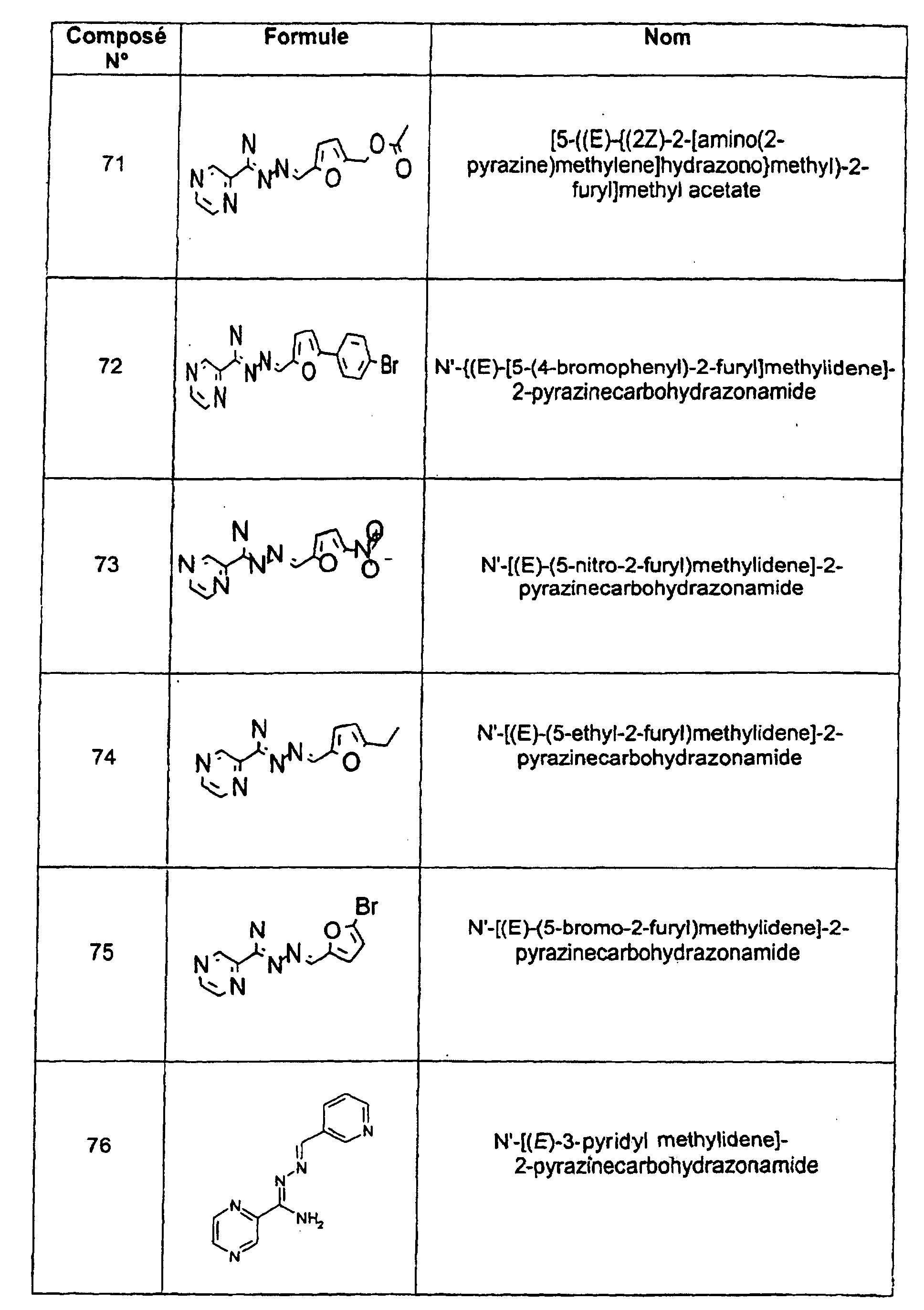

3. Composition selon la revendication 1, où le dérivé d'aminoamidine est le N'-[(E)-(3-methoxyphenyl)-methylidene]benzenecarbohydrazonamide de structure :

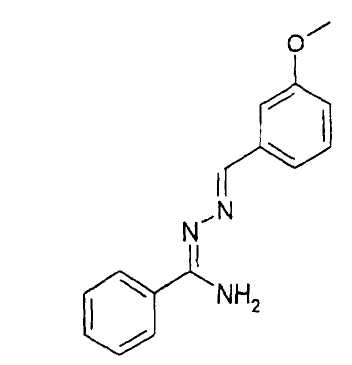

4. Composition selon la revendication 1 ou 2, où le dérivé d'aminoamidine est le N'-[(E)-3-pyridinylmethylidene]-2-pyrazinecarbohydrazonamide de structure :

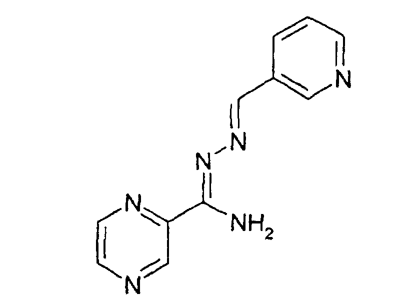

5. Composition selon la revendication 1 ou 2, où le dérivé d'aminoamidine est le N'-[(E)-2-hydroxyphenylmethylidene]pyrazine carbohydrazonamide de structure :

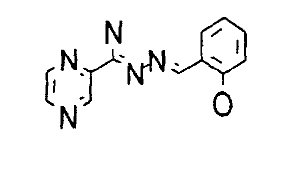

6. Composition selon l'une quelconque des revendications 1 à 5, **caractérisée par le fait que** le composé de formule (1) ou (2) est présent dans la composition à une teneur allant de 0,1 à 20 % en poids, par rapport au poids total de la composition, de préférence allant de 0,5 % à 10 % en poids, par rapport au poids total de la composition.

7. Composition selon l'une quelconque des revendications 1 à 6, **caractérisée par le fait que** ledit support cosmétiquement acceptable se présente sous la forme d'une émulsion de type huile-dans-eau.

8. Composition selon l'une quelconque des revendications 1 à 7, **caractérisée par le fait qu'**elle comprend en outre un ou plusieurs filtres organiques complémentaires actifs dans l'UV-A et/ou UV-B.

9. Composition selon la revendication 8, **caractérisée par le fait que** lesdits filtres organiques complémentaires sont choisis parmi les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate; les dérivés de benzimidazole; les dérivés bis-benzoazolyle; les dérivés de l'acide p-aminobenzoïque ; les polymères filtres et silicones ; les filtres UV organiques insolubles micronisés.

10. Composition selon l'une quelconque des revendications 1 à 9, **caractérisée par le fait qu'**elle comprend en outre, à titre d'agents photoprotecteurs UV complémentaires, des pigments ou des nanopigments d'oxydes métalliques, enrobés ou non.

11. Composition selon la revendication 10, **caractérisée par le fait que** lesdits pigments ou nanopigments sont choisis parmi les oxydes de titane, de zinc, de fer, de zirconium, de cérium et leurs mélanges, enrobés ou non.

12. Composition selon l'une quelconque des revendications 1 à 11, **caractérisée par le fait qu'**elle comprend en outre au moins un agent de bronzage et/ou de brunissage artificiel de la peau.

13. Composition selon l'une quelconque des revendications 1à 12, **caractérisée par le fait qu'**elle comprend en outre au moins un adjuvant choisi parmi les corps gras, les solvants organiques, les épaississants, les adoucissants, les antioxydants, les opacifiants, les stabilisants, les émollients, les agents anti-mousse, les agents hydratants, , les parfums, les conservateurs, les tensioactifs, les séquestrants, les polymères, les propulseurs, les agents alcalinisants ou acidifiants.

14. Composition selon l'une quelconque des revendications 1 à 12, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

15. Composition selon l'une quelconque des revendications 1 à 12, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

16. Composition selon l'une quelconque des revendications 1 à 12, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

17. Utilisation d'un dérivé d'aminoamidine de formule (I) tel que défini dans l'une quelconque des revendications 1 à 5 pour la fabrication d'une composition destinées à protéger des matières sensibles au rayonnement ultraviolet, en particulier au rayonnement solaire.

18. Utilisation d'un dérivé d'aminoamidine de formule (I) tel que défini dans l'une quelconque des revendications 1 à 5 pour la fabrication d'une composition cosmétique ou dermatologique comme agent filtrant les radiations UV.

19. Utilisation d'un dérivé d'aminoamidine de formule (I) tel que défini dans l'une quelconque des revendications 1 à 5 pour la fabrication d'une composition cosmétique comme agent de contrôle de la variation de la couleur de la peau due aux rayonnement UV.

20. Dérivé d'aminoamidine choisi parmi les composés suivants :

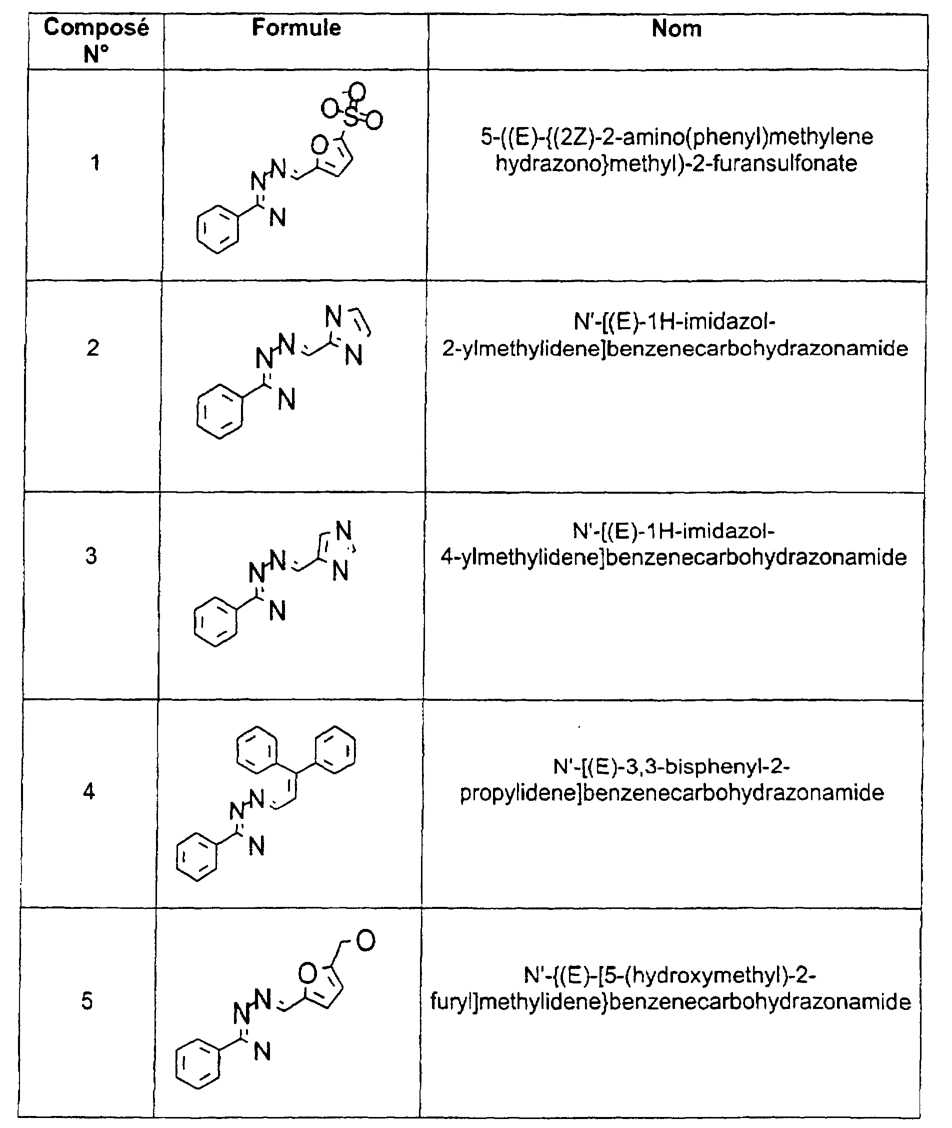

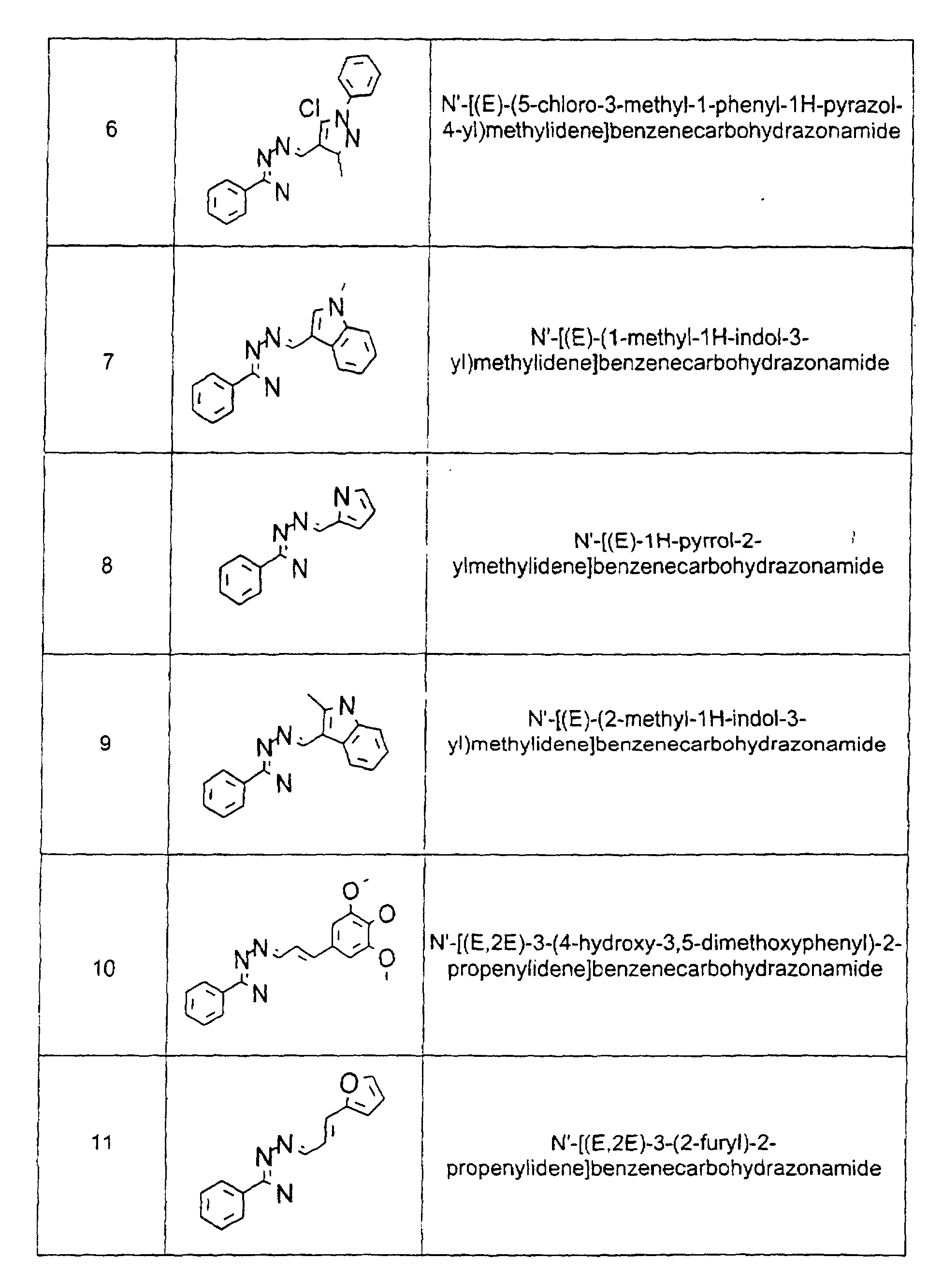

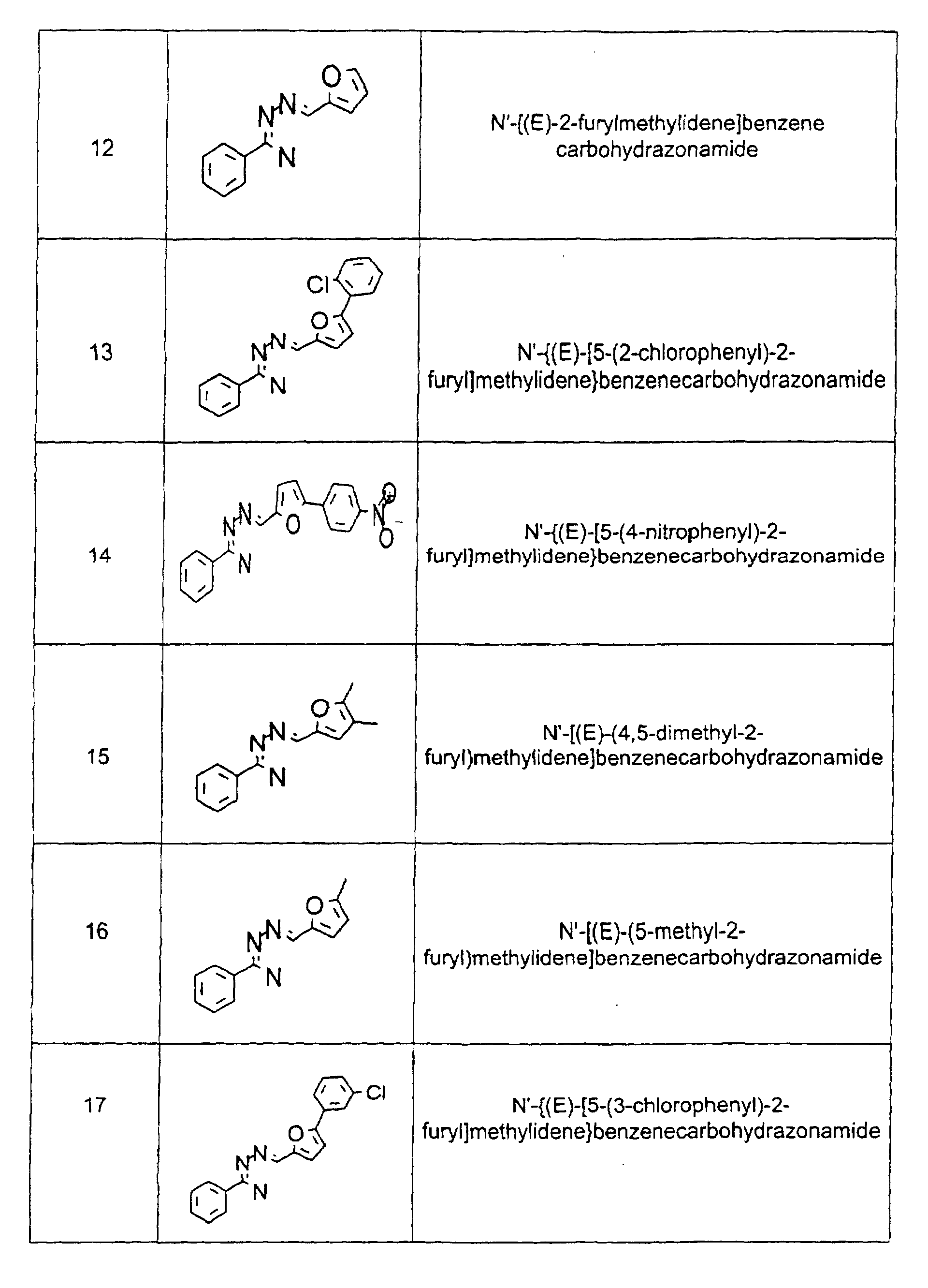

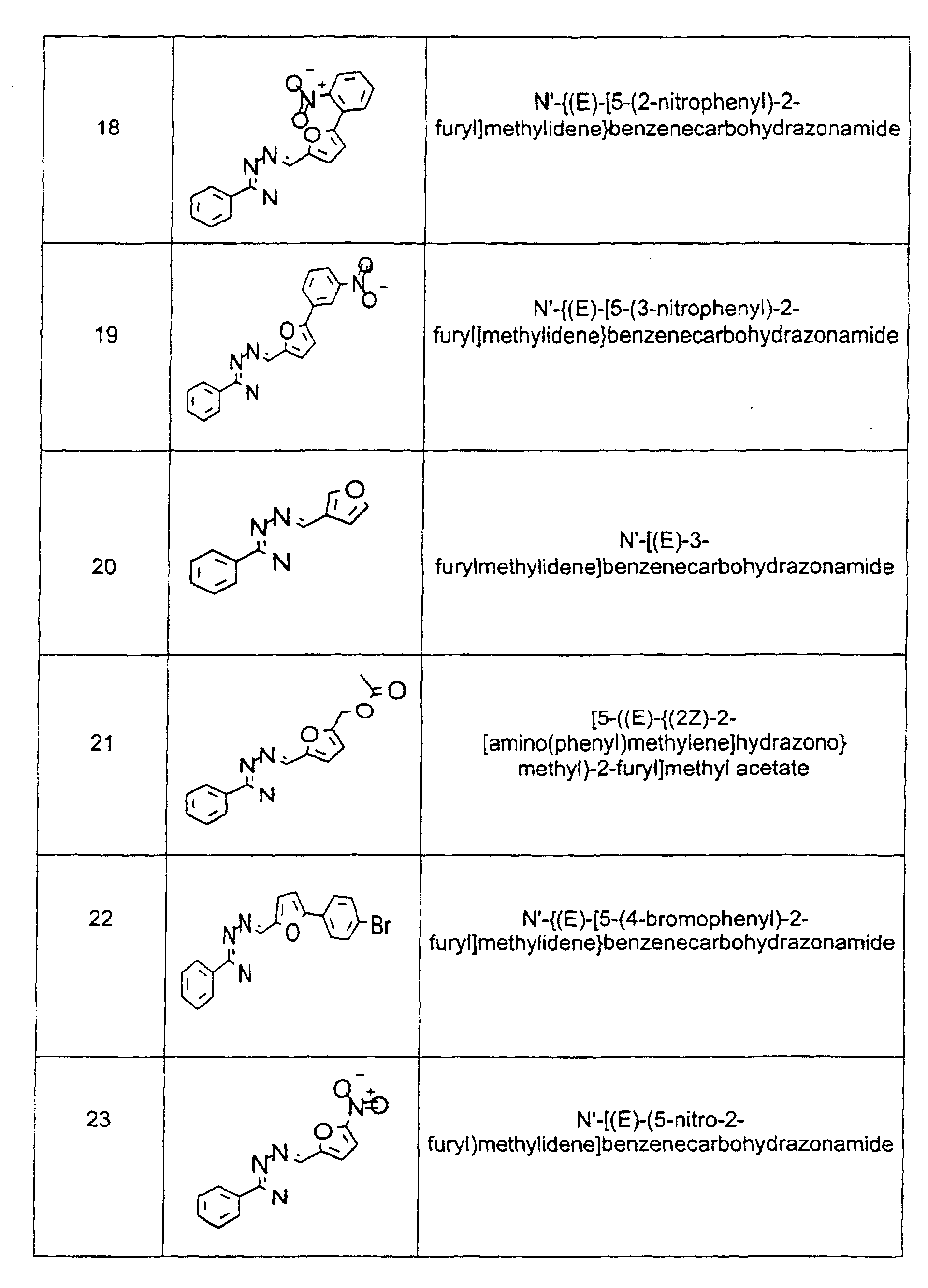

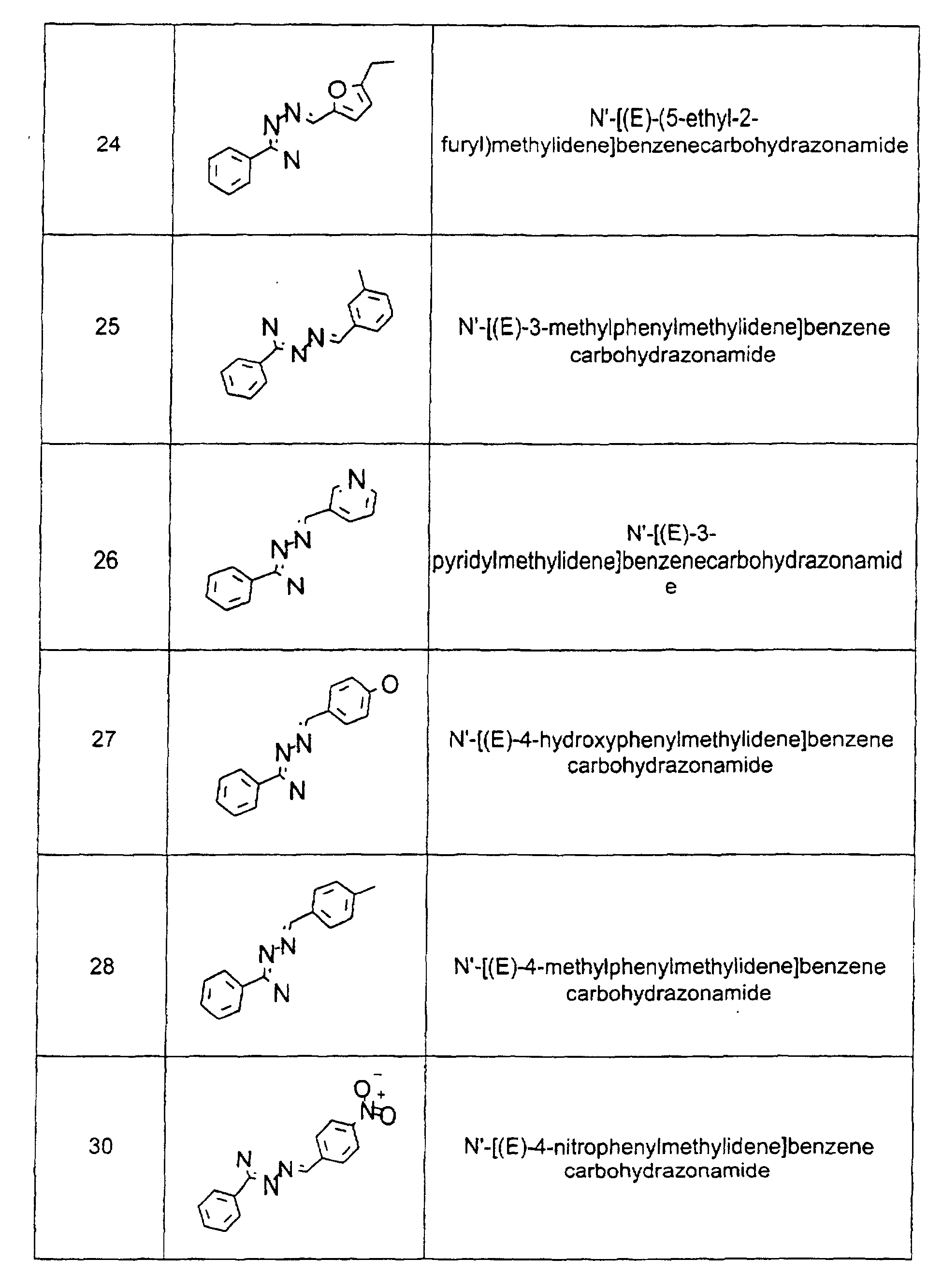

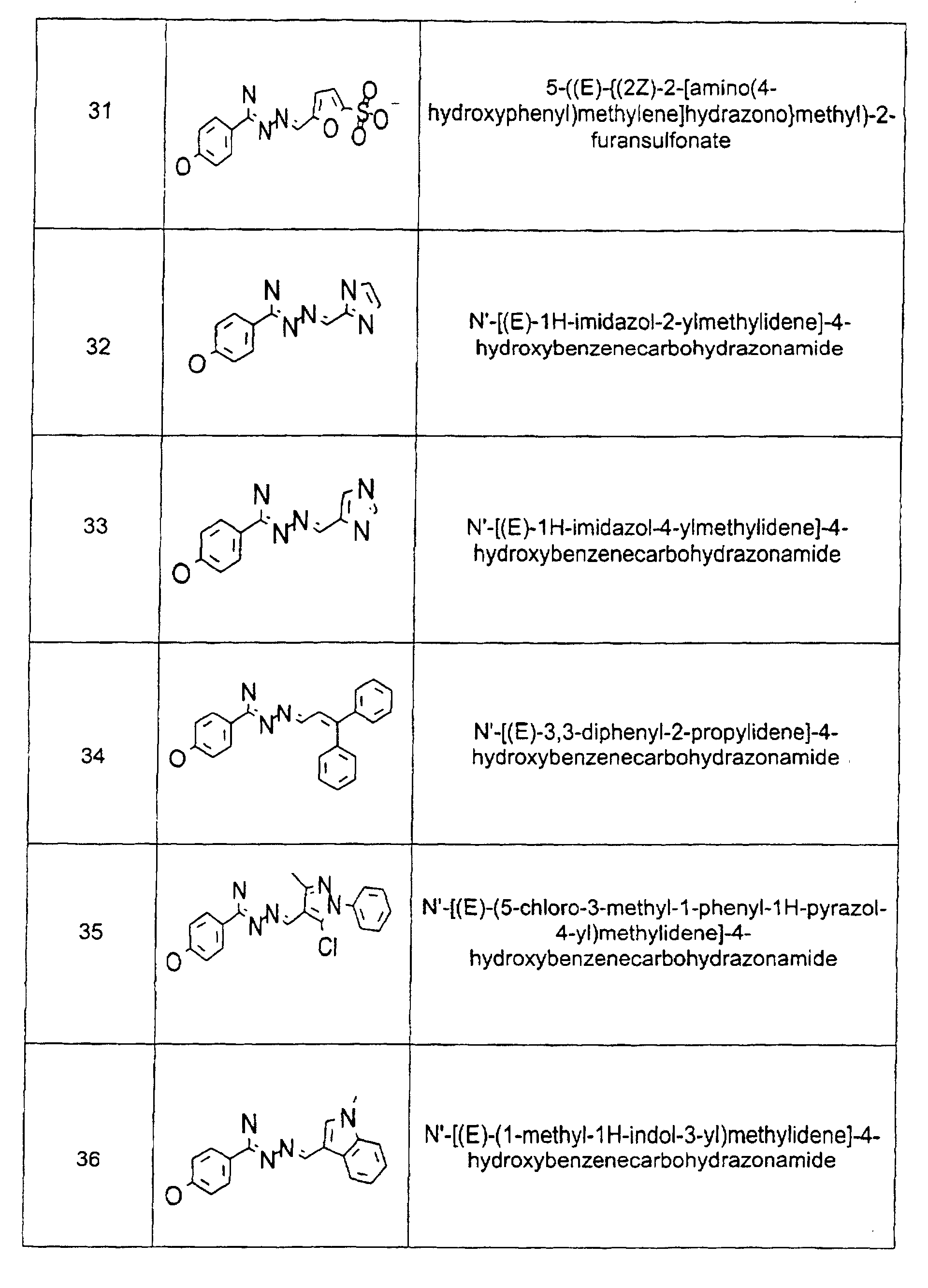

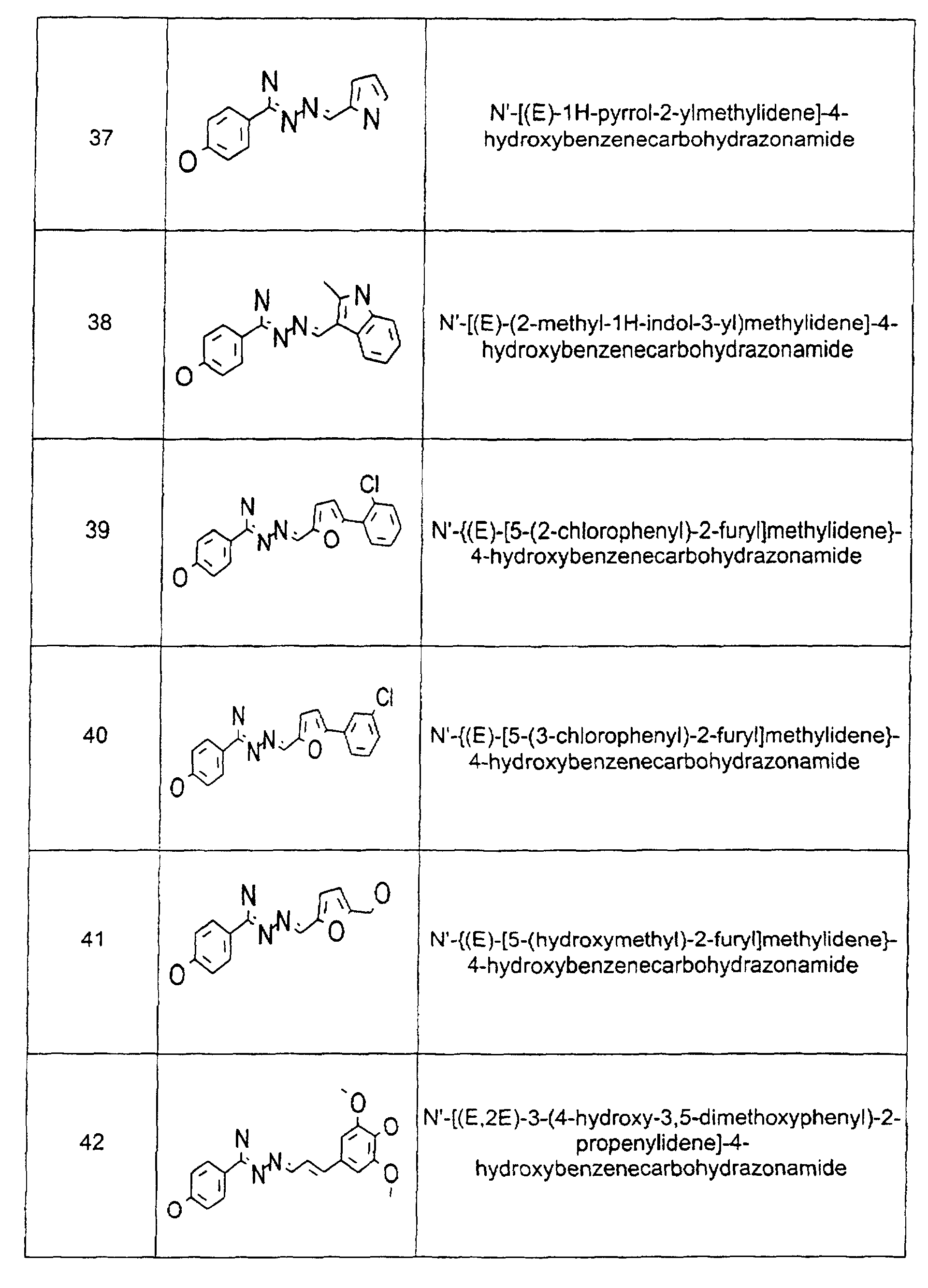

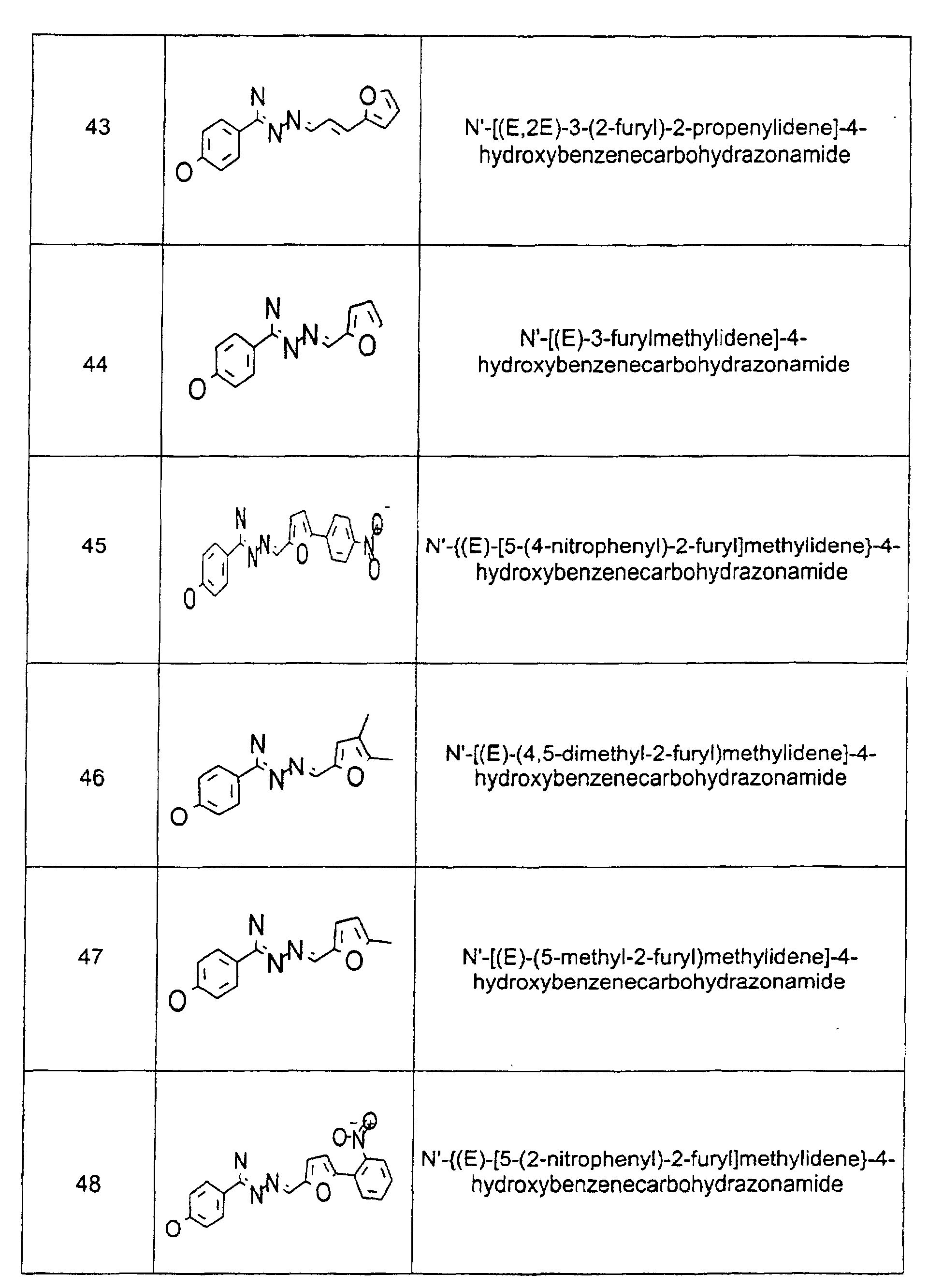

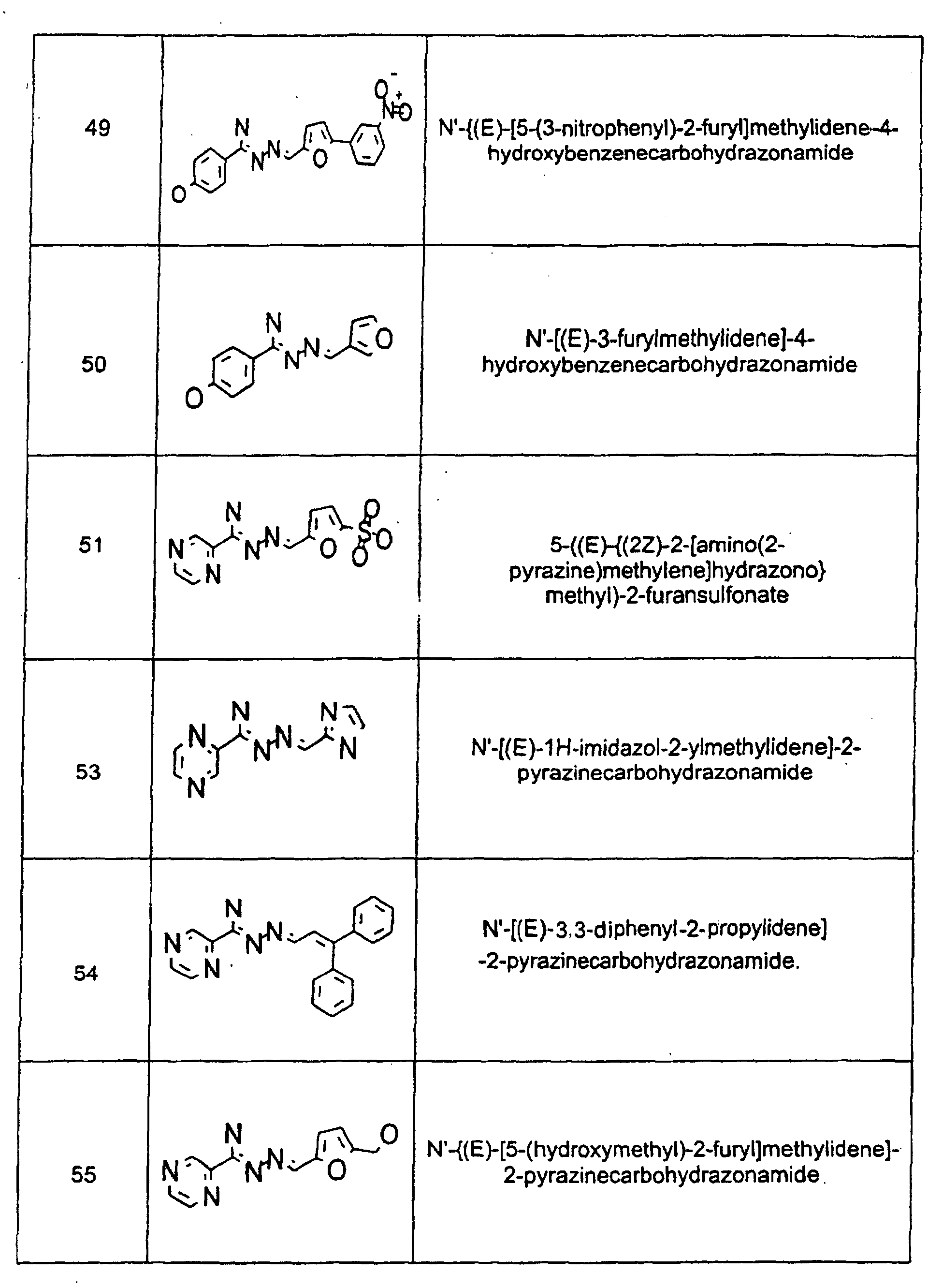

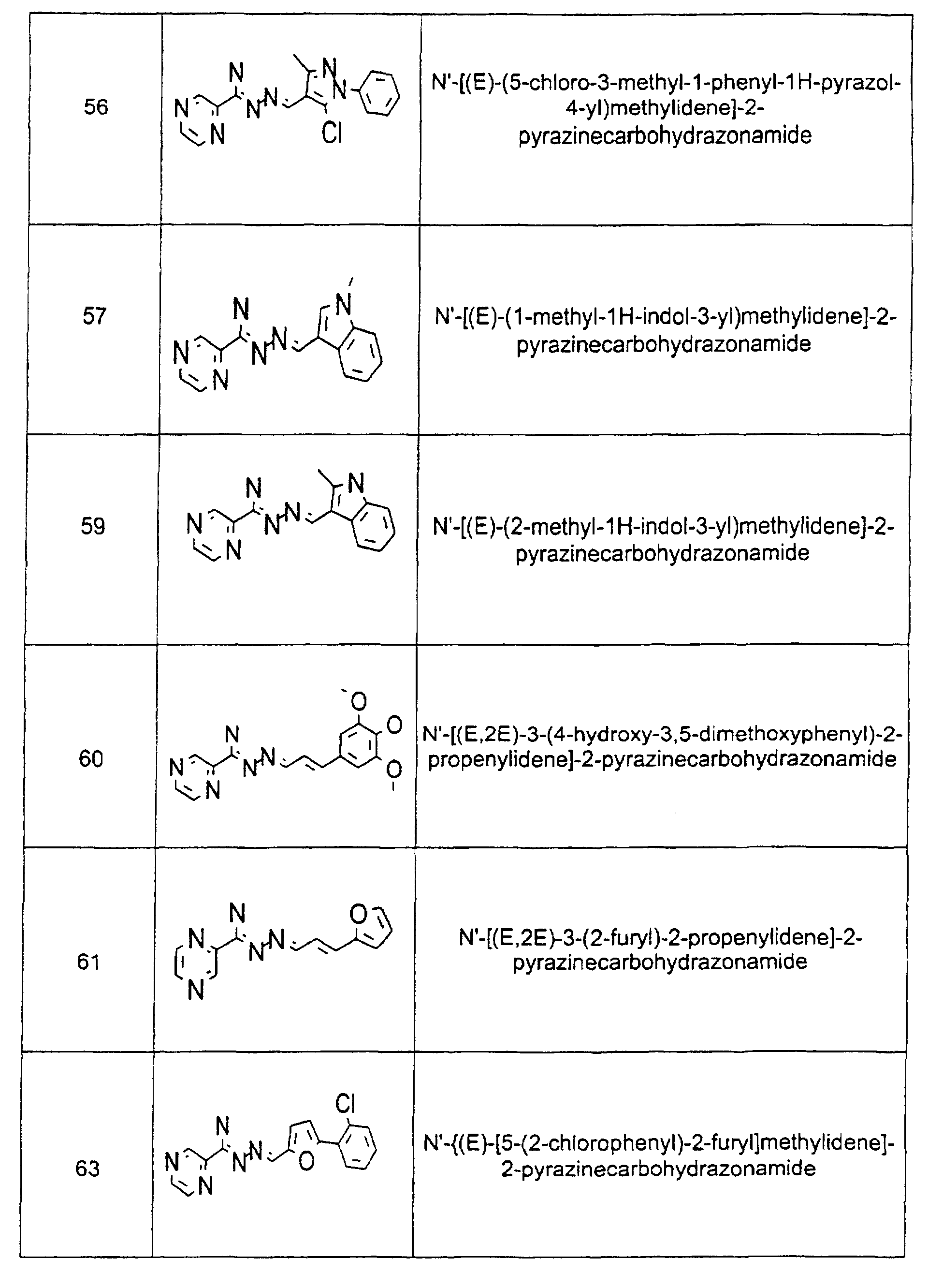

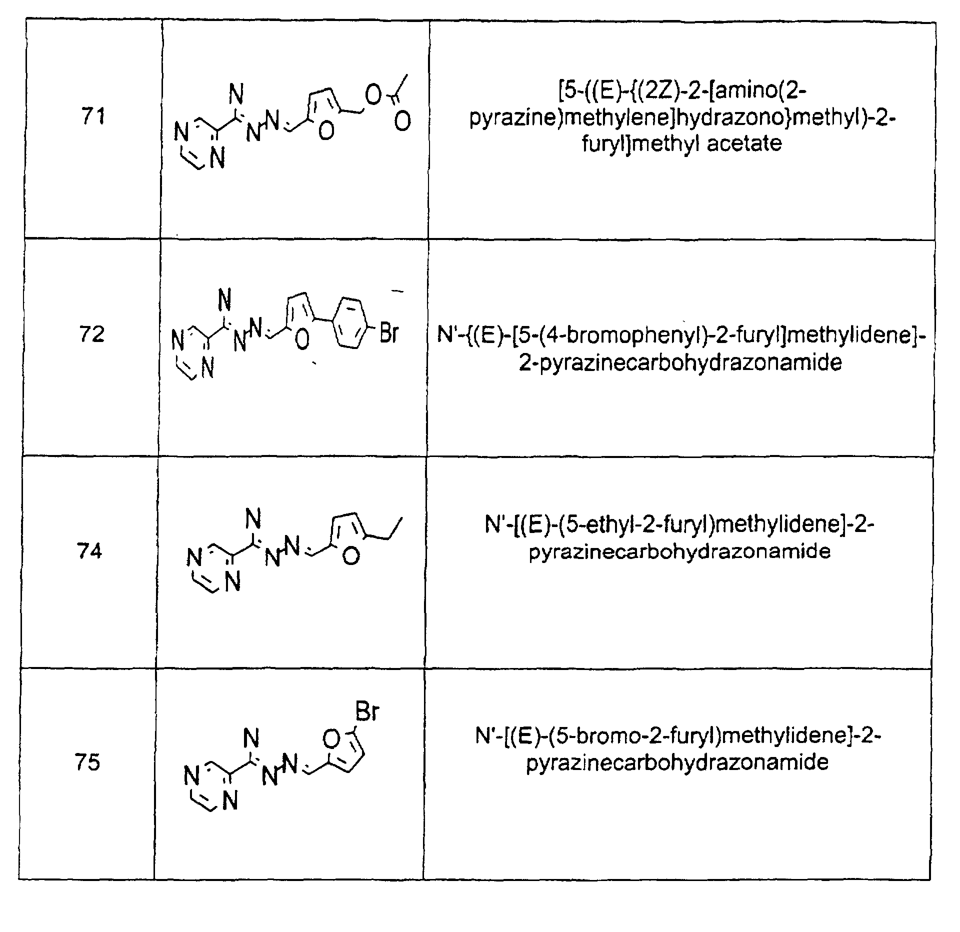

## Patentansprüche

1. Kosmetische Zusammensetzung zur topischen Anwendung insbesondere zum Lichtschutz der Haut und/oder der Haare, **dadurch gekennzeichnet, dass** sie in einem kosmetisch akzeptablen Träger mindestens ein Aminoamidinderivat der folgenden Formel (I) enthält: 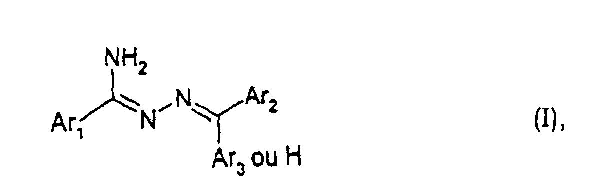 worin bedeuten:
- Ar₁:
(i) einen aromatischen Ring der folgenden Formel: 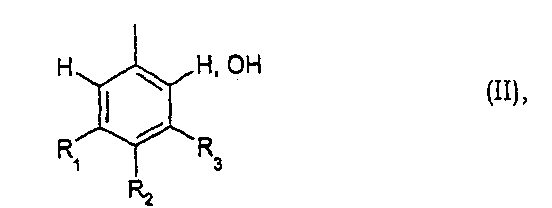 worin die Gruppen R₁, R₂ und R₃, die gleich oder verschieden sind, ein Wasserstoffatom; eine geradkettige oder verzweigte C₁₋₈-Alkylgruppe; oder eine C₁₋₈-Alkoxygruppe bedeuten; oder worin die Gruppen R₁, R₂ und R₃ einen aromatischen oder nicht aromatischen Ring oder einen aromatischen Heterocyclus mit 5 oder 6 Gliedern bilden können;
(ii) einen aromatischen Ring mit 6 Atomen der folgenden Struktur: 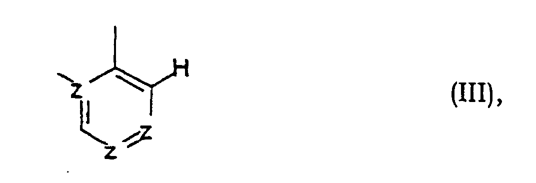 worin Z C oder N bedeutet;
- Ar₂ einen aromatischen Ring gemäß der oben für Ar₁ angegebenen Definition oder einen aromatischen Ring mit 5 Atomen der folgenden Struktur: 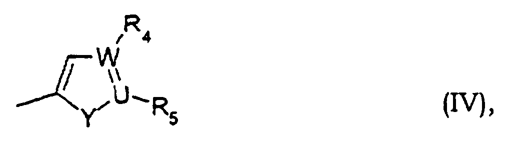 worin bedeuten:
- Y ein Schwefelatom, Sauerstoffatom, Kohlenstoffatom oder Stickstoffatom;
- W ein Kohlenstoffatom oder Stickstoffatom;
- R₄:
(i) ein Wasserstoffatom, wenn W ein Stickstoffatom ist;
(ii) ein Wasserstoffatom, eine geradkettige oder verzweigte C₁₋₈-Alkylgruppe; eine C₁₋₈-Alkoxygruppe oder R₄ kann einen aromatischen oder nicht aromatischen Ring bilden, wenn W ein Kohlenstoffatom ist;
- U ein Kohlenstoffatom oder ein Stickstoffatom, wenn W ein Kohlenstoffatom ist;
- R₅:
(i) ein Wasserstoffatom, wenn U Stickstoff bedeutet;
(ii) ein Wasserstoffatom; eine geradkettige oder verzweigte C₁₋₈-Alkylgruppe; eine C₁₋₈-Alkoxygruppe; oder R₅ kann einen aromatischen oder nicht aromatischen Ring bilden, wenn U ein Kohlenstoffatom ist;
- Ar₃ einen aromatischen Ring gemäß der oben für Ar₂ angegebenen Definition oder ein Wasserstoffatom.

2. Zusammensetzung nach Anspruch 1, wobei das Aminoamidinderivat unter den in der folgenden Tabelle definierten Verbindungen ausgewählt ist:

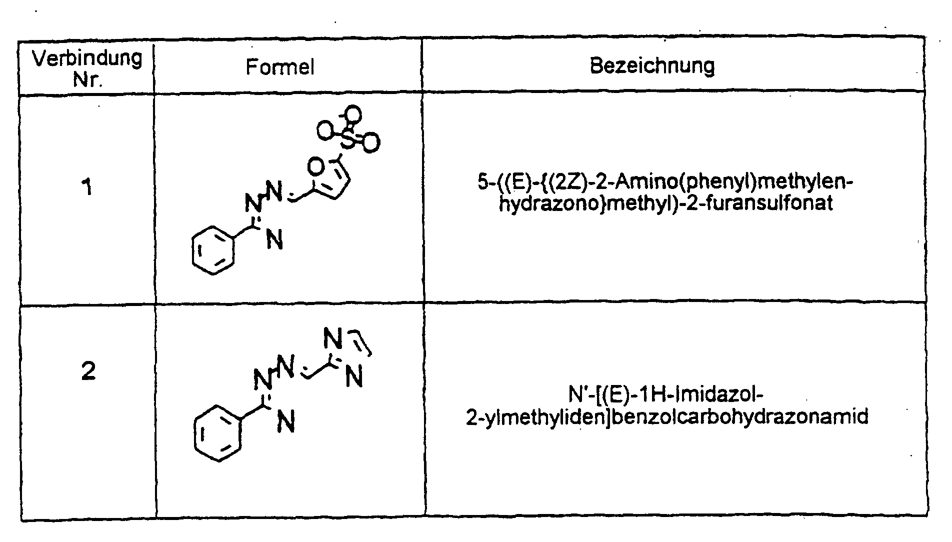

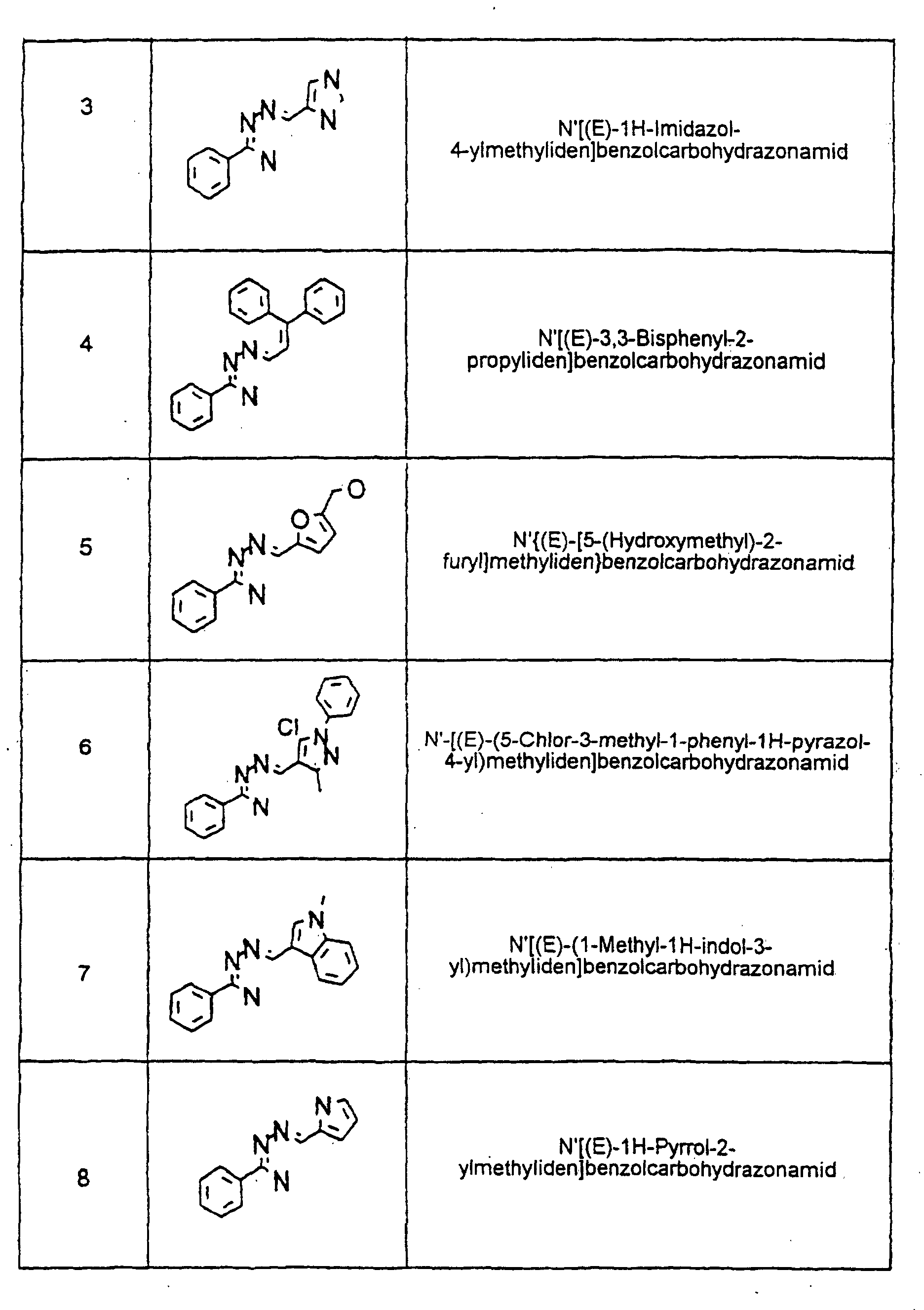

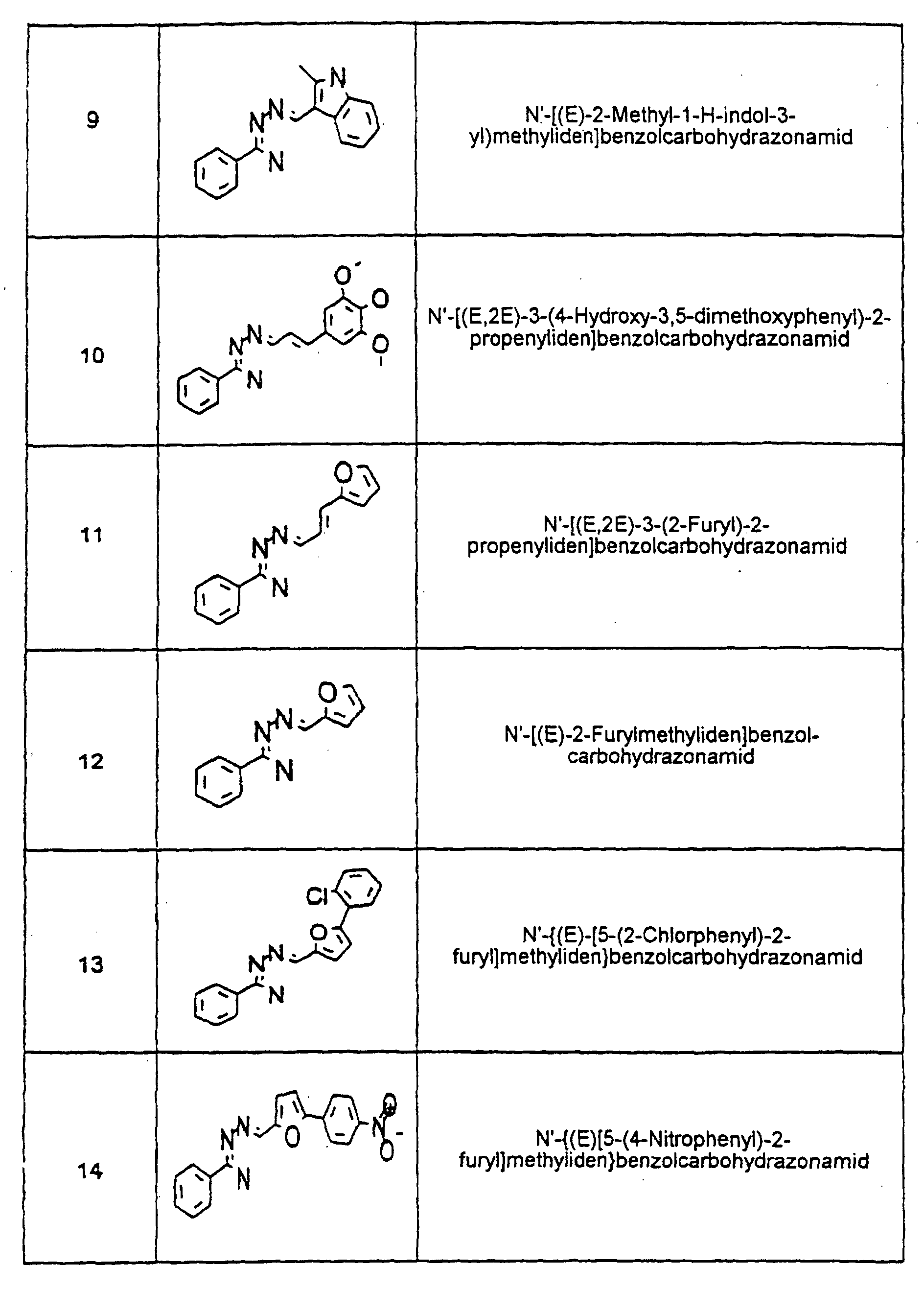

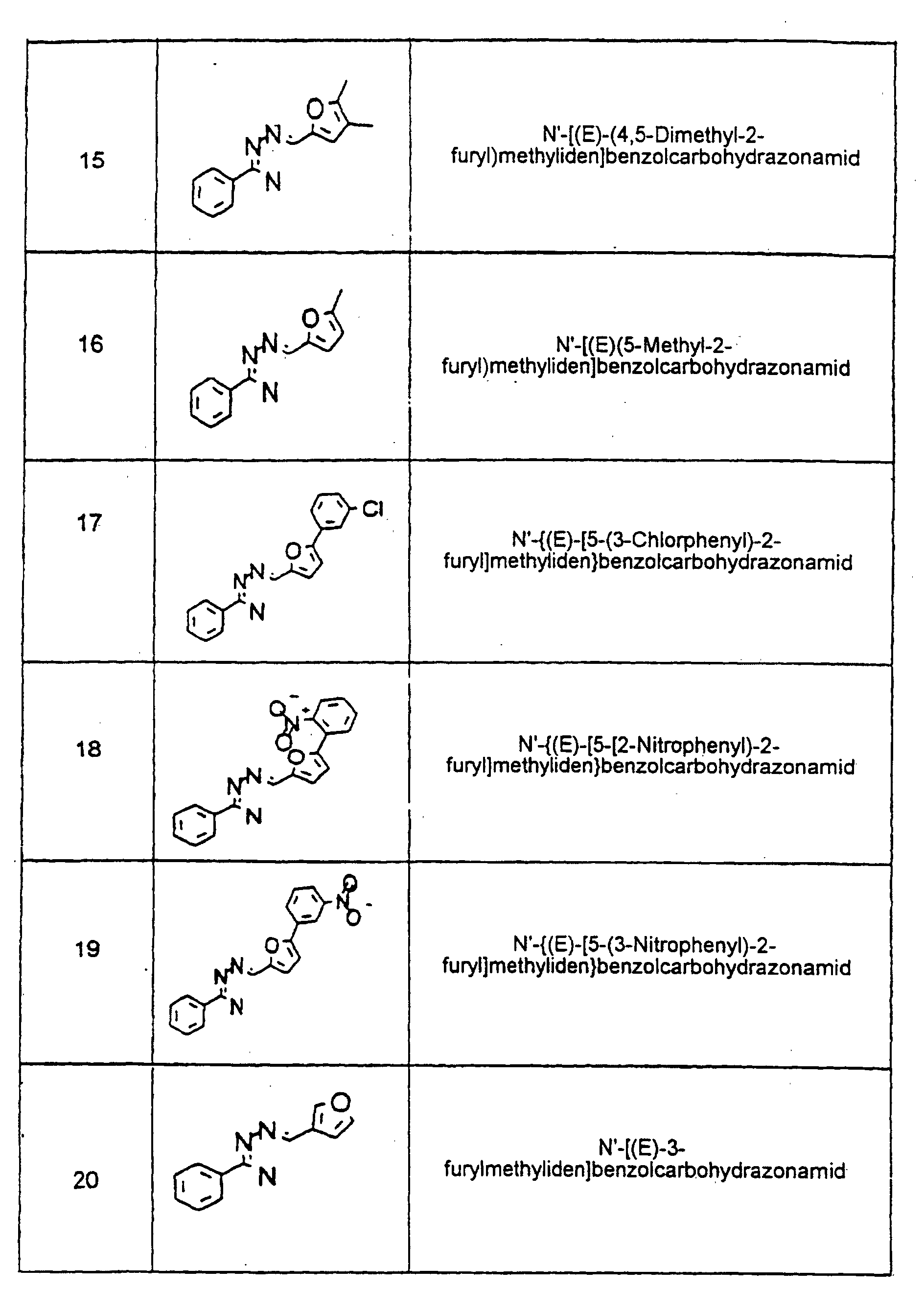

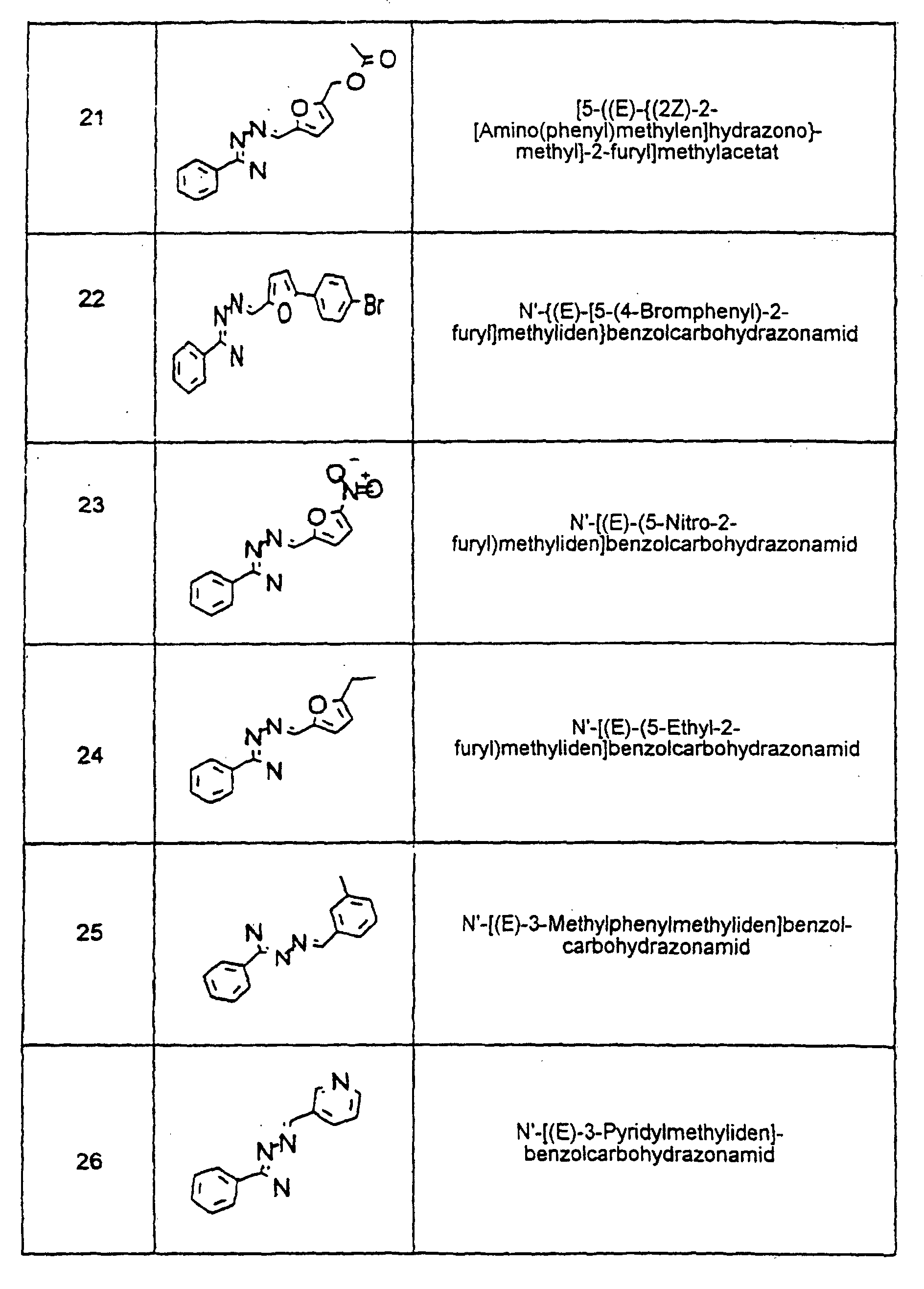

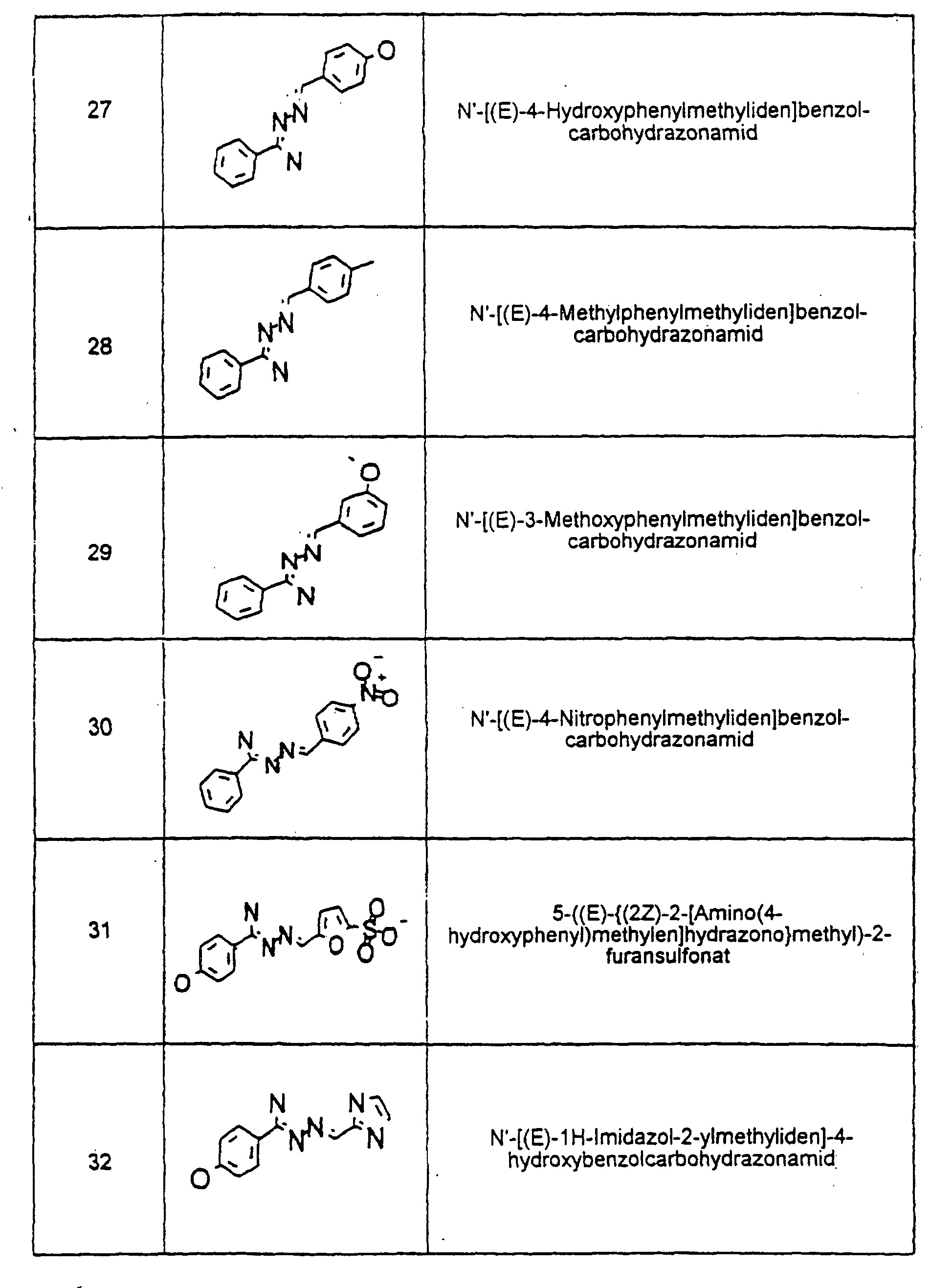

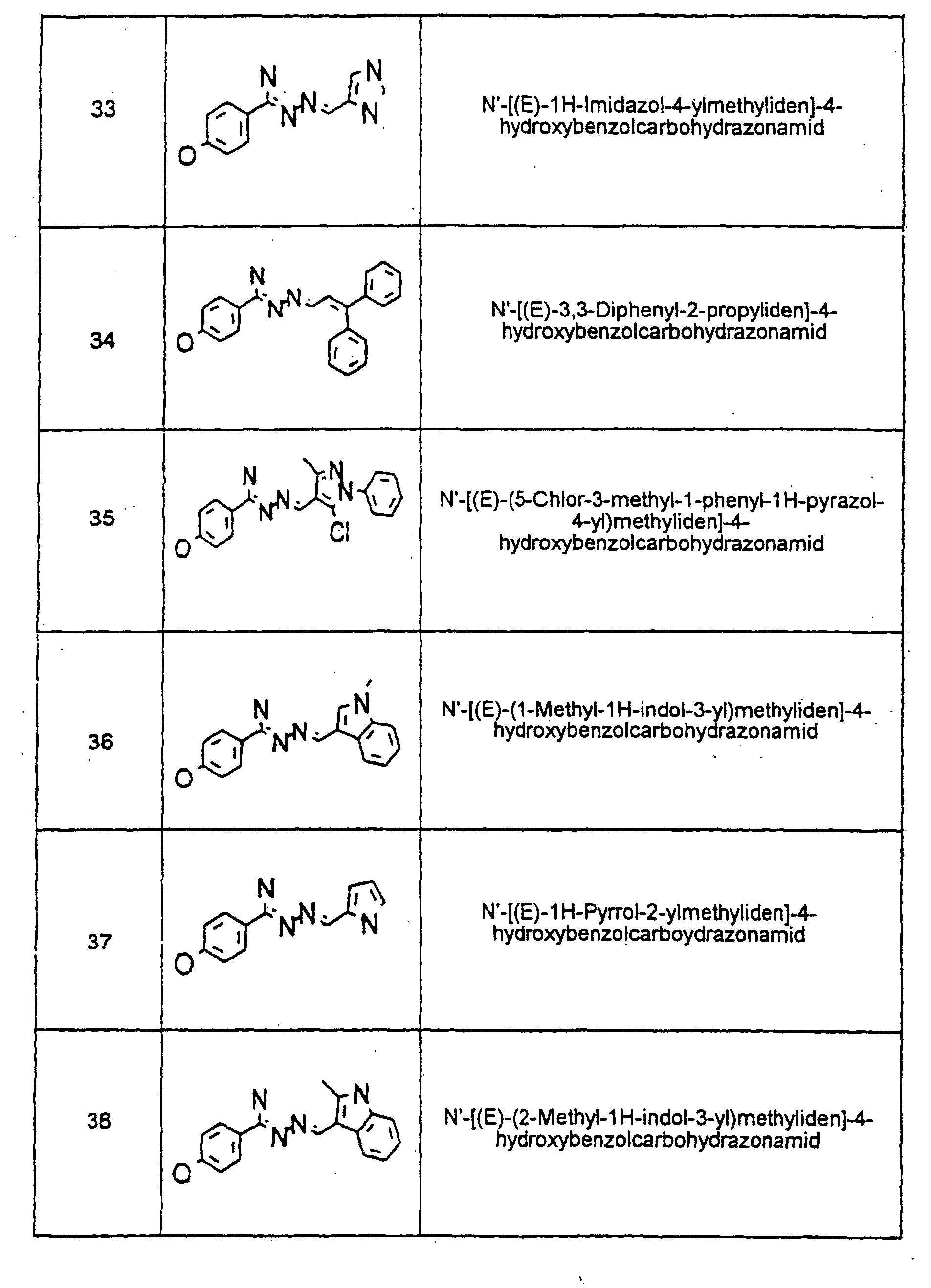

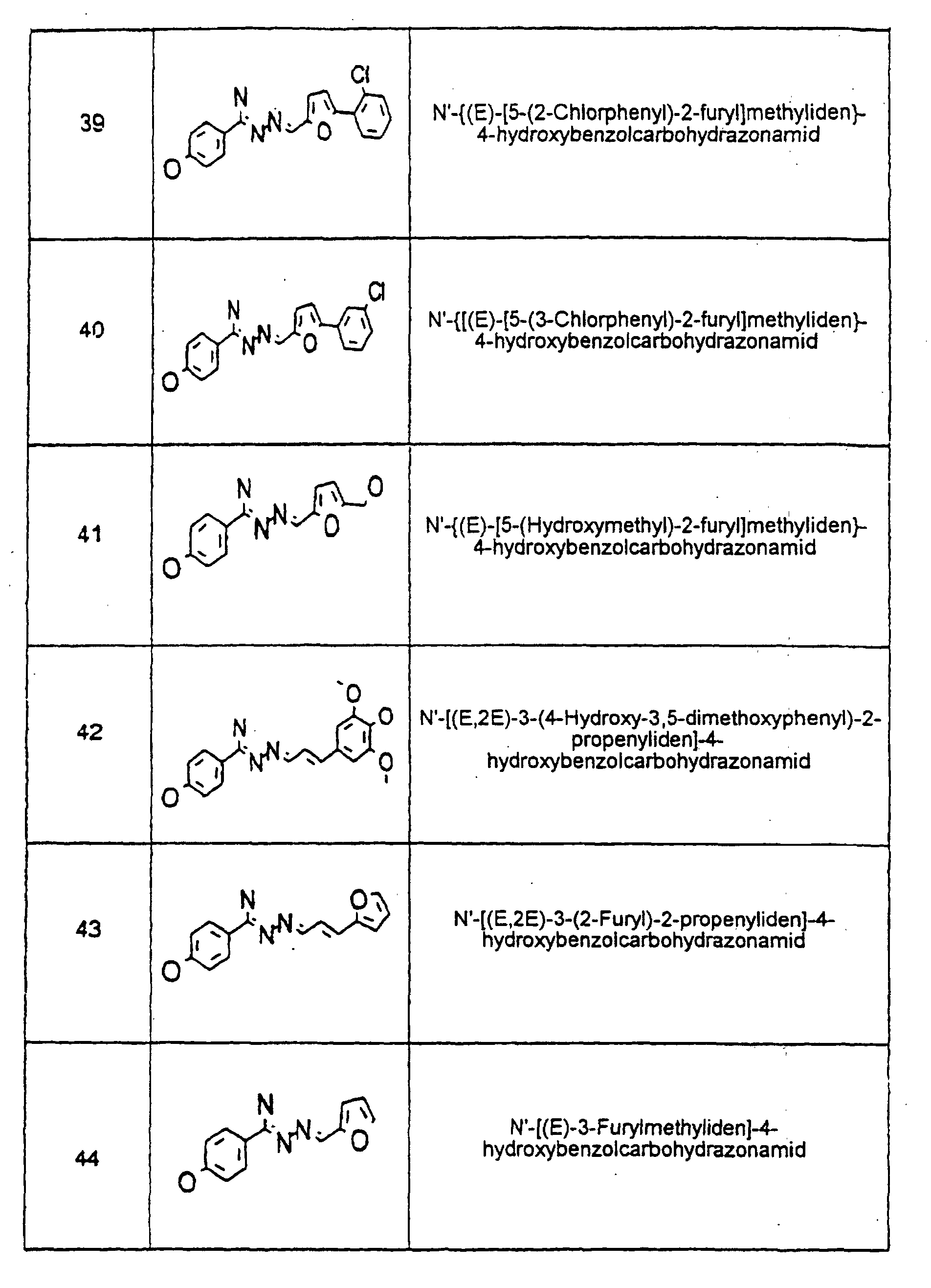

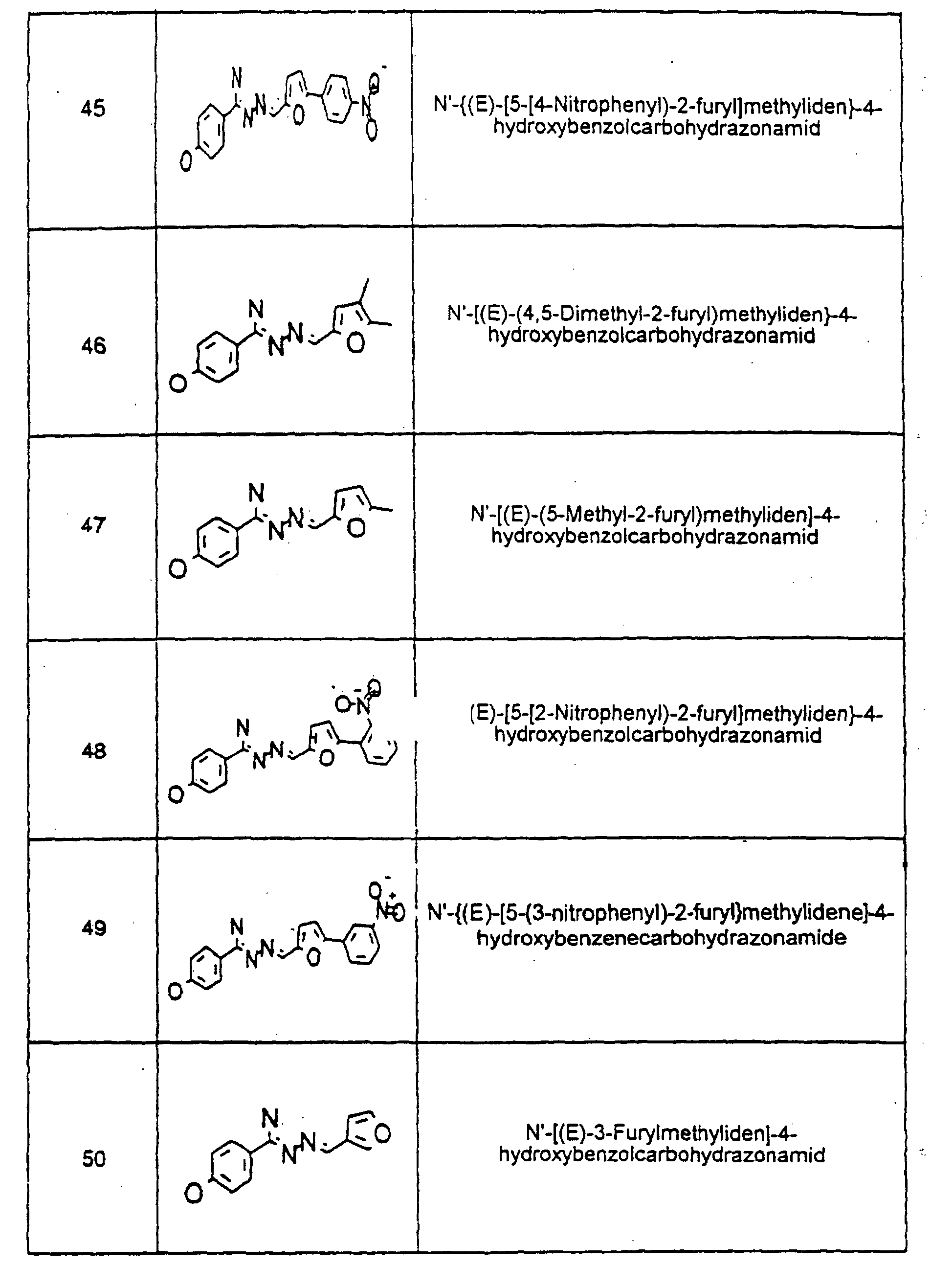

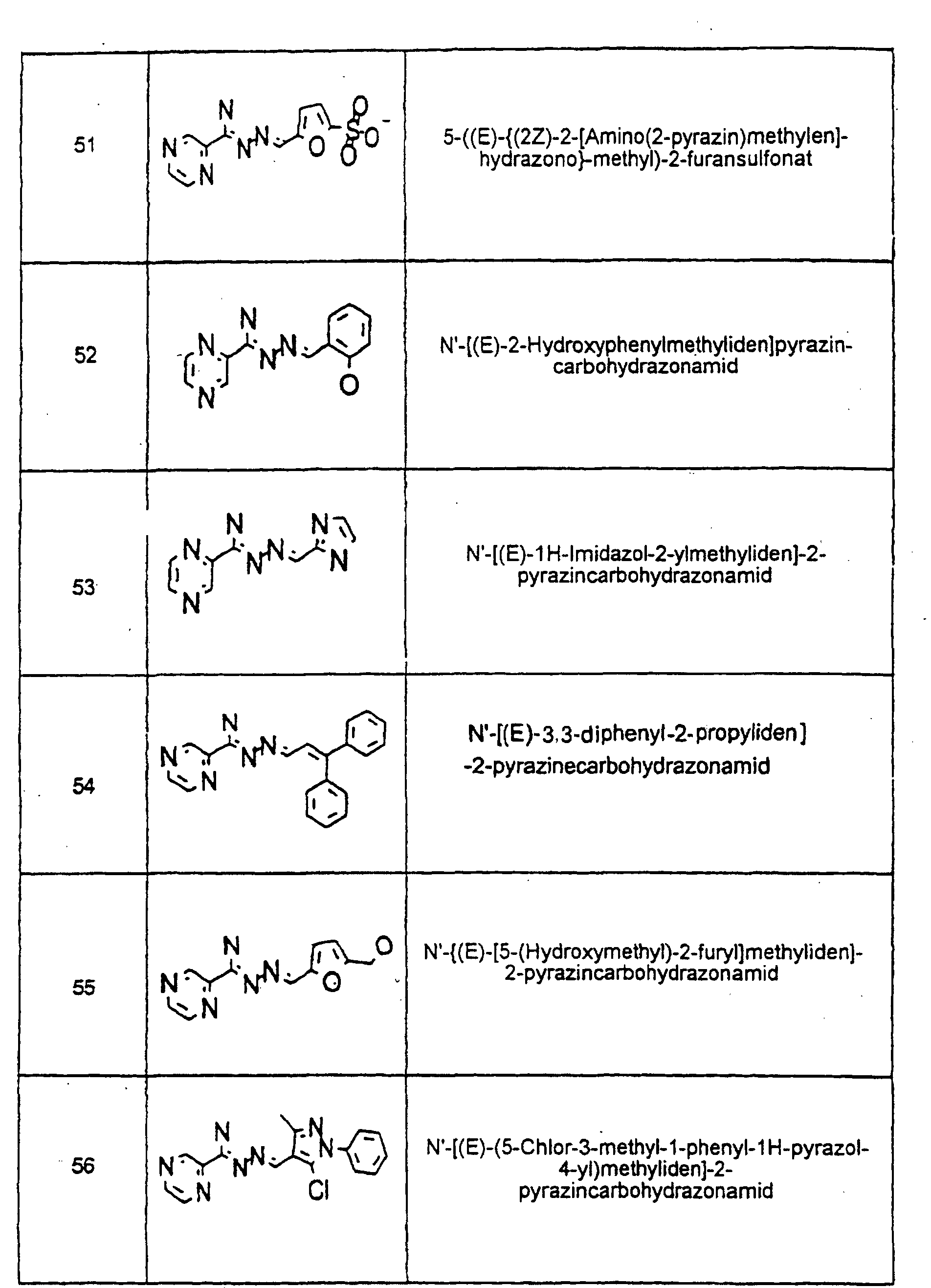

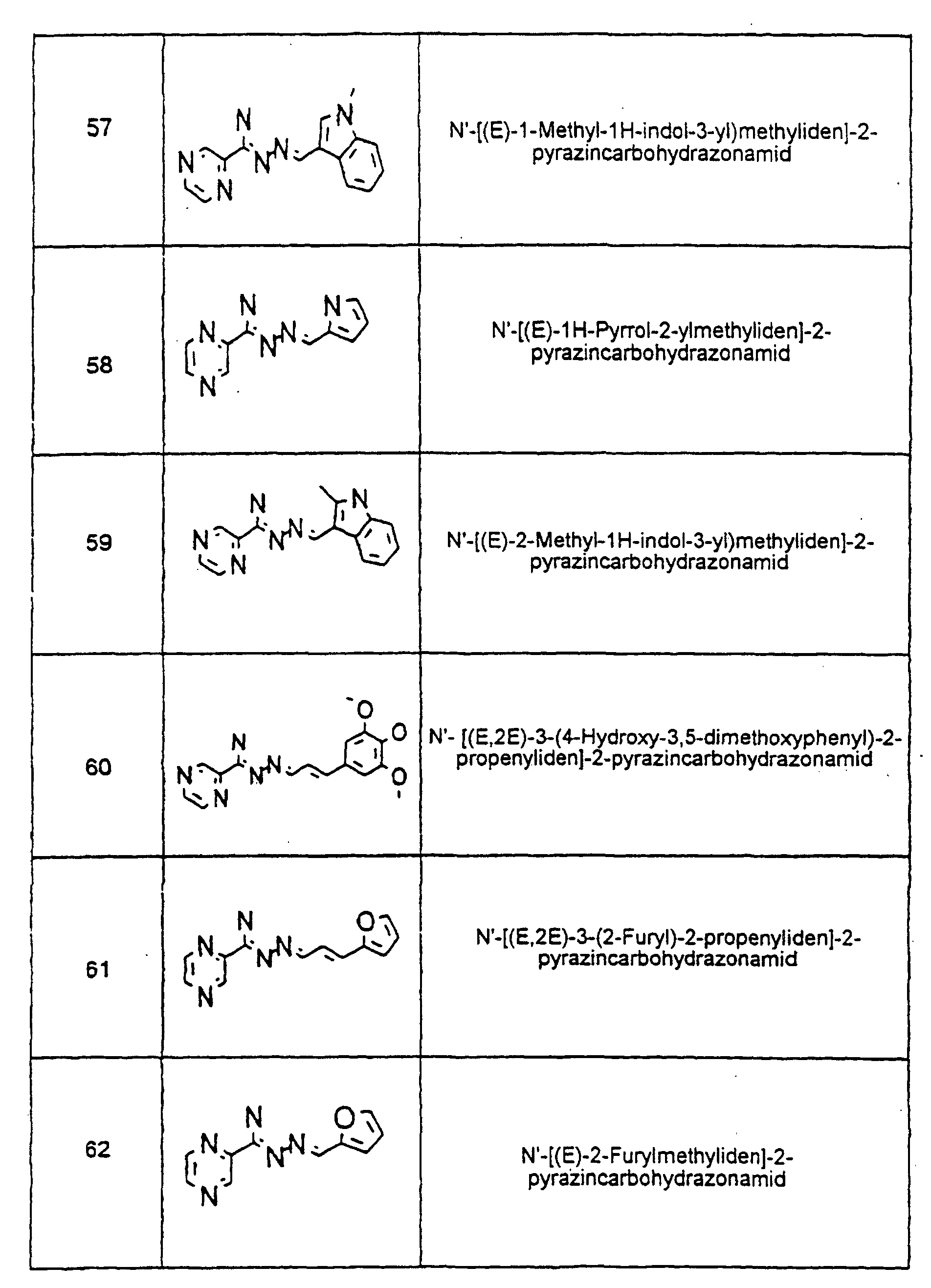

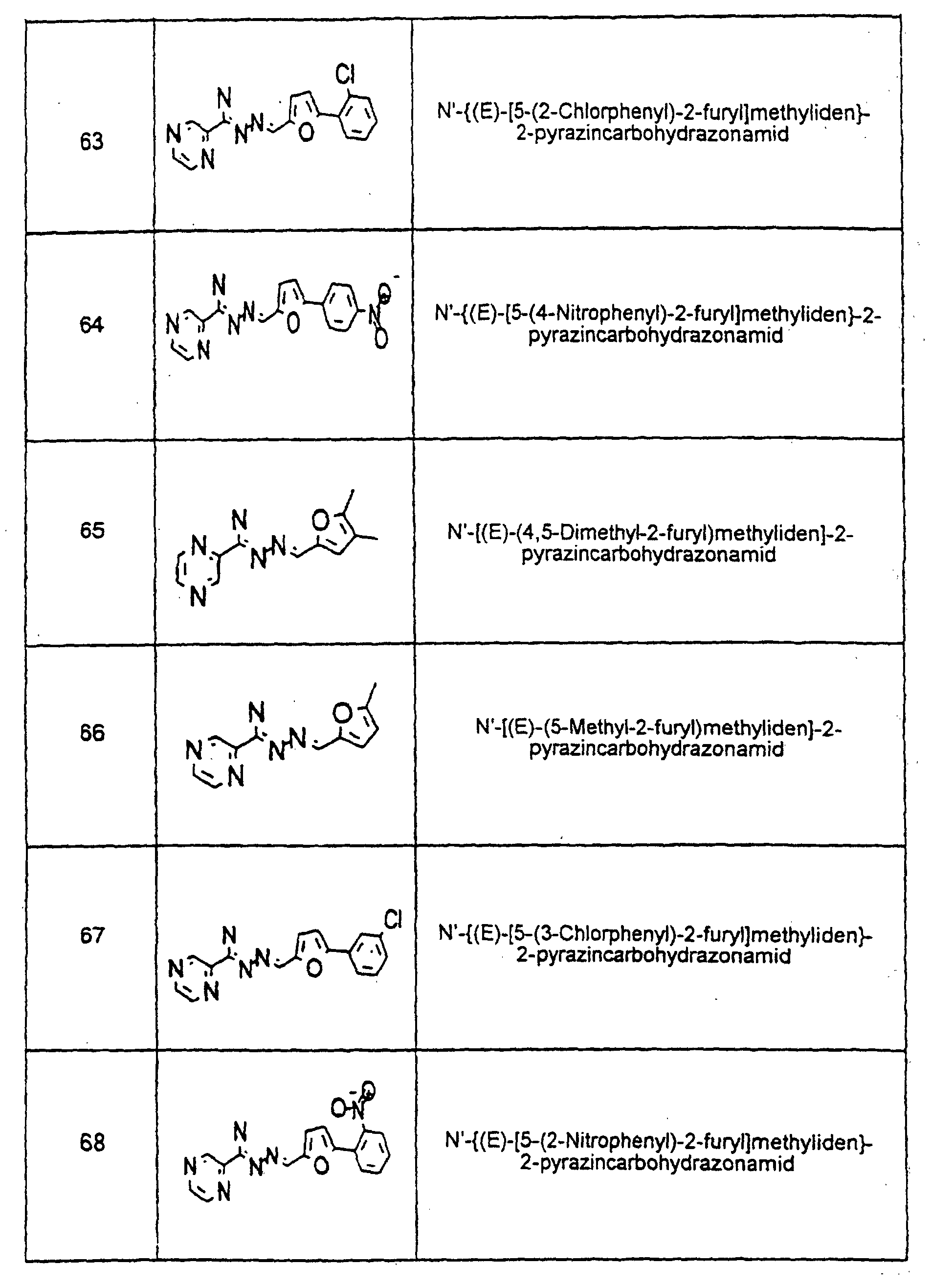

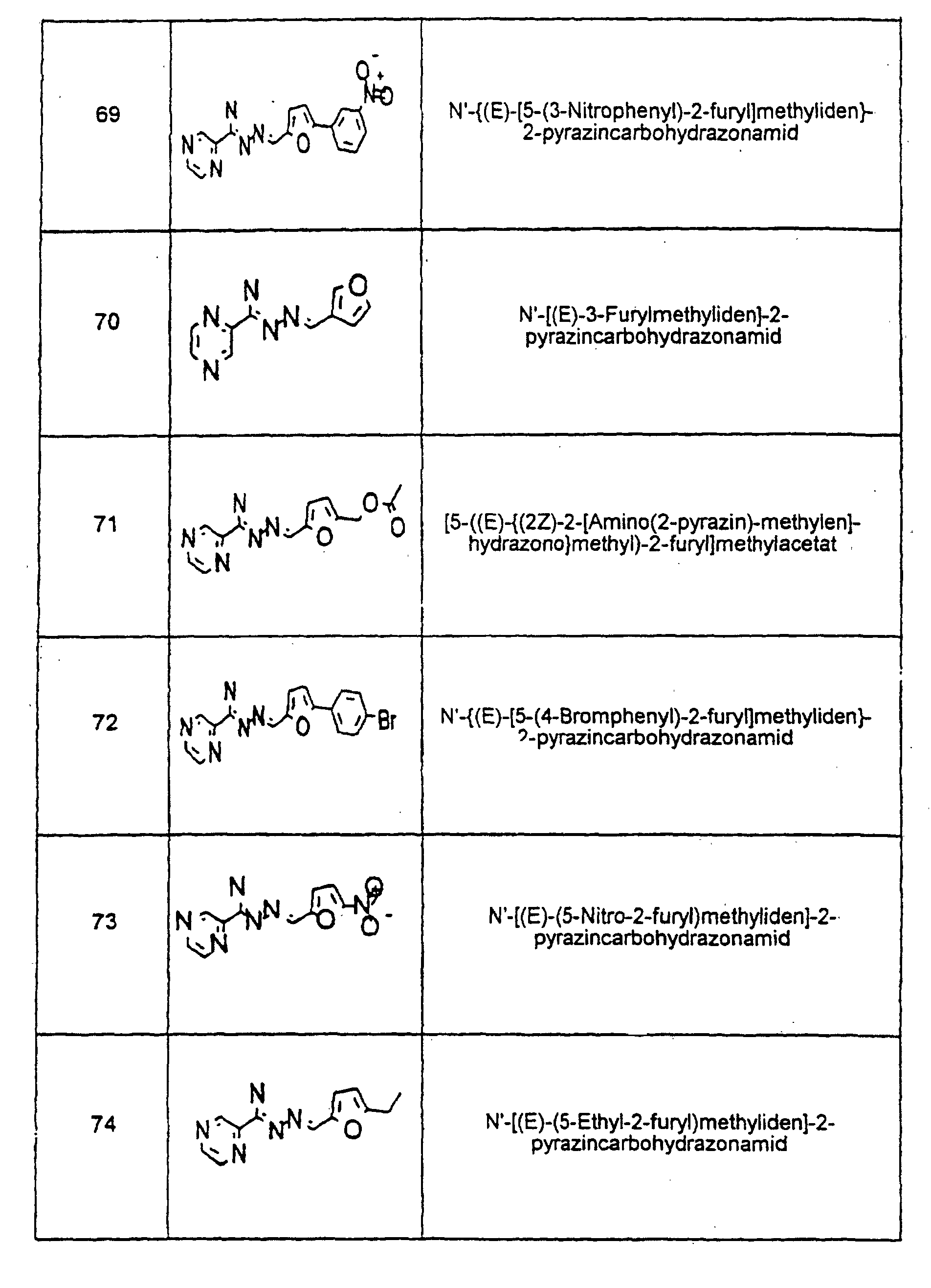

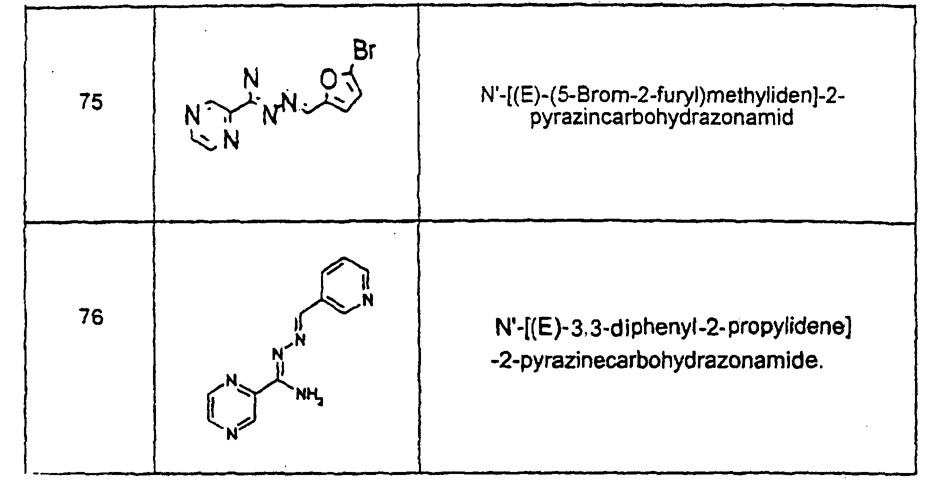

3. Zusammensetzung nach Anspruch 1, wobei das Aminoamidinderivat das N'-[(E)-(3-Methoxyphenyl)-methyliden]benzolcarbohydrazonamid der folgenden Struktur ist:

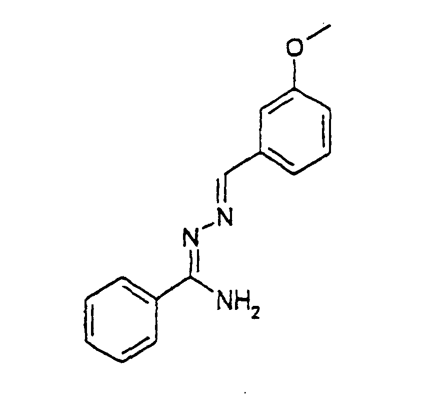

4. Zusammensetzung nach Anspruch 1 oder 2, wobei das Aminoamidinderivat das N'[(E)-3-Pyridinylmethyliden]-2-pyrazincarbohydrazonamid der folgenden Struktur ist:

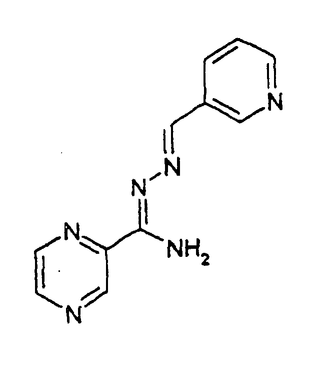

5. Zusammensetzung nach Anspruch 1 oder 2, wobei das Aminoamidinderivat das N'-[(E)-2-ydroxyphenylmethyliden]pyrazin carbohydrazonamid der folgenden Struktur ist:

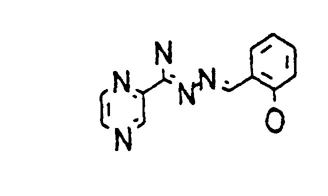

6. Zusammensetzung nach einem der Ansprüche 1 bis 5, **dadurch gekennzeichnet, dass** die Verbindung der Formel (I) oder (II) in der Zusammensetzung in einer Menge von 0,1 bis 20 Gew.-%, bezogen auf das Gesamtgewicht der Zusammensetzung, und vorzugsweise im Bereich von 0,5 bis 10 Gew.-%, bezogen auf das Gesamtgewicht der Zusammensetzung, vorliegt.

7. Zusammensetzung nach einem der Ansprüche 1 bis 6, **dadurch gekennzeichnet, dass** der kosmetisch akzeptable Träger in Form einer Emulsion vom Öl-in-Wasser-Typ vorliegt.

8. Zusammensetzung nach einem der Ansprüche 1 bis 7, **dadurch gekennzeichnet, dass** sie ferner ein oder mehrere ergänzende, im UV-A- und UV-B-Bereich wirksame organische Filter enthält.

9. Zusammensetzung nach Anspruch 8, **dadurch gekennzeichnet, dass** die ergänzenden organischen Filter unter Zimtsäurederivaten; Dibenzoylmethanderivaten; Salicylsäurederivaten; Campherderivaten; Triazinderivaten; Benzophenonderivaten; ß,ß-Diphenylacrylatderivaten; Benzimidazolderivaten; Bisbenzoazolylderivaten; p-Aminobenzoesäurederivaten; polymeren Filtern und Siliconfiltern; und unlöslichen organischen mikronisierten UV-Filtern ausgewählt sind.

10. Zusammensetzung nach einem der Ansprüche 1 bis 9, **dadurch gekennzeichnet, dass** sie ferner als zusätzliche UV-Lichtschutzmittel Pigmente oder Nanopigmente von Metalloxiden, die gegebenenfalls umhüllt sind, enthält.

11. Zusammensetzung nach Anspruch 10, **dadurch gekennzeichnet, dass** die Pigmente oder Nanopigmente unter den Oxiden von Titan, Zink, Eisen, Zirconium, Cer und deren Gemischen ausgewählt sind, wobei diese umhüllt oder nicht umhüllt vorliegen.

12. Zusammensetzung nach einem der Ansprüche 1 bis 11, **dadurch gekennzeichnet, dass** sie ferner mindestens ein Mittel zur Bräunung und/oder künstlichen Braunfärbung der Haut enthält.

13. Zusammensetzung nach einem der Ansprüche 1 bis 12, **dadurch gekennzeichnet, dass** sie außerdem mindestens einen Zusatzstoff enthält, der unter den Fettsubstanzen, organischen Lösungsmitteln, Verdickungsmitteln, reizlindernden Stoffen, Antioxidantien, Trübungsmitteln, Stabilisierungsmitteln, Emollientien, Schaumverhütungsmitteln, Hydratisierungsmitteln, Parfums, Konservierungsmitteln, grenzflächenaktiven Stoffen, Maskierungsmitteln, Polymeren, Treibmitteln, Alkalisierungsmitteln oder Ansäuerungsmitteln ausgewählt ist.

14. Zusammensetzung nach einem der Ansprüche 1 bis 12, **dadurch gekennzeichnet, dass** es sich um eine Zusammensetzung zum Schutz der menschlichen Epidermis oder eine Lichtschutzzusammensetzung handelt und dadurch, dass sie als nichtionische Vesikeldispersion, Emulsion und insbesondere Emulsion vom Öl-in-Wasser-Typ, Creme, Milch, Gel, Gelcreme, Suspension, Dispersion, Pulver, fester Stift, Schaum oder Spray vorliegt.

15. Zusammensetzung nach einem der Ansprüche 1 bis 12, **dadurch gekennzeichnet, dass** es sich um eine Zusammensetzung zum Schminken der Wimpern, Augenbrauen, Nägel oder der Haut handelt und dadurch, dass sie in fester oder pastöser, wässriger oder wasserfreier Form als Emulsion, Suspension oder Dispersion vorliegt.

16. Zusammensetzung nach einem der Ansprüche 1 bis 12, **dadurch gekennzeichnet, dass** es sich um eine Zusammensetzung zum Schutz der Haare gegen UV-Strahlung handelt und dadurch, dass sie als Haarwaschmittel, Lotion, Gel, Emulsion oder nichtionische Vesikeldispersion vorliegt.

17. Verwendung eines Aminoamidinderivats der Formel (I) nach einem der Ansprüche 1 bis 5 zur Herstellung einer Zusammensetzung, die dazu vorgesehen ist, gegenüber UV-Strahlung und insbesondere gegenüber Sonnenlicht empfindliche Materialien zu schützen.

18. Verwendung eines Aminoamidiriderivats der Formel (I) nach einem der Ansprüche 1 bis 5 als UV-Filter zur Herstellung einer kosmetischen oder dermatologischen Zusammensetzung.

19. Verwendung eines Aminoamidinderivats der Formel (I) nach einem der Ansprüche 1 bis 5 als Mittel, um die durch UV-Strahlung verursachte Farbänderung der Haut zu kontrollieren, für die Herstellung einer kosmetischen Zusammensetzung.

20. Aminoamidinderivat, das unter den folgenden Verbindungen ausgewählt ist:

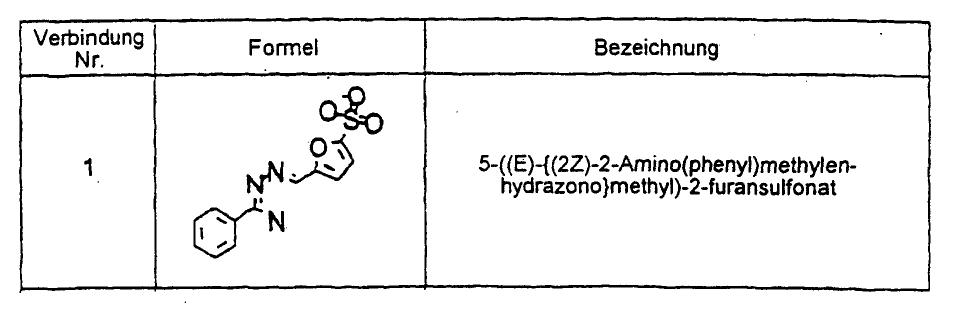

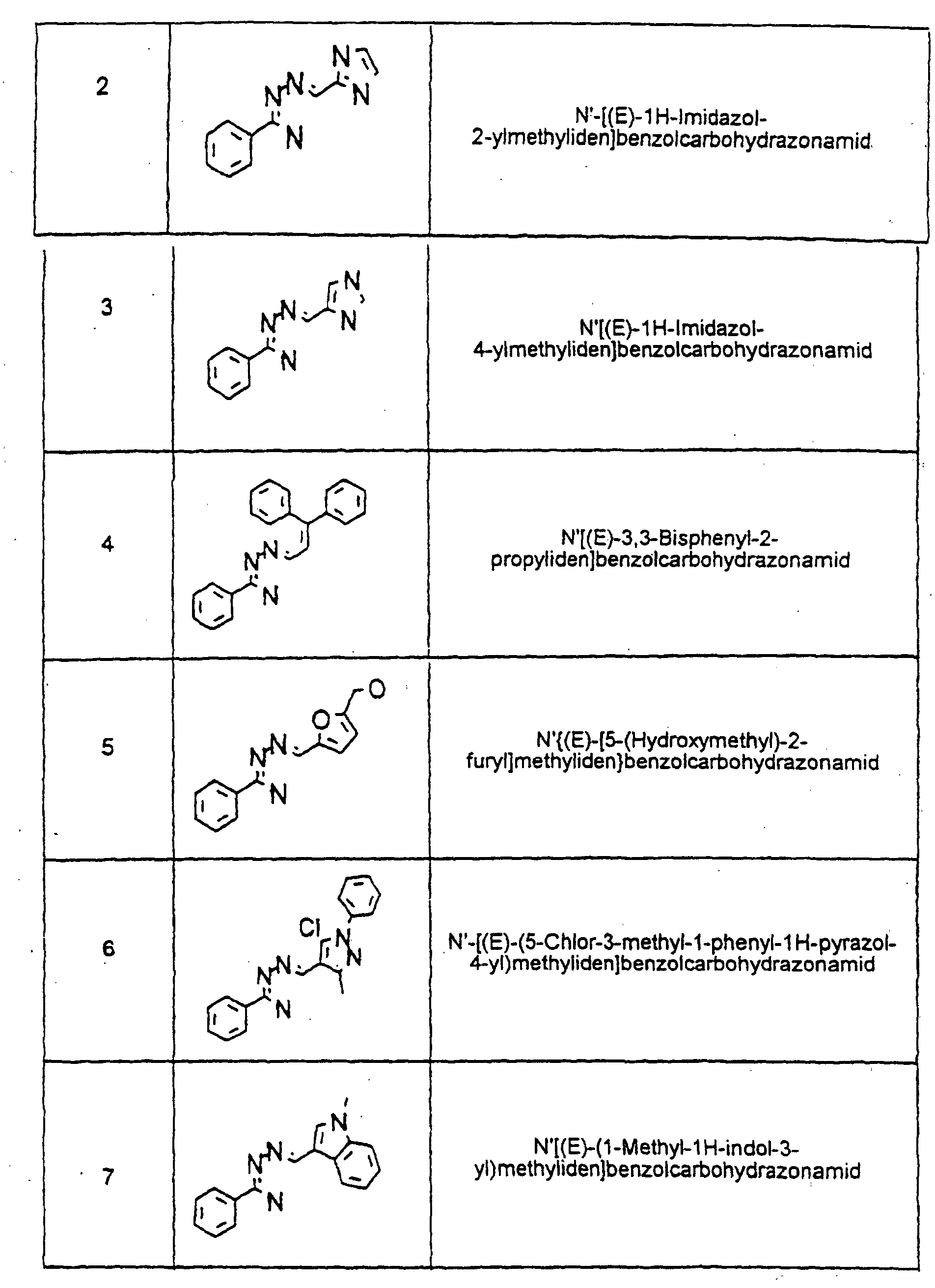

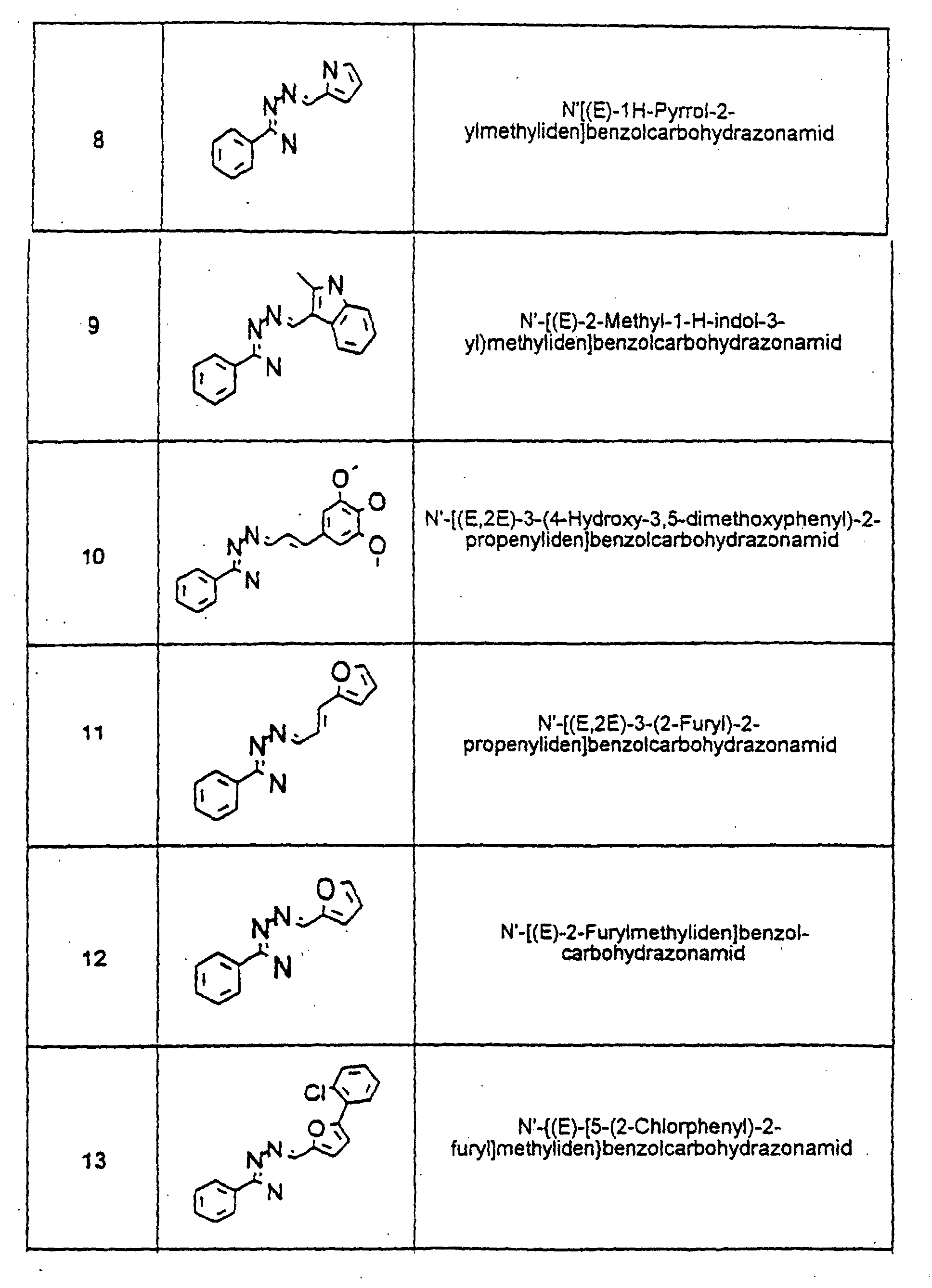

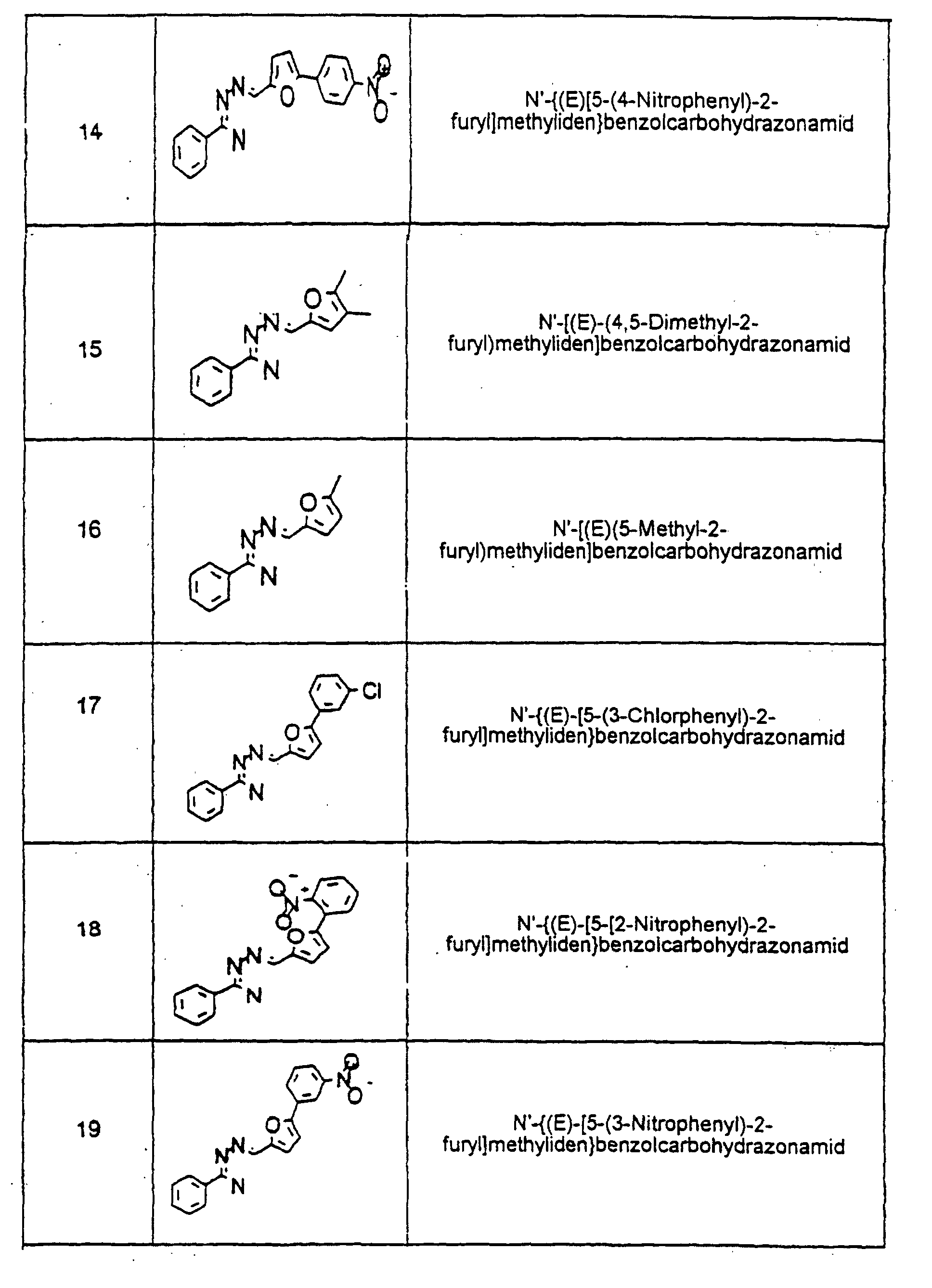

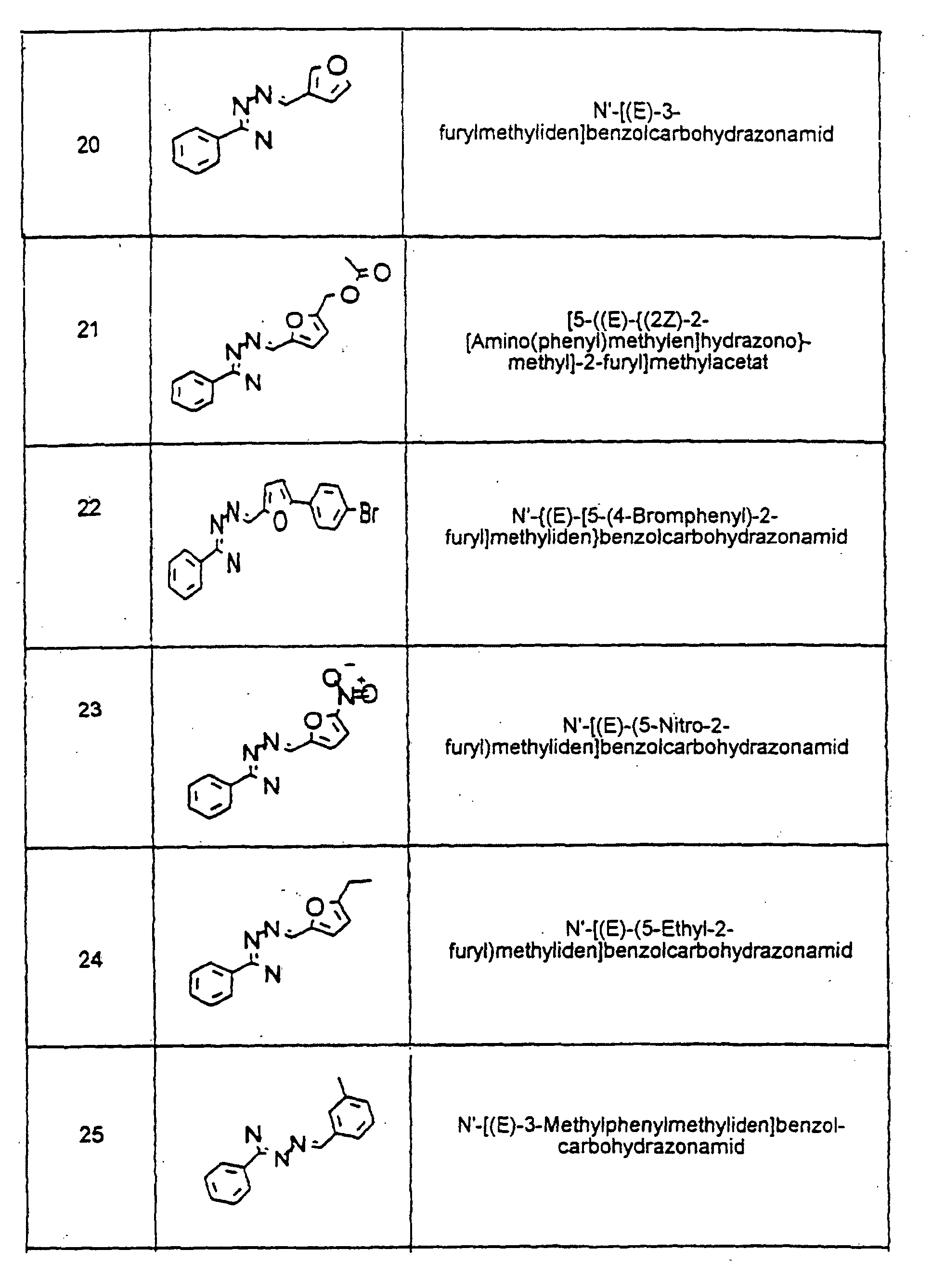

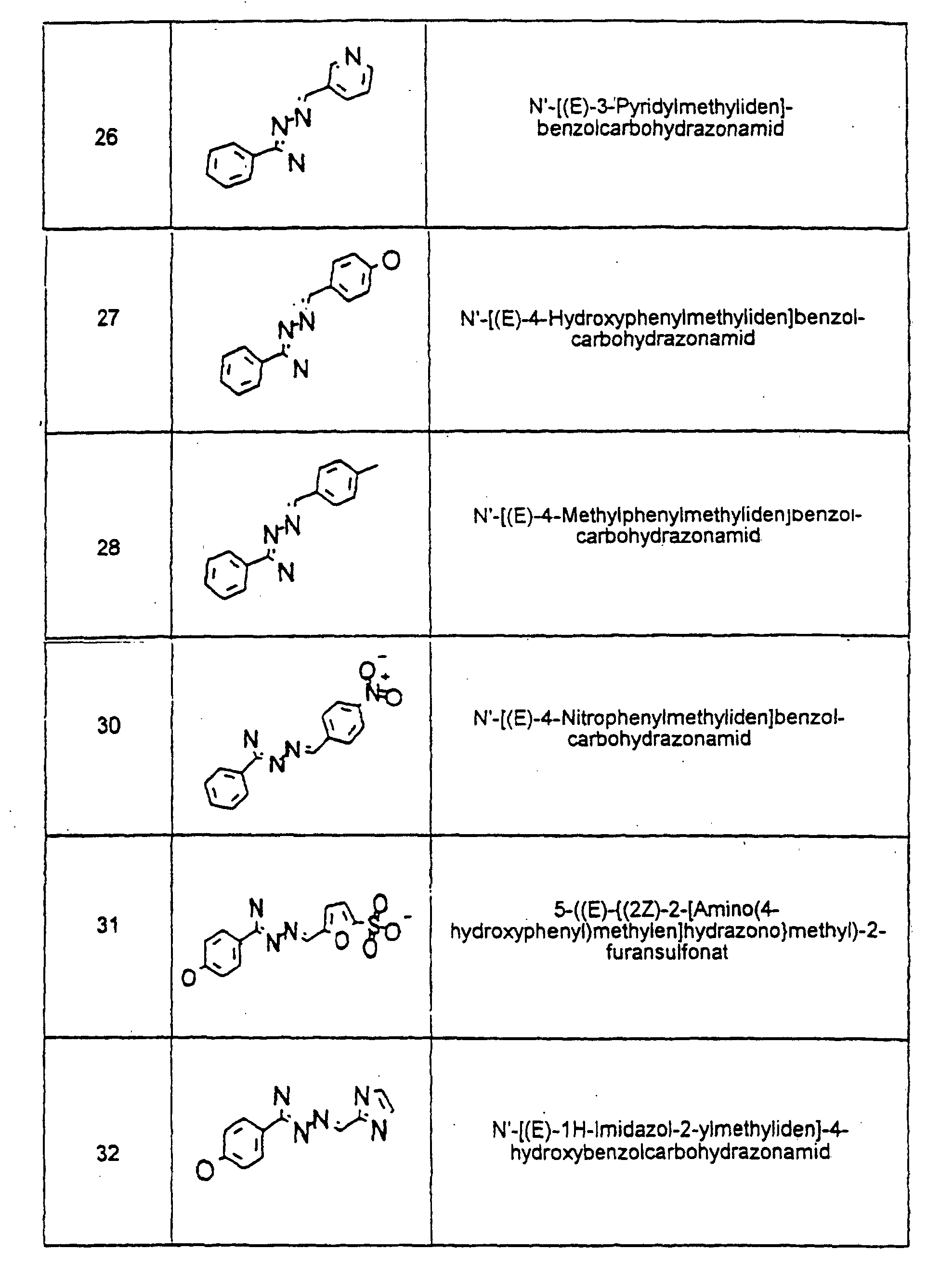

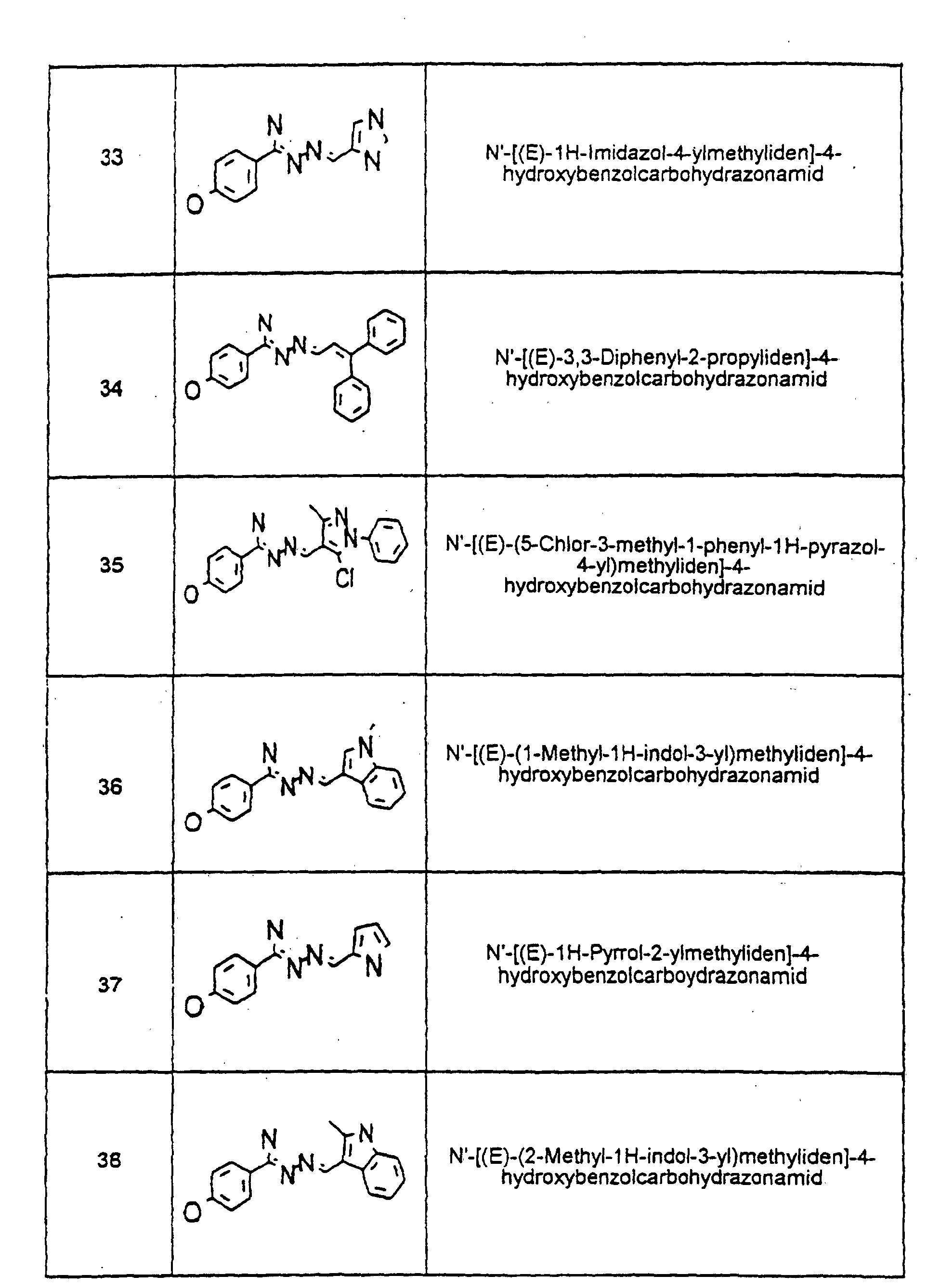

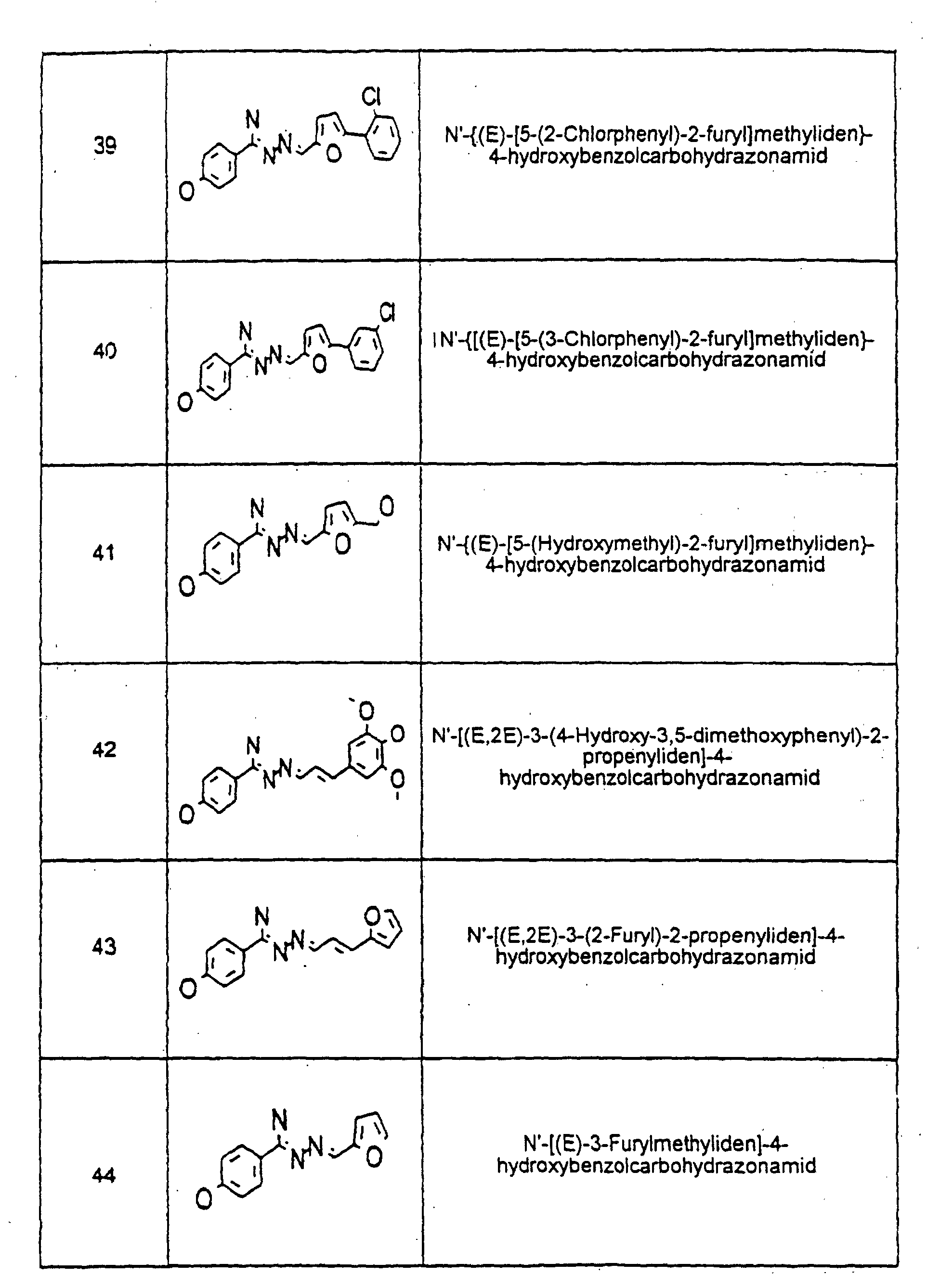

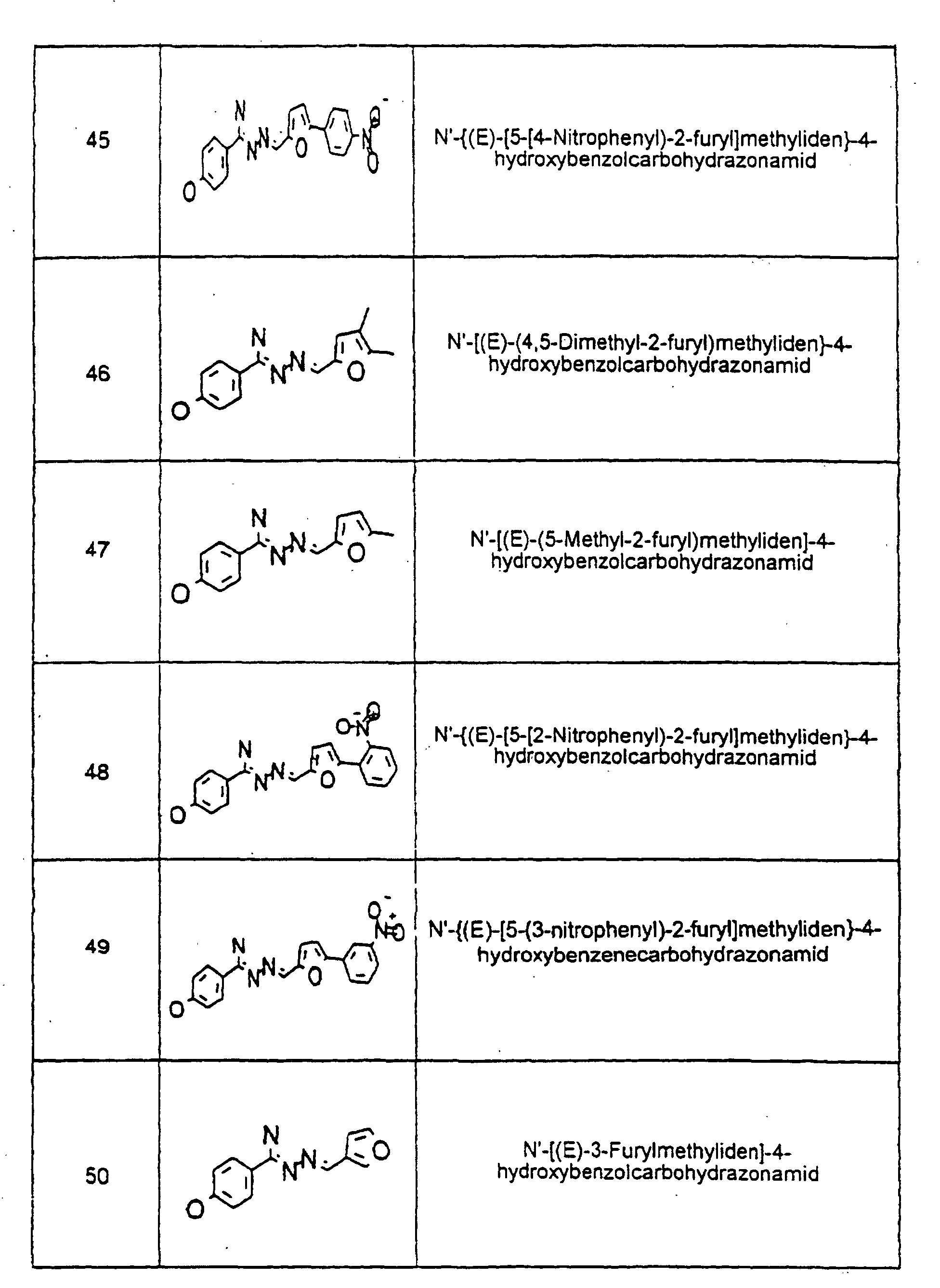

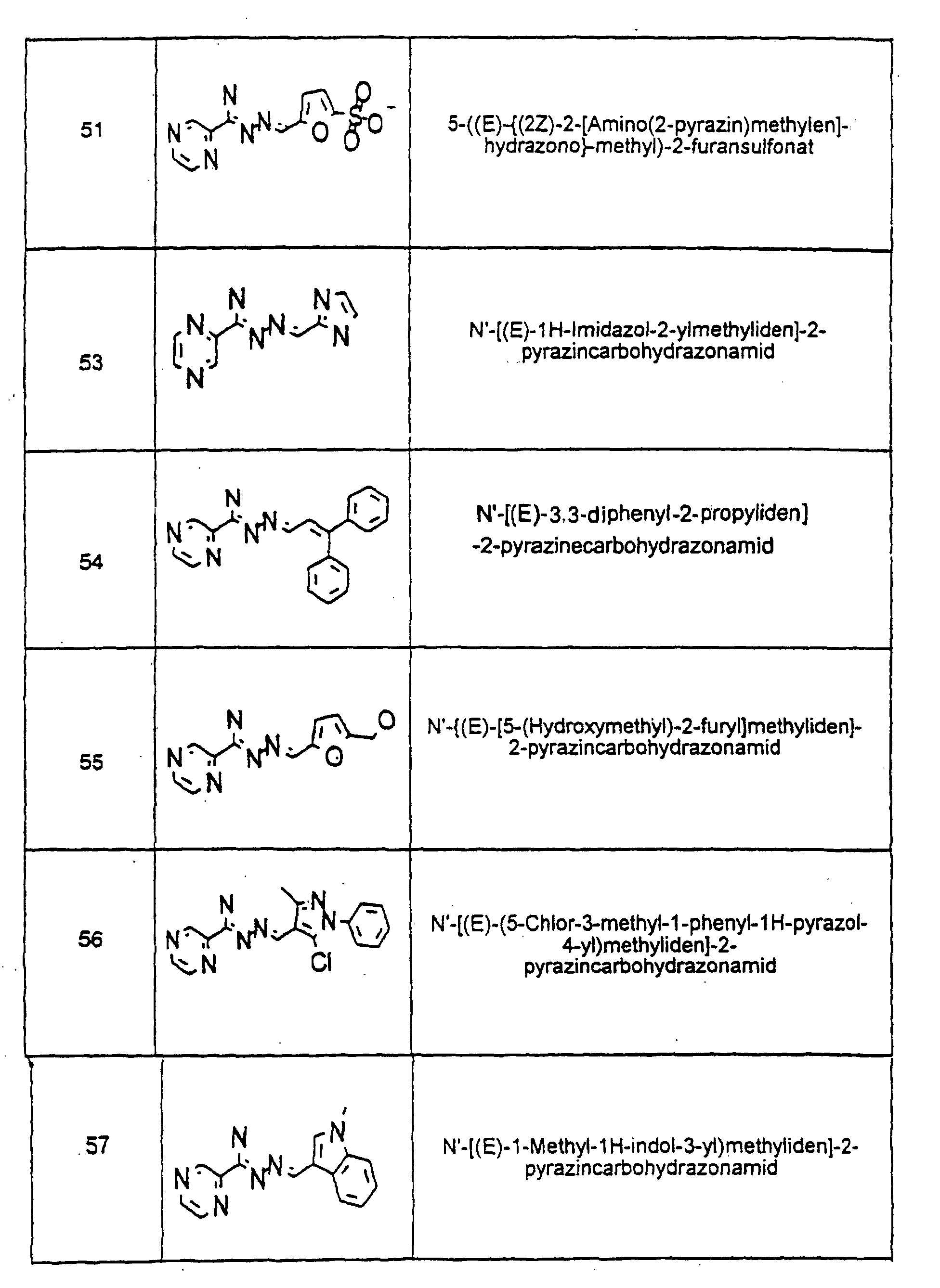

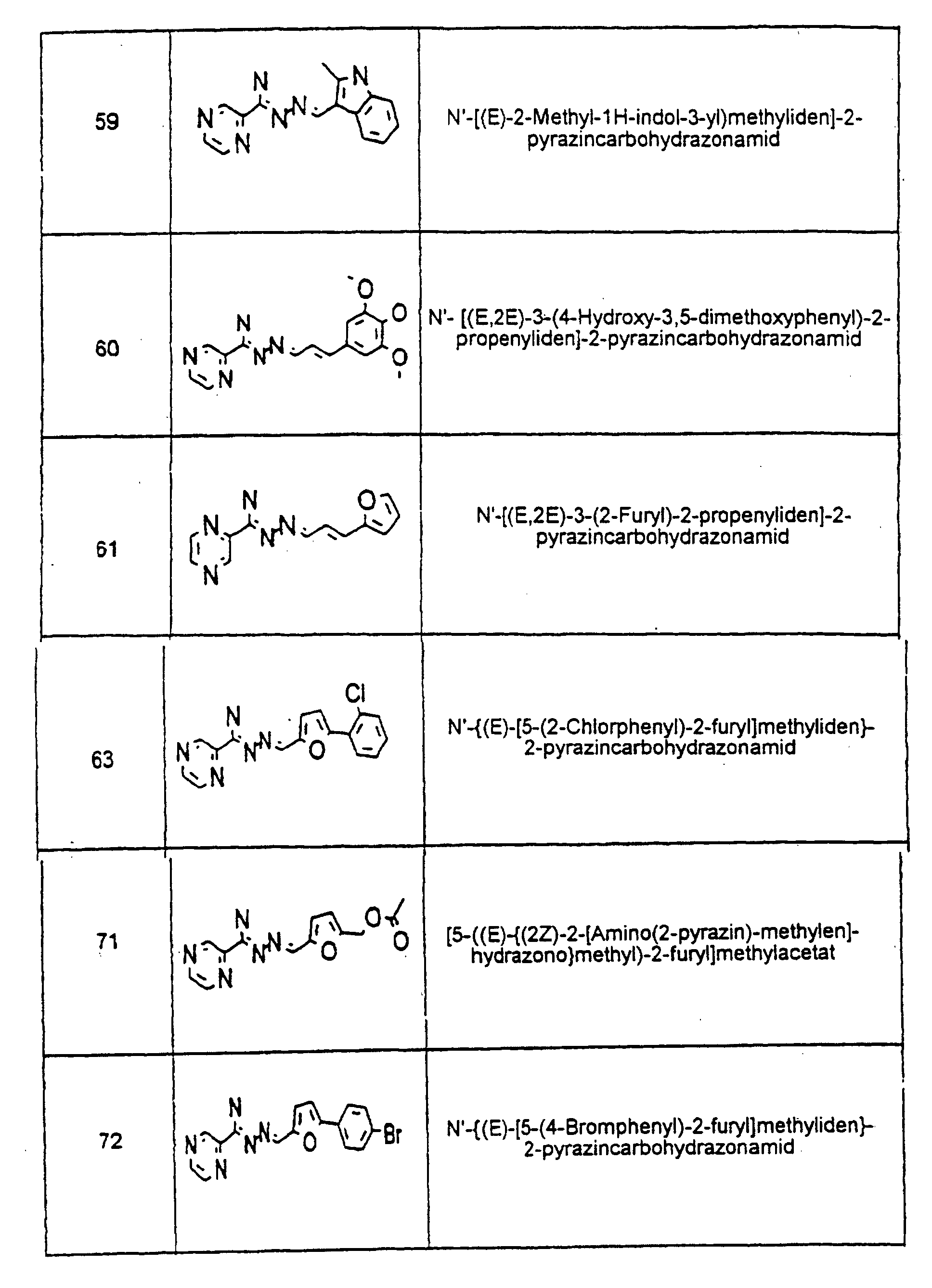

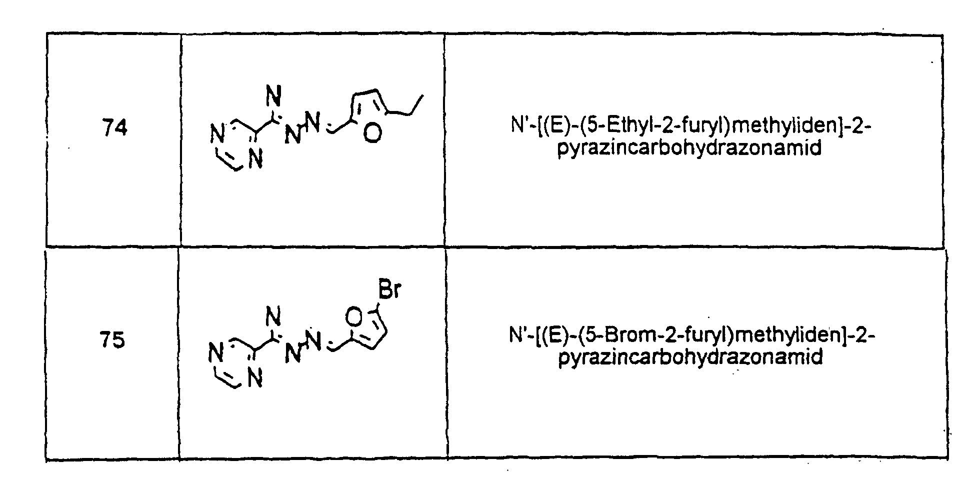

## Claims

1. Cosmetic composition for topical use, in particular for the photoprotection of the skin and/or of the hair, **characterized in that** it comprises, in a cosmetically acceptable carrier, at least one aminoamidine derivative corresponding to the following formula (I) : 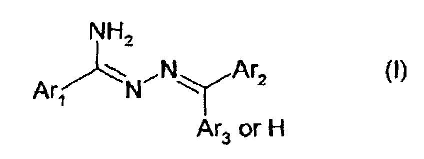 in which:
- Ar₁ represents
(i) an aromatic ring having the following formula: 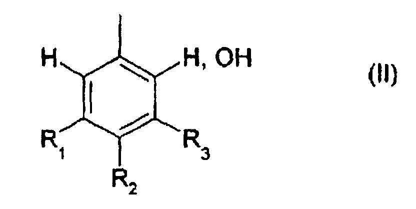 where R₁, R₂, R₃, which are identical or different, denote a hydrogen atom; a linear or branched C₁-C₈ alkyl radical; a C₁-C₈ alkoxy radical;
R₁, R₂, R₃ can form an aromatic or unfused ring or alternatively an aromatic heterocycle containing 5 or 6 members;
(ii) an aromatic nucleus containing 6 atoms, corresponding to the following structure: 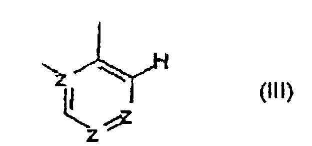
where 2 denotes C or N;
- Ar₂ represents an aromatic nucleus corresponding to the definition of Ar₁ as defined above or alternatively an aromatic nucleus containing 5 atoms corresponding to the following structure: 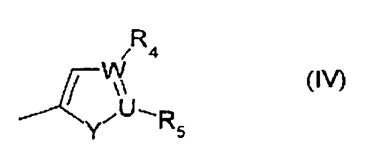 in which:
- Y represents a sulphur, oxygen, carbon or nitrogen atom;
- W represents a carbon or nitrogen atom;
- R₄ denotes:
(i) a hydrogen atom when W is a nitrogen atom;
(ii) R₄ denotes a hydrogen atom, a linear or branched C₁-C₈ alkyl radical; a C₁-C₈ alkoxy radical or can form an aromatic or unfused ring, when W is a carbon atom;
- U represents a carbon or nitrogen atom, when W is a carbon atom;
- R₅ denotes:
(i) a hydrogen atom when U denotes nitrogen;
(ii) a hydrogen atom; a linear or branched C₁-C₈ alkyl radical; a C₁-C₈ alkoxy radical or can form an aromatic or unfused ring, when U is a carbon atom;
- Ar₃ denotes an aromatic nucleus corresponding to the definition of Ar₂ as defined above or alternatively represents a hydrogen atom.

2. Composition according to Claim 1, where the aminoamidine derivative is chosen from the group consisting of the compounds defined in the following table:

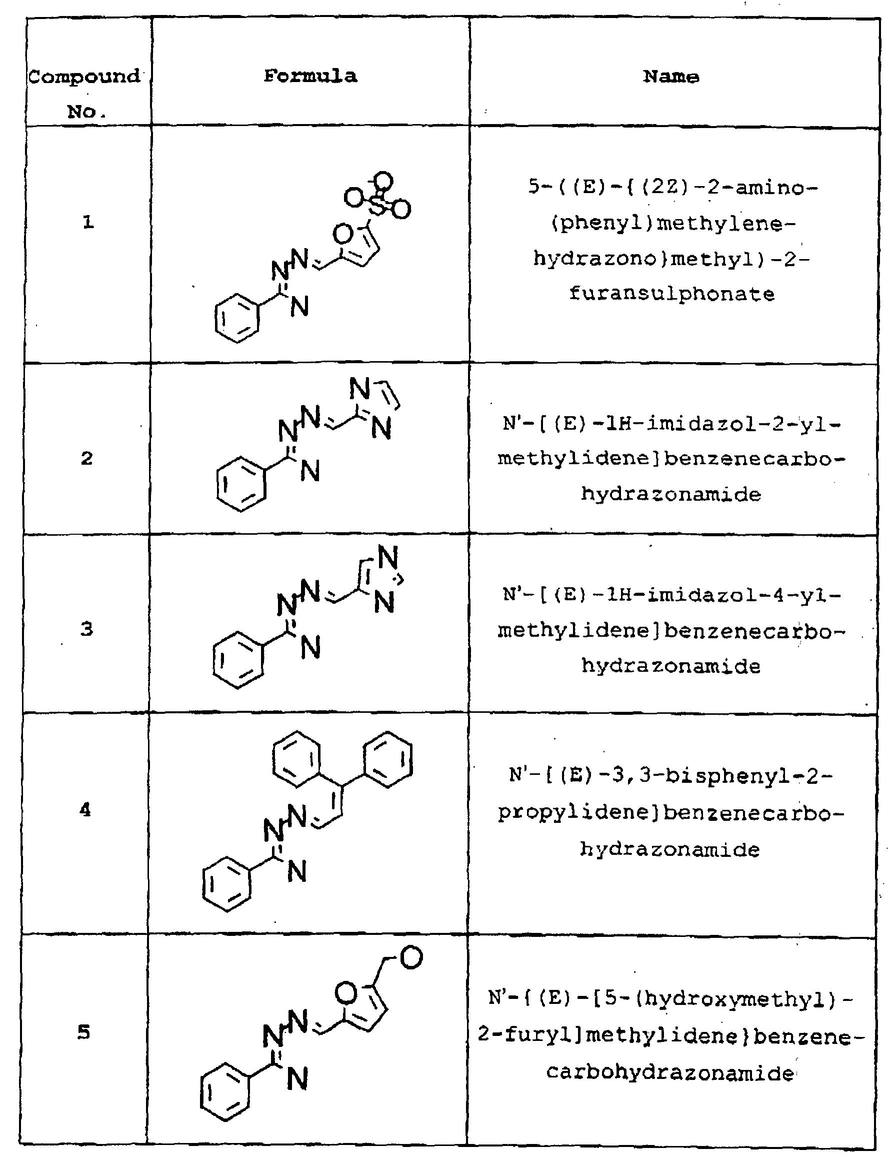

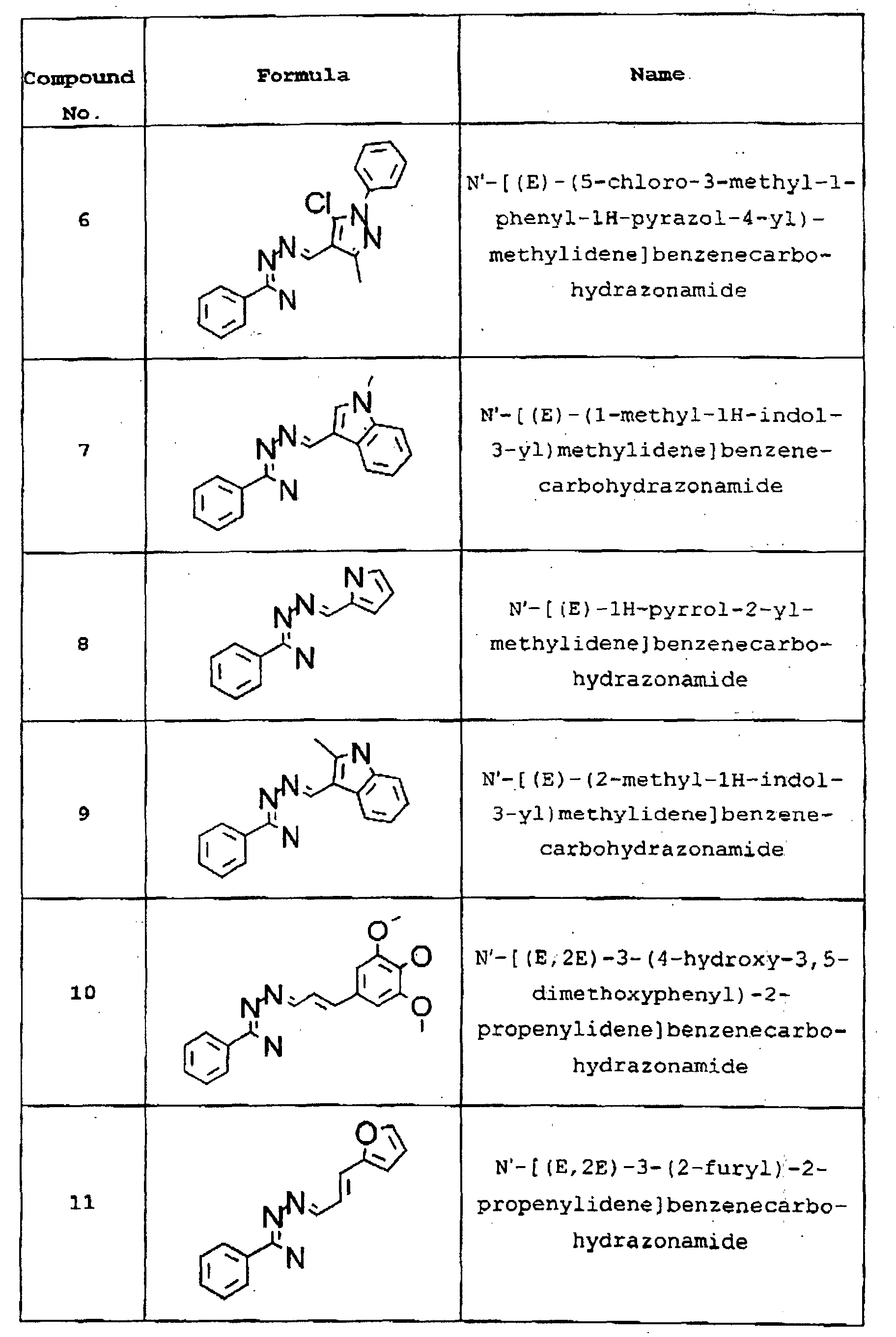

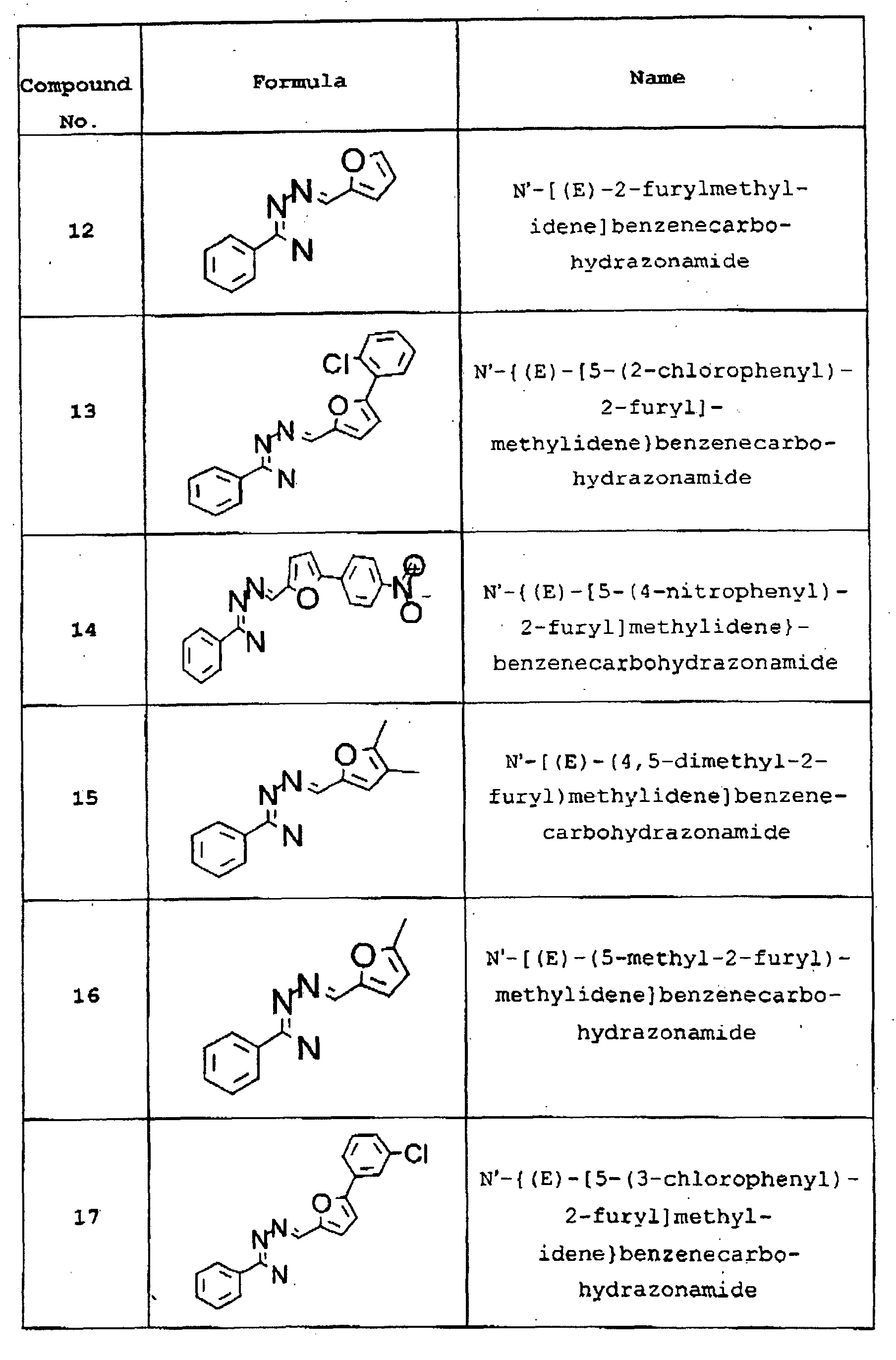

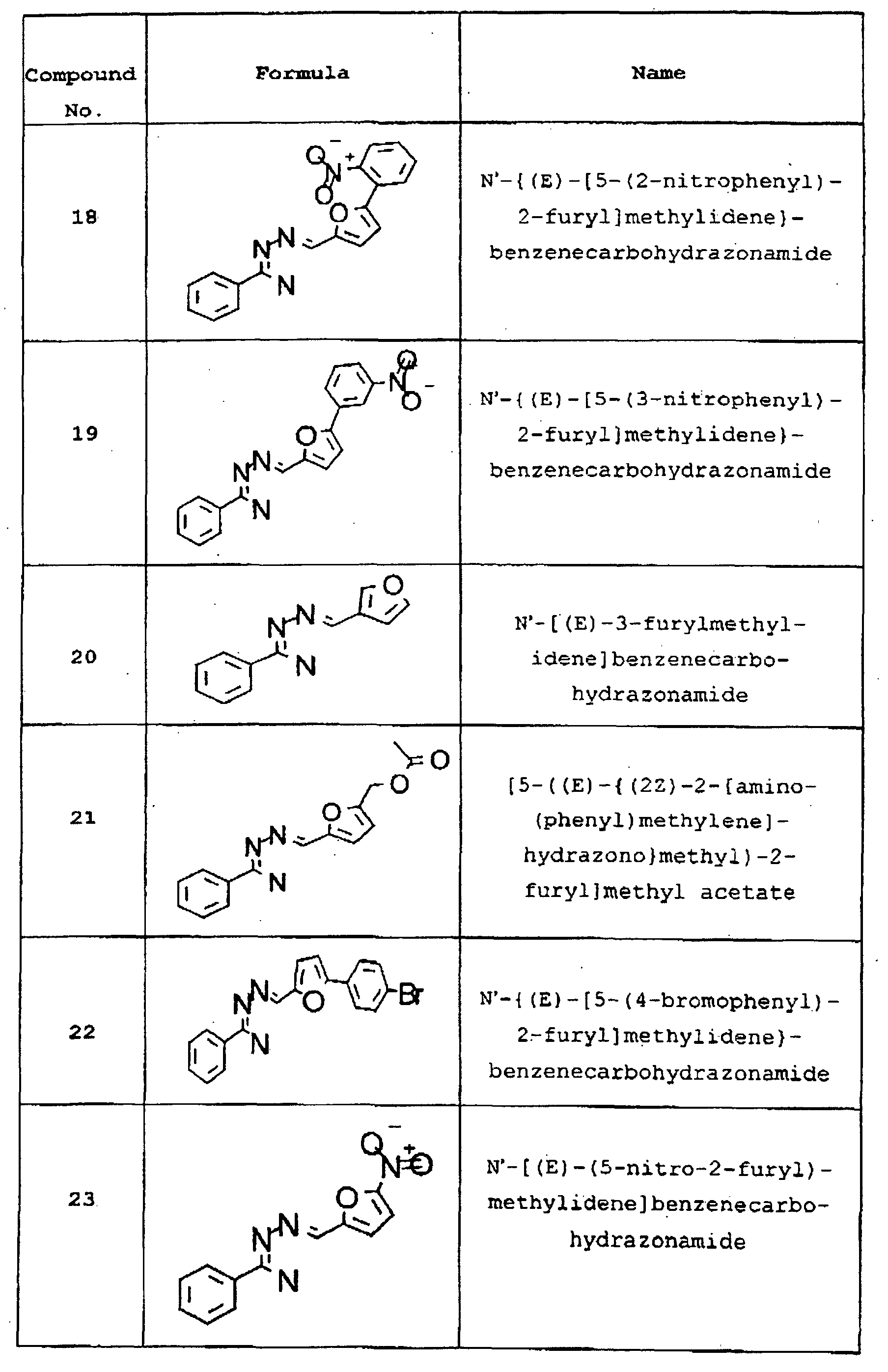

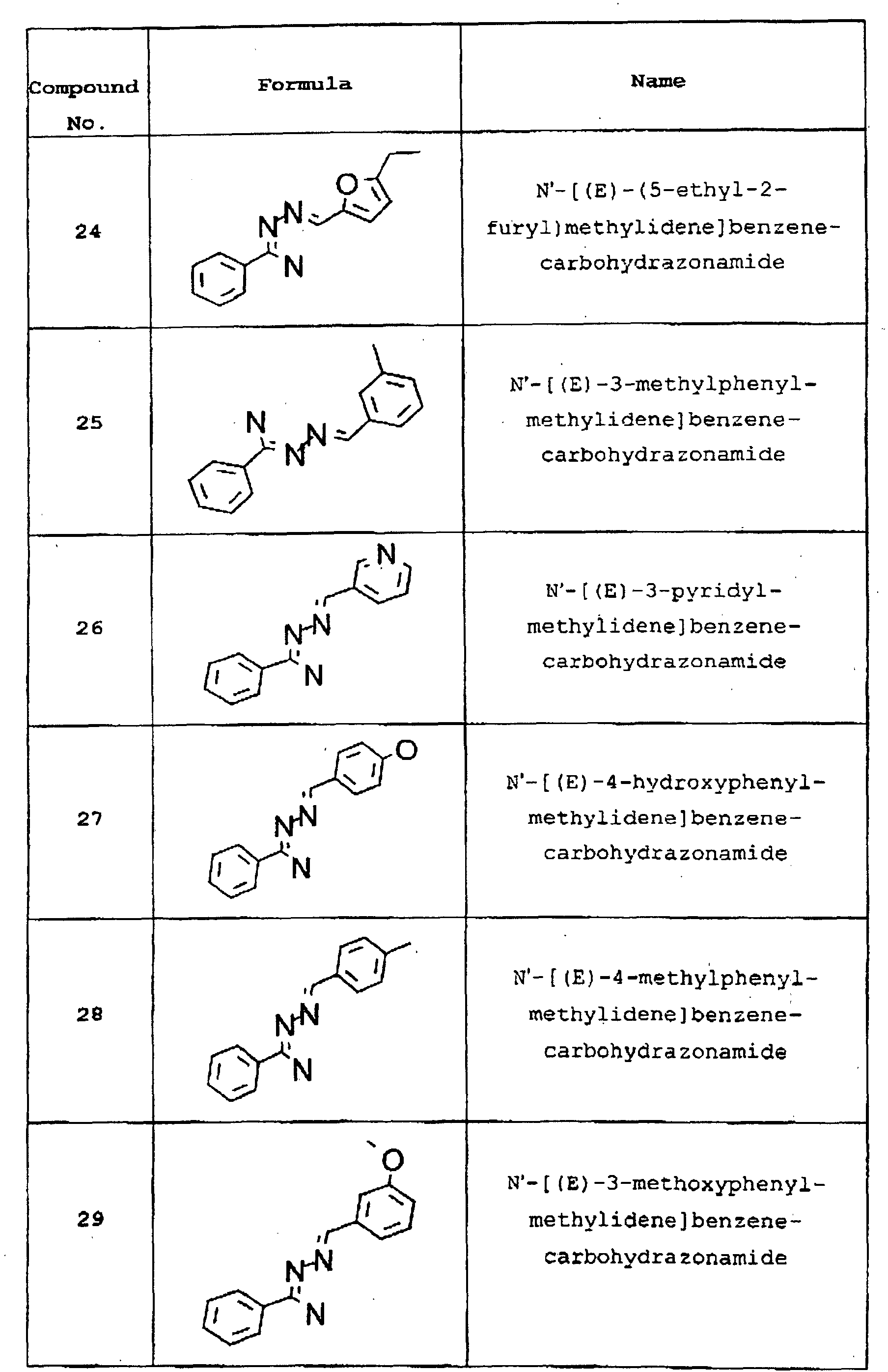

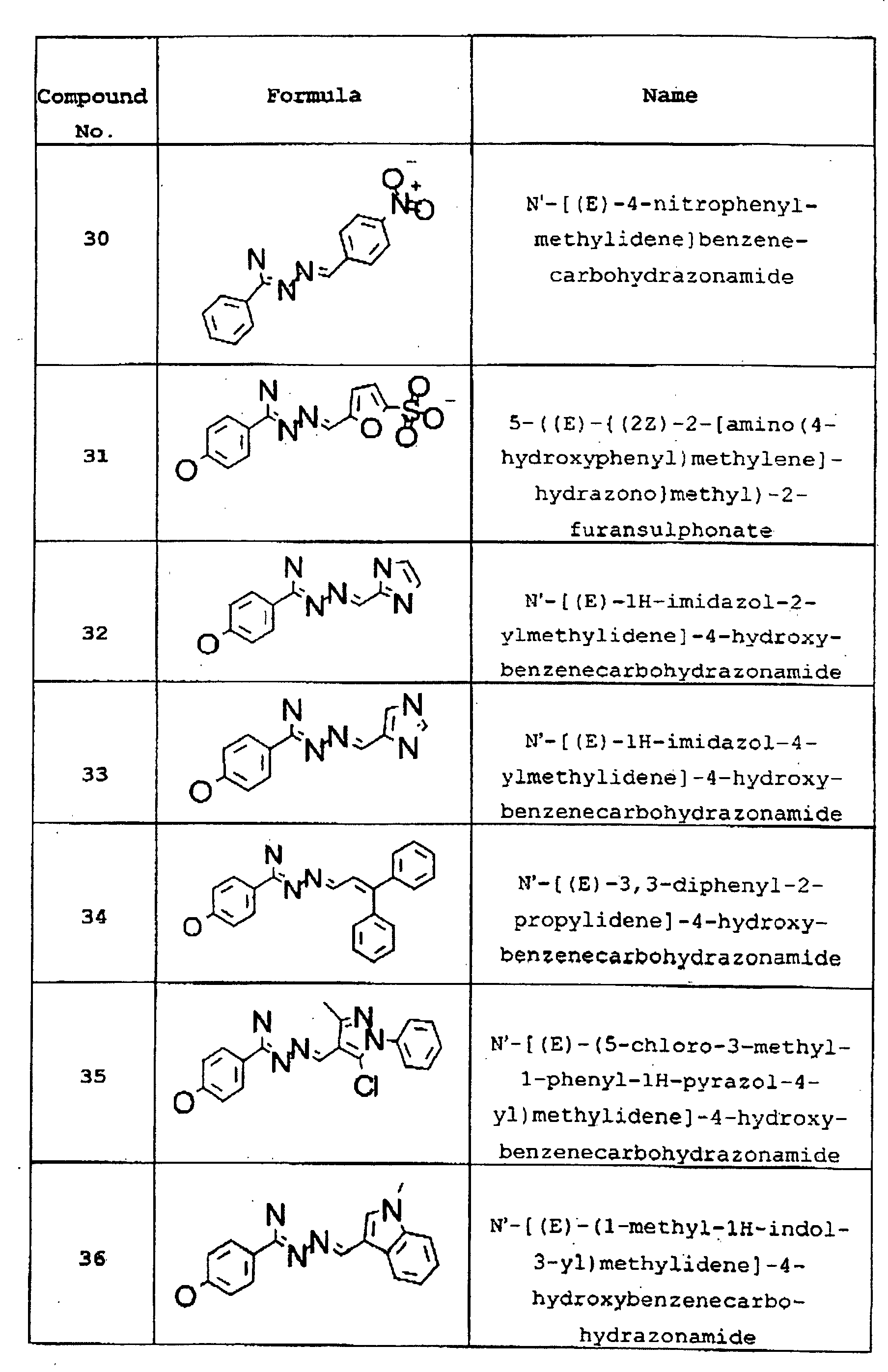

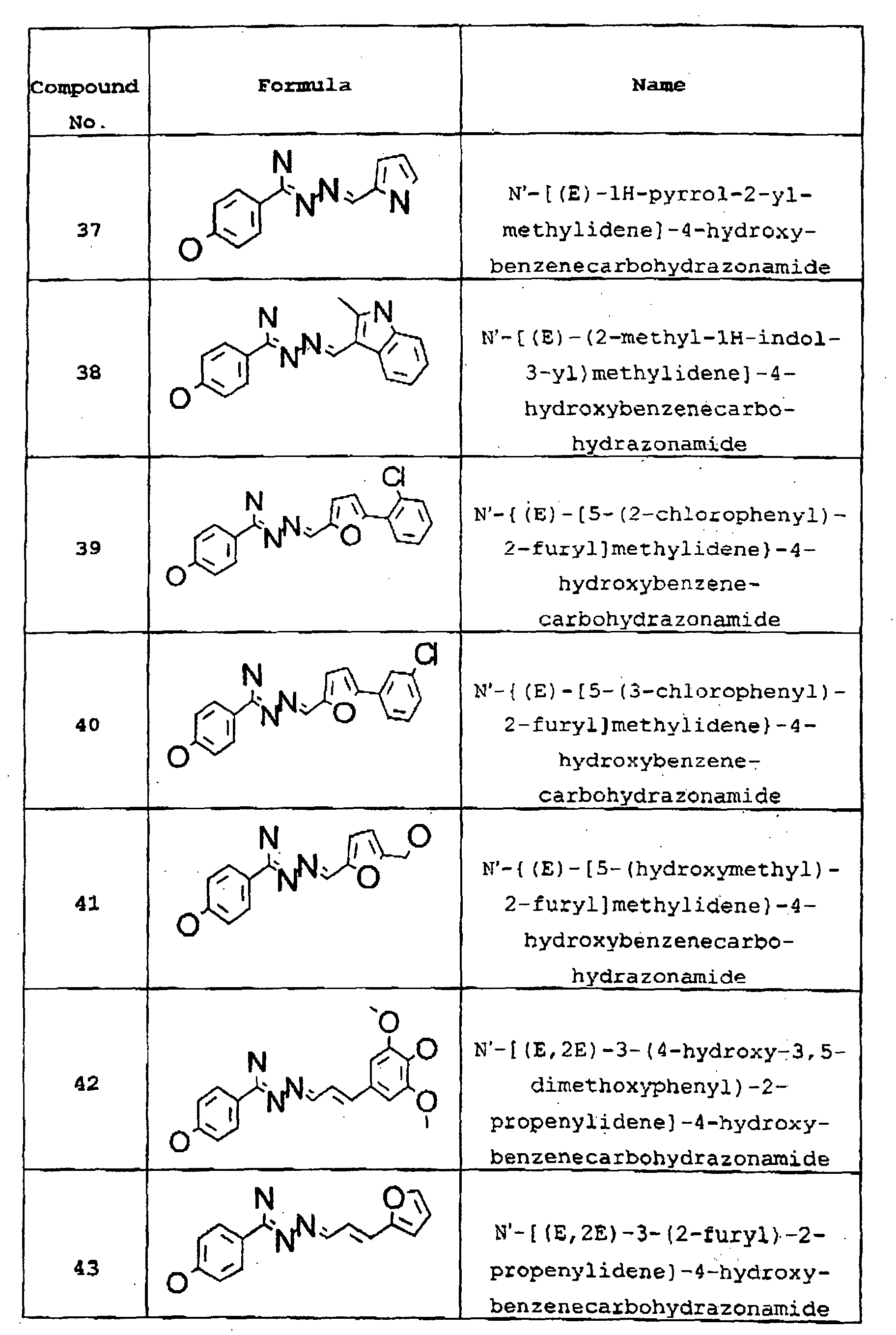

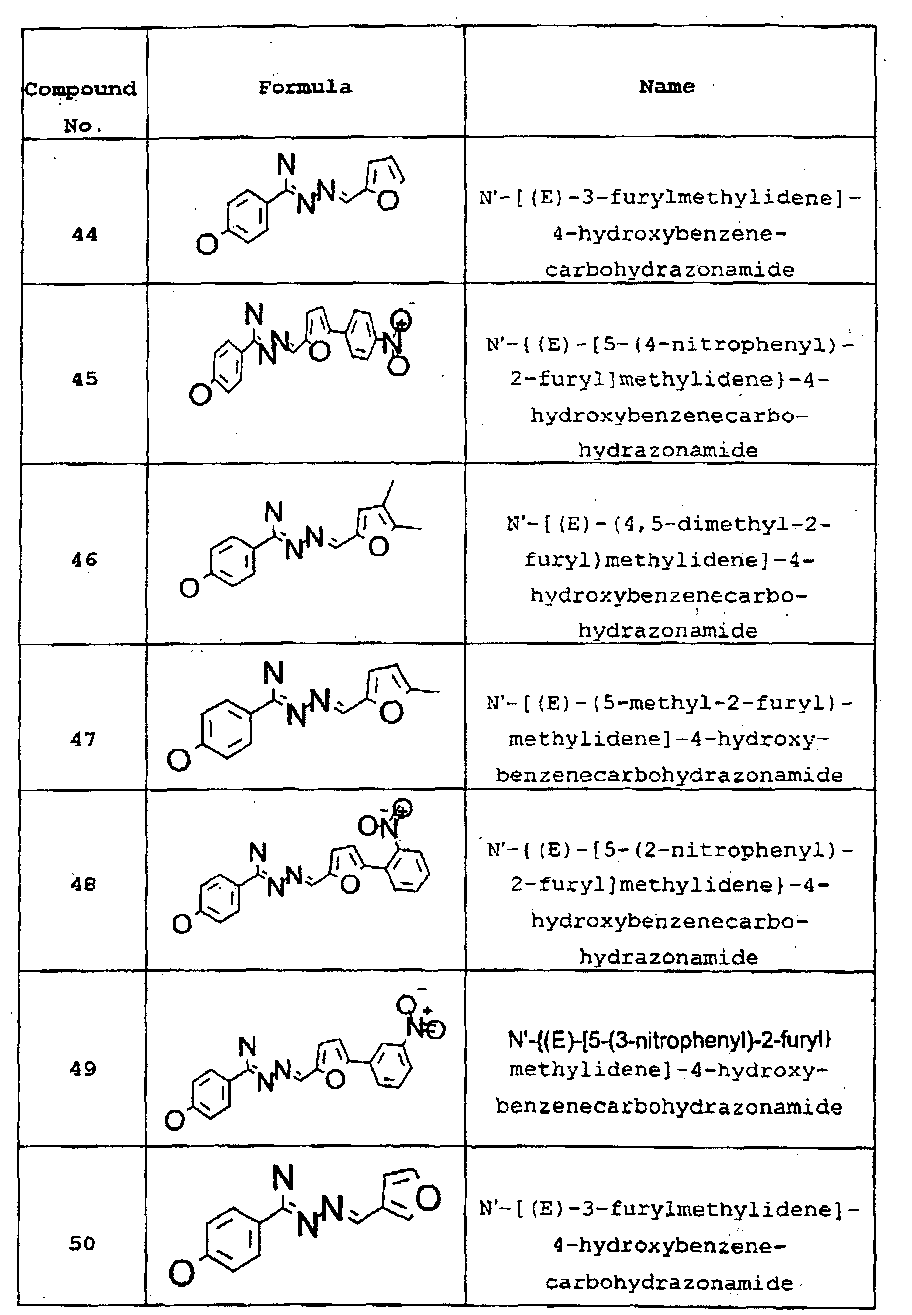

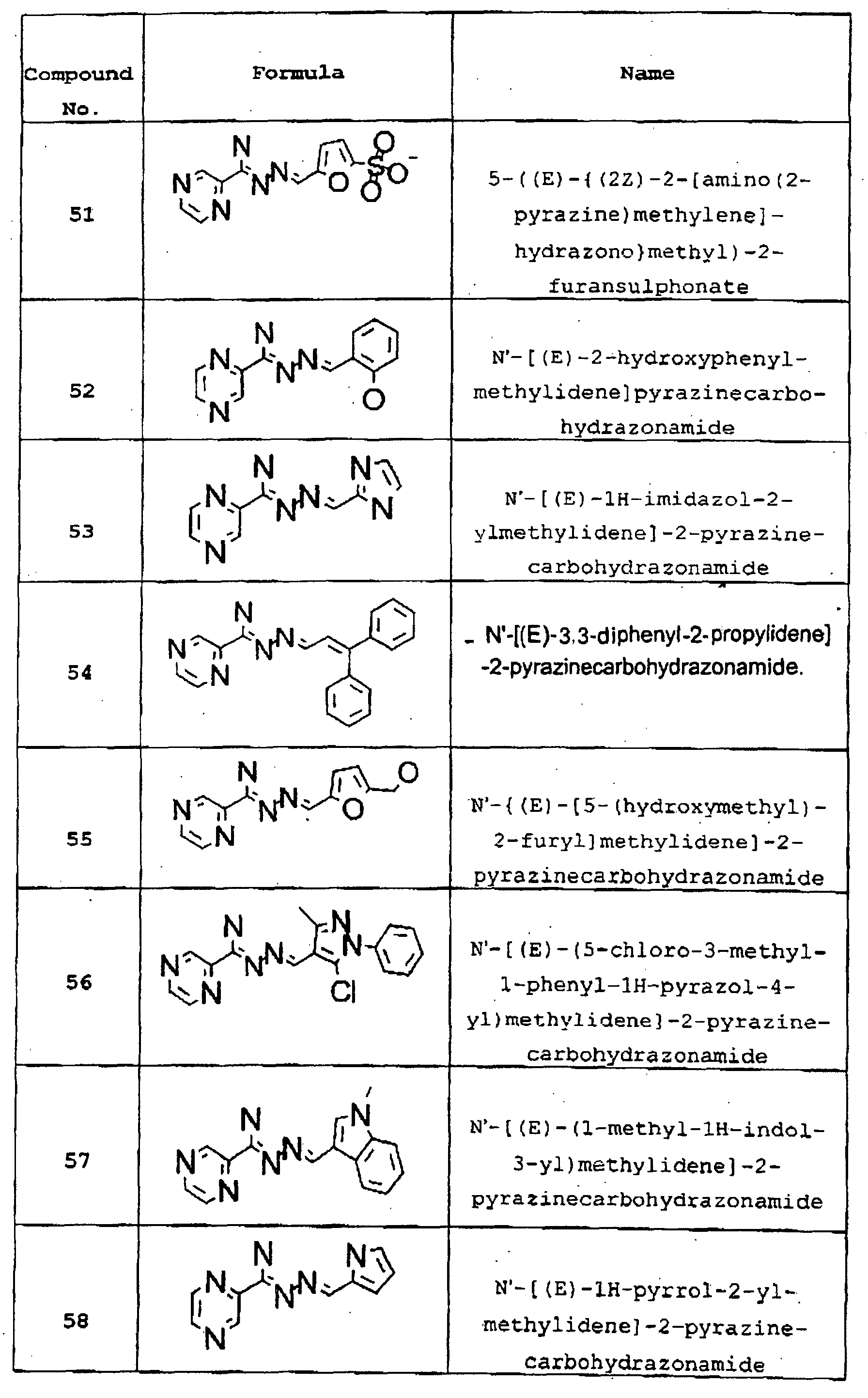

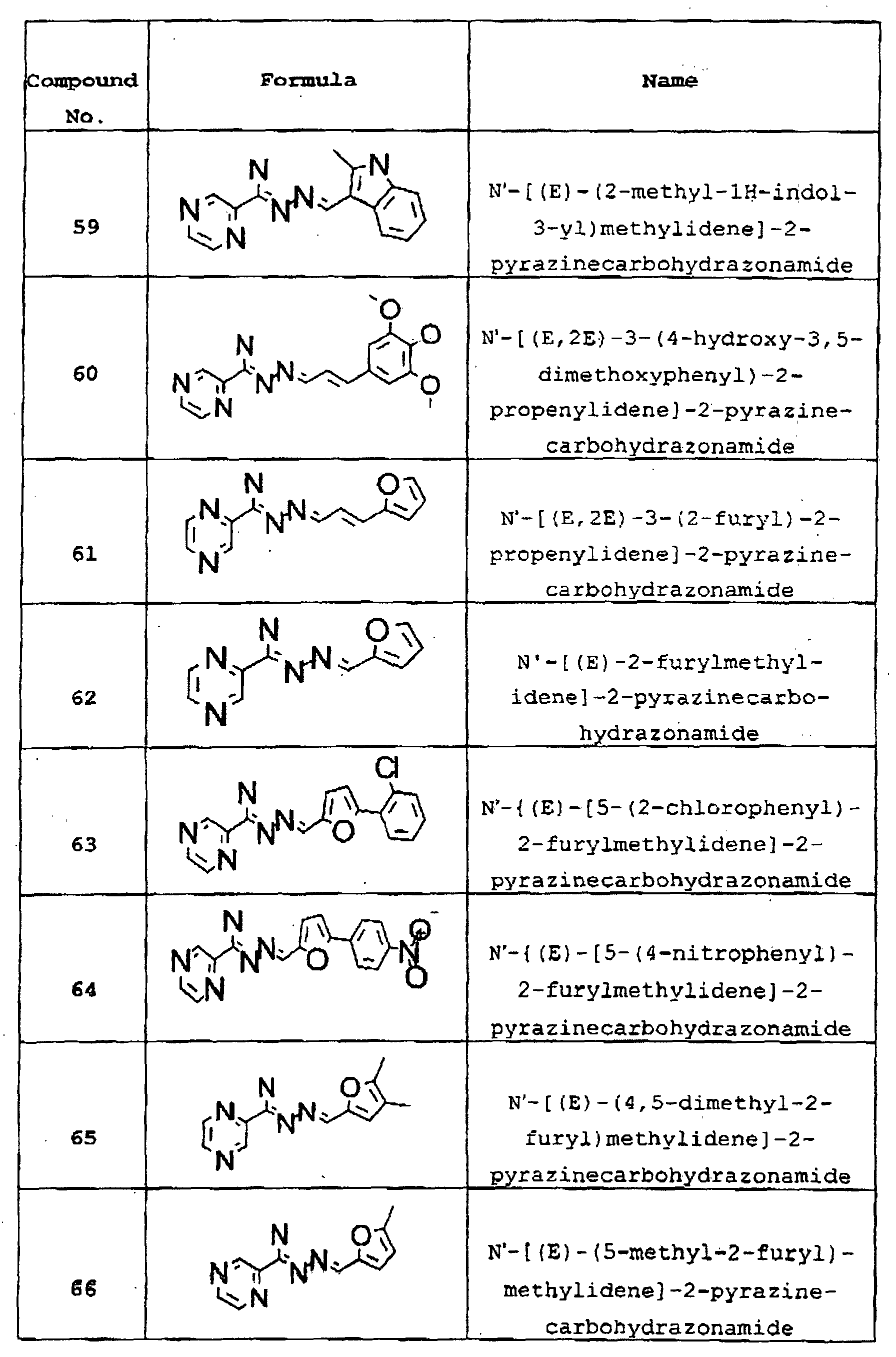

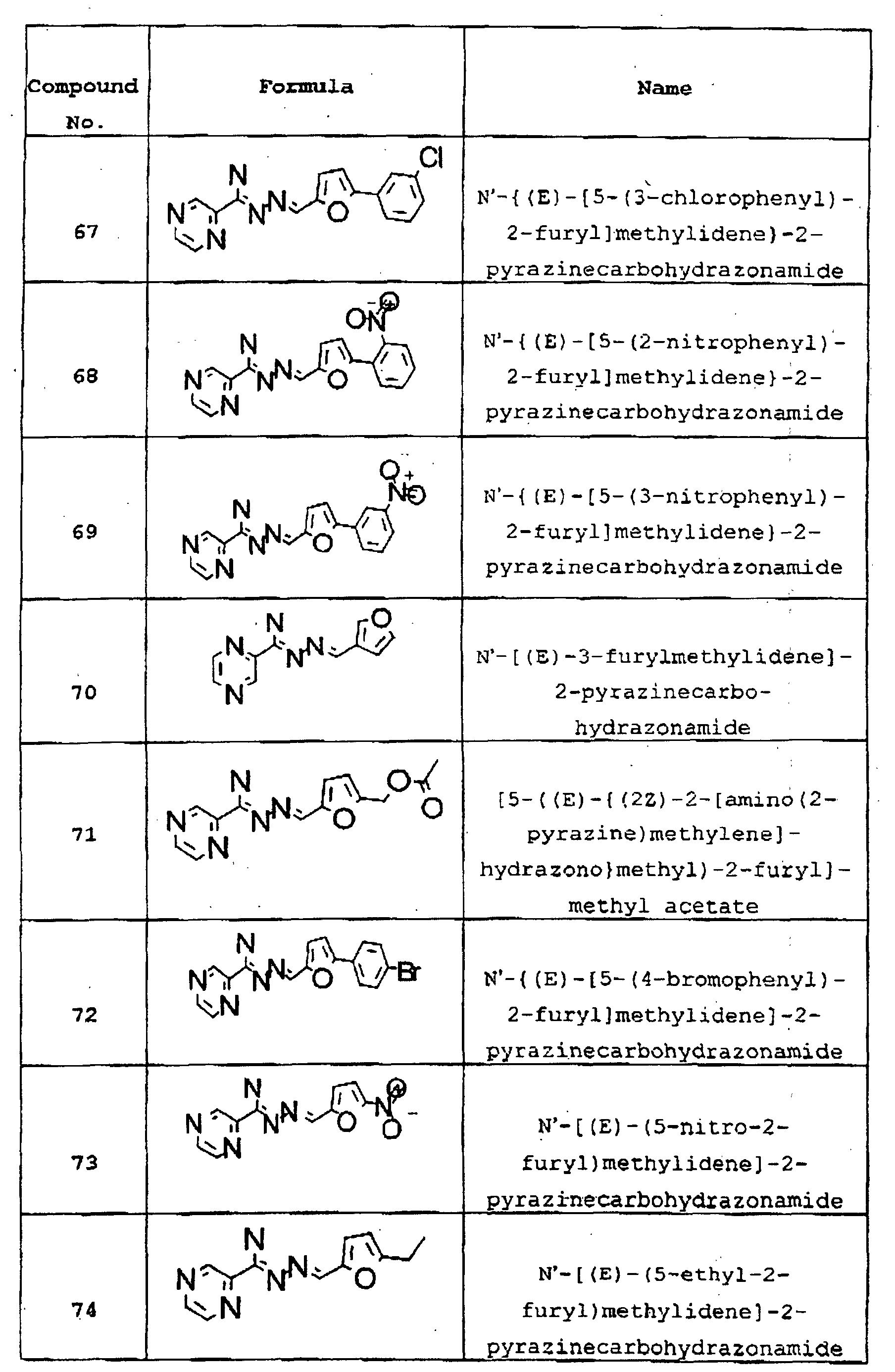

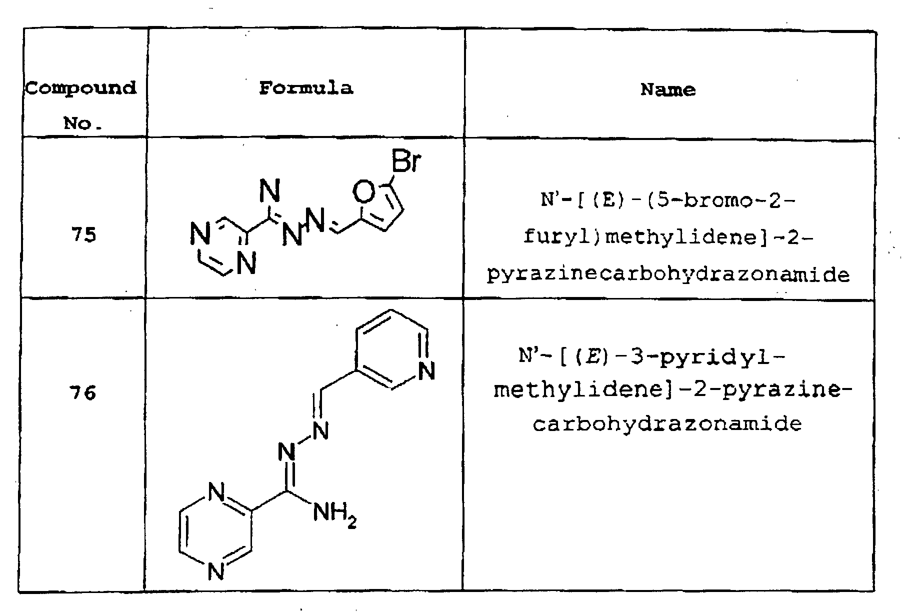

3. Composition according to Claim 1, where the aminoamidine derivative is N'- [(E)- (3-methoxyphenyl)-methylidene]benzenecarbohydrazonamide having the structure:

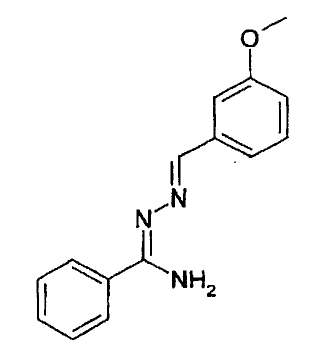

4. Composition according to Claim 1 or 2, where the aminoamidine derivative is N'- [(*E*)-3-pyridinylmethylidene]-2-pyrazinecarbohydrazonamide having the structure:

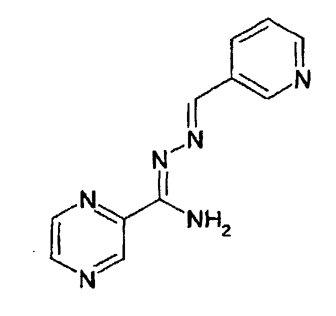

5. Composition according to Claim 1 or 2, where the aminoamidine derivative is N'-[(E)-2-hydroxyphenylmethylidene]pyrazinecarbohydrazonamide having the structure:

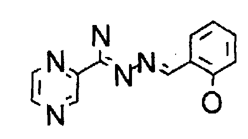

6. Composition according to any one of Claims 1 to 5, **characterized in that** the compound of formula (1) or (2) is present in the composition in a content ranging from 0.1 to 20% by weight, relative to the total weight of the composition, preferably ranging from 0.5% to 10% by weight, relative to the total weight of the composition.

7. Composition according to any one of Claims 1 to 6, **characterized in that** the said cosmetically acceptable carrier is present in the form of an emulsion of the oil-in-water type.

8. Composition according to any one of Claims 1 to 7, **characterized in that** it comprises, in addition, one or more additional organic screening agents which are active in UV-A and/or UV-B.

9. Composition according to Claim 8, **characterized in that** the said additional organic screening agents are chosen from cinnamic derivatives; dibenzoylmethane derivatives; salicylic derivatives, camphor derivatives; triazine derivatives; benzophenone derivatives; β,β'-diphenylacrylate derivatives; benzimidazole derivatives; bisbenzoazolyl derivatives; p-aminobenzoic acid derivatives; screening polymers and silicones; micronized insoluble organic UV-screening agents.

10. Composition according to any one of Claims 1 to 9, **characterized in that** it comprises, in addition, as additional UV-photoprotective agents, pigments or nanopigments of metal oxides, coated or otherwise.

11. Composition according to Claim 10, **characterized in that** the said pigments or nanopigments are chosen from titanium, zinc, iron, zirconium and cerium oxide and mixtures thereof, coated or otherwise.

12. Composition according to any one of Claims 1 to 11, **characterized in that** it comprises, in addition, at least one agent for artificially darkening and/or tanning the skin.

13. Composition according to any one of Claims 1 to 12, **characterized in that** it comprises, in addition, at least one adjuvant chosen from fatty substances, organic solvents, thickeners, demulcents, antioxidants, opacifiers, stabilizers, emollients, anti-foaming agents, moisturizing agents, perfumes, preservatives, surfactants, sequestrants, polymers, propellants, alkalinizing or acidifying agents.

14. Composition according to any one of Claims 1 to 12, **characterized in that** it is a composition for protecting the human epidermis or an anti-sun composition and **in that** it is provided in the form of a nonionic vesicular dispersion, an emulsion, in particular an emulsion of the oil-in-water type, a cream, a milk, a gel, a gel cream, a suspension, a dispersion, a powder, a solid stick, a foam or a spray.

15. Composition according to any one of Claims 1 to 12, **characterized in that** it is a make-up composition for the eyelashes, the eyebrows, the nails or the skin and **in that** it is provided in solid or pasty, anhydrous or aqueous form or in the form of an emulsion, a suspension or a dispersion.

16. Composition according to any one of Claims 1 to 12, **characterized in that** it is a composition intended for protecting the hair against ultraviolet rays and **in that** it is provided in the form of a shampoo, a lotion, a gel, an emulsion or a nonionic vesicular dispersion.

17. Use of an aminoamidine derivative of formula (I) as defined in any one of Claims 1 to 5 for the manufacture of a composition intended for protecting materials which are sensitive to ultraviolet radiation, in particular to solar radiation.

18. Use of an aminoamidine derivative of formula (I) as defined in any one of Claims 1 to 5 for the manufacture of a cosmetic or dermatological composition as an agent for screening out UV radiation.

19. Use of an aminoamidine derivative of formula (I) as defined in any one of Claims 1 to 5 for the manufacture of a cosmetic composition as an agent for controlling the variation of skin colour due to UV radiation.

20. Aminoamidine derivative chosen from the following compounds:
